# EUROPEAN PATENT APPLICATION

(11) **EP 3 932 422 A1**
(43) Date of publication of application: **05.01.2022**
(21) Application number: 21173406.6
(22) Date of filing: 24.07.2013
(51) Int. Cl.: A61K 39/12, A61K 39/00, A61P 43/00, A61P 31/14

(54) **VACCINE COMPOSITIONS FOR THE PREVENTION OF DENGUE VIRUS INFECTION**

(30) Priority: 24.07.2012 EP 12305907; 25.07.2012 EP 12305912
(62) Divisional of application: 13741748.1
(71) Applicant: Sanofi Pasteur, 69007 Lyon (FR)
(72) Inventor: TORNIEPORTH, Nadia, 69890 La Tour de Salvagny (FR); BOUCKENOOGHE, Alain, 588279 Singapore (SG); FORRAT, Remi, 69360 Serezin du Rhone (FR); LANG, Jean, 69780 Mions (FR); SAVILLE, Mélanie, 69370 St Didier au Mont D'Or (FR)
(74) Representative: Abel & Imray LLP

(57) **Abstract**

The present invention relates to vaccine compositions that are useful in a method of protecting a human subject against dengue disease.

## Description

### FIELD OF THE INVENTION

The present invention relates to vaccine compositions and uses of such compositions in a method of protecting a human subject against dengue disease.

### BACKGROUND

Dengue is the second most important infectious tropical disease after malaria with approximately one-half of the world's population living in areas where there is a risk of epidemic transmission. There are estimated to be 50-100 million cases of dengue disease every year resulting in 500,000 patients being hospitalized for dengue hemorrhagic fever (DHF) and resulting in approximately 25,000 deaths.

Dengue disease infections are endemic in more than 100 tropical countries and dengue hemorrhagic fever (DHF) has been documented in 60 of these countries (Gubler, 2002, TRENDS in Microbiology, 10: 100-103).

Dengue disease is caused by four antigenically distinct, but closely related dengue virus serotypes of the flavivirus genus (Gubler et al., 1988, in: Epidemiology of arthropod-borne viral disease. Monath TPM, editor, Boca Raton (FL): CRC Press: 223-60; Kautner et al., 1997, J. of Pediatrics, 131 : 516-524; Rigau-Perez et al., 1998, Lancet, 352: 971-977; Vaughn et al., 1997, J. Infect. Dis., 176: 322-30).

Dengue disease is usually transmitted by injection of the dengue virus during the blood meal of an *Aedes aegypti* mosquito infected by the virus. After an incubation period of 4-10 days, the illness begins abruptly and is followed by three phases: febrile (2 to 7 days), critical (24-48 hours - during which severe complications may occur) and recovery (48-72 hours). During the critical phase, life threatening complications such as hemorrhages, shock and acute organ impairment may occur. A proper management of these unpredictable outcomes can reduce the case fatality rate. Cure of dengue fever is complete after 7 to 10 days, but prolonged asthenia is normal. Reduced leukocyte and platelet numbers are frequently observed.

Dengue haemorrhagic fever (DHF) is a potentially deadly complication of dengue virus infection. DHF is characterized by a high fever and symptoms of dengue disease, but with extreme lethargy and drowsiness. Increased vascular permeability and abnormal homeostasis can lead to a decrease in blood volume, hypotension, and in severe cases, hypovolemic shock and internal bleeding. Two factors appear to play a major role in the occurrence of DHF - rapid viral replication with a high level of viremia (the severity of the disease being associated with the level of viremia; Vaughn et al., 2000, J. Inf. Dis., 181: 2-9) and a major inflammatory response with the release of high levels of inflammatory mediators (Rothman and Ennis, 1999, Virology, 257: 1-6; Alan L. Rothman. 2011, Nature Reviews Immunology, 11: 532-543)). The mortality rate for DHF can reach 10% without treatment, but is < 1 % in most centres with access to treatment.

Dengue shock syndrome (DSS) is a common progression of DHF and is frequently fatal. DSS results from generalized vasculitis leading to plasma leakage into the extravascular space. DSS is characterized by rapid and poor volume pulse, hypotension, cold extremities, and restlessness.

In Asia, DHF and DSS are observed primarily in children, with approximately 90% of those with DHF being less than 15 years of age (Malavige et al., 2004, Postgrad Med. J., 80: 588-601; Meulen et al., 2000, Trop. Med. Int. Health, 5:325-9). In contrast, outbreaks in the Caribbean and Central America have predominantly affected adults (Malavige et al., 2004, Postgrad Med. J., 80: 588-601).

The four serotypes of dengue virus possess approximately 60-80% sequence homology. Infection with one dengue serotype provides durable homologous immunity but limited heterologous immunity (Sabin, 1952, Am. J. Trop. Med. Hyg., 1: 30-50). Accordingly, an individual that has been infected with one serotype of dengue may subsequently become infected with a different serotype. In the past, it has been considered that a second infection arising from a different dengue virus serotype is theoretically a risk factor for the development of DHF, since the majority of patients that exhibit DHF have been previously exposed to at least one of the other four serotypes of dengue viruses.

To date, there is no specific treatment for dengue disease. Treatment for dengue disease is symptomatic, with bed rest, control of the fever and pain through antipyretics and analgesics, and adequate drinking. The treatment of DHF requires balancing of liquid losses, replacement of coagulation factors and the infusion of heparin.

Since dengue prevention measures, such as mosquito control and personal protection from bites, are limited in efficacy, difficult to enforce and expensive, a safe and efficacious dengue vaccine would be the best mode of prevention. However, there is no licensed vaccine of this type that is currently available.

It is therefore desirable to develop a vaccine composition that demonstrates efficacy when used in a method of protecting a human subject against dengue disease.

### SUMMARY OF THE INVENTION

The present invention relates to a vaccine composition for use in a method of protecting a human subject against dengue disease, wherein said composition comprises:
(i) a dengue antigen selected from the group consisting of:
   (a) a live attenuated dengue virus;
   (b) an inactivated dengue virus;
   (c) a live attenuated or inactivated chimeric dengue virus;
   (d) a dengue virus-like particle (VLP); and
   (e) a combination of two or more of (a) to (d);
   or
(ii) a nucleic acid construct or viral vector which is able to express in a human cell a dengue antigen which is a dengue VLP.

The present invention further relates to the use of a vaccine composition of the present invention for the manufacture of a medicament for protecting a human subject against dengue disease.

The present invention further relates to a method of protecting a human subject against dengue disease, wherein said method comprises administering to said human subject an effective amount of a composition according to the present invention.

Additionally, the present invention relates to a kit comprising a composition according to the present invention and instructions for the use of said composition in a method of protecting a human subject against dengue disease.

### Description of the Figure

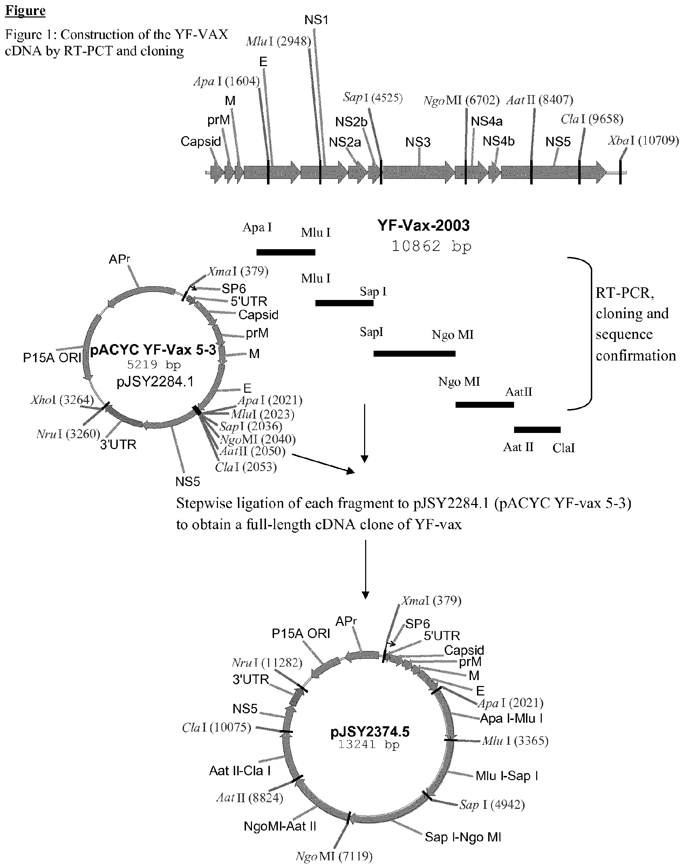

Figure 1 illustrates the construction of the YF-VAX cDNA by RT-PCR and cloning

### Definitions

The term "Dengue disease", as used herein, refers to the clinical symptoms exhibited by an individual following infection by any one of the four Dengue virus serotypes. Since 1970, clinical dengue has been classified according to World Health Organization guidelines as (i) dengue fever or (ii) dengue hemorrhagic fever (World Health Organization. Dengue hemorrhagic fever: Diagnosis, treatment, prevention and control 2nd Ed. Geneva: WHO, 1997; ISBN 92 4 154500 3). In 2009, the WHO issued new guidelines that classify clinical dengue as (i) dengue with or without warning signs or (ii) severe dengue. Both classifications are shown in Figures 1 & 2 of Srikiatkachorn et al., Clin. Infect. Dis. (2011) 53(6): 563. According to the earlier classification, dengue fever is characterized by at least two symptoms selected from headache, arthralgia, retro-orbital pain, rash, myalgia, hemorrhagic manifestations, and leucopenia, together with supportive serology or occurrence at the same location and time as other confirmed dengue cases. Progression to Dengue hemorrhagic fever is confirmed when fever, hemorrhagic manifestations, thrombocytopenia and evidence of plasma leakage are all observed. According to the more recent classification, diagnosis of dengue requires the presence of fever and at least two clinical symptoms selected from nausea, vomiting, rash, aches and pains, a positive tourniquet test, or any warning signs selected from abdominal pain and tenderness, persistent vomiting, clinical fluid accumulation, mucosal bleed, lethargy or restlessness, liver enlargement greater than 2 cm or an increase in hematocrit concurrent with a rapid decrease in platelet count. Severe dengue is diagnosed when any of the following events are observed: severe plasma leakage leading to shock or respiratory distress, severe bleeding as evaluated by clinicians or severe organ involvement

The term "Dengue hemorrhagic fever or DHF", as used herein, refers to virologically-confirmed dengue disease wherein fever, hemorrhagic manifestations, thrombocytopenia and evidence of plasma leakage are all observed. DHF, as used herein, may be further defined on the basis of its severity. For instance, DHF may be defined as being of Grade I, Grade II, Grade III or Grade IV (World Health Organization. Dengue hemorrhagic fever: Diagnosis, treatment, prevention and control 2nd Ed. Geneva: WHO, 1997; ISBN 92 4 154500 3). Grade I is defined as fever accompanied by nonspecific constitutional symptoms; the only haemorrhagic manifestation is a positive tourniquet test and/or easy bruising. Grade II is defined as spontaneous bleeding in addition to the manifestations of Grade I patients, usually in the form of skin or other haemorrhages. Grade III is defined as circulatory failure manifested by a rapid, weak pulse and narrowing of pulse pressure or hypotension, with the presence of cold clammy skin and restlessness. Grade IV is defined as profound shock with undetectable blood pressure or pulse. As would be understood by a person of skill in the art, in the practice of the present invention, e.g. a method of protecting against DHF, said DHF need not be virologically-confirmed.

The term "virologically-confirmed dengue", as used herein, refers to an acute febrile episode which is confirmed to be induced by a dengue virus, e.g. by reverse transcriptase polymerase chain reaction (RT-PCR) or by a dengue non-structural 1 (NS1) protein enzyme-linked immunosorbent assay (ELISA). In the RT-PCR method, serum samples are tested according to the method of Callahan et al, J. Clin. Microbiol. (2001) 39: 4119. Briefly, RNA is extracted from the serum to discard potential Taq polymerase inhibitors or interfering factors, using a commercial kit. Then an RT-PCR reaction is carried out with serotype specific primers from the dengue NS5 gene sequence. Results are expressed as a concentration of log₁₀GEQ (genome equivalent)/mL, by comparison with standards containing known concentrations of viral genomic serotype-specific nucleic acid sequences integrated into plasmids. In the ELISA method, 50 µL of patient serum, a positive control, a negative control, or a cut-off control are diluted 1:2 in sample diluent and combined with 100 µL of diluted horseradish peroxidase (HRP)-labeled anti-NS1 monoclonal Ab (MAb). The diluted serum and conjugate are added to capture anti-NS1 MAb-coated microwells, and plates are incubated for 90 minutes at 37°C. Capture MAb/NS1/HRP-labeled-MAb complexes are formed when NS1 is present in the serum. Complexes are detected via a colorimetric reaction in positive wells which is induced by adding 160 µL of 3,3',5,5' tetramethylbenzidine (TMB) substrate and incubating for 30 minutes at room temperature in the dark. The reaction is stopped with the addition of 100 µL of stop solution (1N H₂SO4) and the plate is read. A sample ratio is determined for each sample by dividing the average optical density (OD) of the test sample by the average OD of the cut-off control (tested in quadruplicate). Sample ratios of <0.5, 0.5-<1.0, and ≥1 are indicative of negative, equivocal, and positive results, respectively.

The term "severe virologically-confirmed dengue", as used herein, refers to dengue haemorrhagic fever (DHF) as defined by the 1997 WHO classification and further characterized by the following additional list of symptoms: haemorrhage requiring blood transfusion, objective evidence of capillary permeability, signs of circulatory failure or visceral manifestations.

The term "dengue shock syndrome", as used herein, refers to the most severe complications of DHF as defined above. According to the 1997 WHO classification, DSS corresponds to DHF of Grades III and IV.

The term "dengue fever viruses", "dengue viruses" and "DEN" are used interchangeably. They refer to positive single-strand RNA viruses belonging to the Flavivirus genus of the family of flaviviridae. There are four different serotypes of dengue virus (serotypes 1, 2 3 and 4), which possess approximately 60-80% sequence homology. The organization of the genome comprises the following elements: a 5' non-coding region (NCR), a region encoding structural proteins (capsid (C), pre-membrane (prM) and envelope (E)) and a region encoding non-structural proteins (NS1-NS2A-NS2B-NS3-NS4A-NS4B-NS5) and a 3' NCR. The dengue viral genome encodes an uninterrupted coding region which is translated into a single polyprotein which undergoes post-translational processing.

In the context of the present invention, "vaccinal dengue virus" refers to a virus which is capable of inducing neutralizing antibodies against the dengue virus serotype from which the vaccinal dengue virus is derived, by the administration of such vaccinal dengue virus to an immunocompetent subject. Examples of vaccinal dengue viruses which may be used in a method of the present invention include inactivated dengue viruses, live attenuated dengue viruses and live attenuated or inactivated chimeric dengue viruses. Serotypes of vaccinal dengue viruses for use in the present invention include serotypes 1, 2, 3, and 4. Preferably a vaccinal dengue virus for use in the present invention is a live attenuated chimeric dengue virus.

The expression "inactivated virus", as used herein, refers to a virus that is incapable of replication to any significant degree in cells permissive for replication of the corresponding wild type virus. Viruses may be inactivated by a number of means well known to those skilled in the art. Examples of methods for inactivating a virus include chemical treatments, or radiation treatments (including heat or electromagnetic radiation typically in the forms of X-ray or ultraviolet radiation).

The term "inactivated dengue virus", as used herein refers to an inactivated wild-type virus containing all the dengue structural proteins (env, premembrane/membrane and capsid proteins) and inactivated viral RNA. An inactivated dengue virus may also refer to an inactivated chimeric dengue virus. Inactivated dengue viruses are for instance described in United States Patent No. 6,254,873.

The term "live attenuated virus or LAV", as used herein, refers to a virus which is not able to induce a disease state characterised by the same sets of symptoms associated with the corresponding wild-type virus. Examples of live attenuated viruses are well known in the art. A live attenuated virus may be prepared from a wild-type virus, for example, by recombinant DNA technology, site directed mutagenesis, genetic manipulation, serial passages on replication-competent cells, chemical mutagenesis treatment or electromagnetic radiation.

The term "live attenuated dengue virus", as used herein, refers to a live dengue virus derived from a virulent wild-type dengue virus by genetic modification resulting in attenuation of virulence and an inability to induce a disease state characterised by the same sets of symptoms associated with the corresponding wild type dengue virus. Examples of live attenuated dengue viruses useful in the practice of the present invention include VDV-1, VDV-2, and the strains described for example in applications WO 02/66621, WO 00/57904, WO 00/57908, WO 00/57909, WO 00/57910, WO 02/0950075 and WO 02/102828. Live attenuated dengue viruses of serotype 1 which may be used in the method of the invention include VDV-1. Live attenuated dengue viruses of serotype 2 which may be used in the method of the invention include VDV-2, and LAV-2.

"VDV" and "Vero dengue vaccine" are used interchangeably herein and designate a live attenuated dengue virus capable of replication in Vero cells and capable of inducing a specific humoral response, including the induction of neutralizing antibodies, in a human.

The DEN-1 16007/PDK13 strain, also called "LAV1", is derived from wild-type DEN-1 (dengue virus serotype 1) 16007 strain which has undergone 11 passages through primary dog kidney (PDK) cells (DEN-1 16007/PDK11). LAV1 has been described in patent application EP1 159968 in the name of Mahidol University and has been filed with the National Microorganisms Cultures Collection (CNCM) under number I-2480. "VDV-1" is a virus derived from LAV1 by subsequent adaptation to Vero cells; in this regard, the RNA from LAV1 has been extracted and purified before being transfected into Vero cells. The VDV-1 strain has subsequently been obtained by plate purification and amplification in Vero cells. The VDV-1 strain has 14 additional mutations in comparison with the DEN-1 16007/PDK13 strain (13 passes through PDK cells). A process for preparing and characterizing the VDV-1 strain has been described in international patent application filed under number WO06/134433 in the names of Sanofi-Pasteur and the Center for Disease Control and Prevention.

The DEN-2 16681/PDK53 strain, also known as "LAV2", has been obtained from wild-type strain DEN-2 (dengue virus serotype 2) 16681 which has undergone 50 passes through PDK cells (DEN-2 16681/PDK50). LAV2 has been described in in patent application EP1159968 in the name of Mahidol University and has been filed with the National Microorganisms Cultures Collection (CNCM) under number 1-2481. "VDV-2" is a strain derived from LAV2 by subsequent adaptation to Vero cells; in this regard, the RNA from LAV2 has been extracted and purified before being transfected in Vero cells. The VDV-2 strain has subsequently been obtained by plate purification and amplification in Vero cells. The VDV-2 strain has 10 additional mutations in comparison with the DEN-2 16681/PDK53 strain (53 passes through PDK cells), including 4 silent mutations. A process for preparing and characterizing the VDV-2 strain has been described in the international patent application filed under number WO06/134443 in the names of Sanofi-Pasteur and the Center for Disease Control and Prevention. The complete nucleic acid sequence of the VDV-2 strain is as set forth in SEQ ID NO: 24. The sequence of the M protein of the VDV-2 strain is as shown in SEQ ID NO: 27 and the sequence of the E protein of the VDV-2 strain is as shown in the SEQ ID NO: 26.

The VDV 1 and 2 strains are prepared by amplification in Vero cells. The viruses produced are harvested and clarified from cell debris by filtration. The DNA is digested by treatment with enzymes. Impurities are eliminated by ultrafiltration. Infectious titers may be increased by a concentration method. After adding a stabilizer, the strains are stored in lyophilized or frozen form before use and then reconstituted when needed.

In the context of the invention, "dengue chimera or chimeric dengue virus" means a recipient flavivirus in which the genetic backbone has been modified by exchanging the sequences encoding the prM and E proteins of the recipient flavivirus by the corresponding sequences of a dengue virus. Typically, the recipient flavivirus may be attenuated. The recipient flavivirus may be a yellow fever (YF) virus such as the attenuated YF 17D, YF 17DD and YF 17D204 (YF-VAX^{®}) viruses; in that case, such chimeras are referred to as YF/dengue chimeras. The recipient flavivirus may also be a dengue virus and in that case, it is referred to as dengue/dengue chimera, the dengue virus serotype characteristic of the prM and E proteins being identical or different from the recipient dengue virus serotype characteristic of the genetic backbone. When the serotypes are identical, the recipient dengue virus and the dengue virus from which the prM and E protein encoding sequences originate, are two different virus strains of the same serotype. For use in the present invention, chimeric dengue viruses are typically YF/dengue chimeras. Chimeric dengue viruses are preferably inactivated or live attenuated chimeric dengue viruses. Advantageously, the recipient flavivirus of a live attenuated chimeric dengue virus of the present invention is YF 17D or YF 17D204. According to one embodiment dengue chimera is an inactivated virus. According to an alternative embodiment the dengue chimera is a live attenuated virus. Dengue Chimera that can be used in the method of protection of the present invention include Chimerivax^{™} Dengue Serotype 1 (also known as CYD-1), Chimerivax^{™} Dengue Serotype 2 (also known as CYD-2), Chimerivax^{™} Dengue Serotype 3 (also known as CYD-3) and Chimerivax^{™} Dengue Serotype 4 (also known as CYD-4).

Examples of chimeric dengue viruses useful in the practice of the present invention include the dengue/YF chimeric viruses described in patent application WO 98/37911 and dengue/dengue fever chimeras such as those described in patent applications WO 96/40933 and WO 01/60847.

In one embodiment, the chimeric YF/dengue virus comprises the genomic backbone of the attenuated yellow fever virus strain YF17D (Theiler M. and Smith H.H., 1937, J.Exp.Med., 65. 767-786), e.g. viruses YF17D/DEN-1, YF17D/DEN-2, YF17D/DEN-3 and YF17D/DEN-4. Examples of YF17D strains which may be used include YF17D204 (YF-VAX(R), Sanofi-Pasteur, Swiftwater, PA, USA; Stamaril(R), Sanofi-Pasteur, Marcy l'Etoile, France; ARILVAX(TM), Chiron, Speke, Liverpool, UK; FLAVIMUN(R), Berna Biotech, Bern, Switzerland; YF17D-204 France (X15067, X15062); YF17D-204,234 US (Rice et al., 1985, Science, 229: 726-733), or the related strains YF17DD (Genbank access number U17066), YF17D-213 (Genbank access number U17067) and the strains YF17DD described by Galler et al. (1998, Vaccines, 16(9/10): 1024-1028).

One example of a chimeric dengue virus particularly suitable for use in the practice of the present invention is a "Chimerivax dengue virus". As used herein, a "Chimerivax dengue virus", is a live attenuated chimeric YF/dengue virus which comprises the genomic backbone of a YF17D or YF17D204 (YF-VAX^{®}) virus in which the nucleic acid sequences encoding the pre-membrane (prM) and envelope (E) proteins have been replaced by nucleic acid sequences encoding the corresponding structural proteins of a dengue virus. A preferred chimeric dengue virus for use in the present invention is a live attenuated chimeric YF/dengue virus which comprises the genomic backbone of a YF17D virus in which the nucleic acid sequences encoding the pre-membrane (prM) and envelope (E) proteins have been replaced by nucleic acid sequences encoding the corresponding structural proteins of a dengue virus. A preferred chimeric dengue virus for use in the present invention is a live attenuated chimeric YF/dengue virus which comprises the genomic backbone of a YF17D204 (YF-VAX^{®}) virus in which the nucleic acid sequences encoding the pre-membrane (prM) and envelope (E) proteins have been replaced by nucleic acid sequences encoding the corresponding structural proteins of a dengue virus. Construction of such Chimerivax viruses may be achieved in accordance with, or in substantial accordance with, the teaching of Chambers, et al. (1999, J.Virology 73(4):3095-3101). The particular Chimerivax (CYD) dengue viruses described in the examples have been generated by using prM and E sequences from strains DEN 1 PU0359 (TYP1 140), DEN2 PUO218, DEN3 PaH881/88 and DEN 4 1228 (TVP 980) and the genomic backbone of YF17D virus. Those particular Chimerivax strains are referred to herein (see the present examples) as "CYD-1", "CYD-2", "CYD-3" and "CYD-4" respectively. The preparation of these particular CYD-1, CYD-2, CYD-3 and CYD-4 strains has been described in detail in international patent applications WO 98/37911, WO 03/101397, WO 07/021672, WO 08/007021, WO 08/047023 and WO 08/065315, to which reference may be made for a precise description of the processes for their preparation. Alternatively, other dengue fever virus strains may be used as a source of nucleic acids to facilitate construction of chimeric viruses useful in the practice of the present invention, for example in the construction of other Chimerivax dengue serotype 1 (CYD-1), Chimerivax dengue serotype 2 (CYD-2), Chimerivax dengue serotype 3 (CYD-3) and Chimerivax dengue serotype 4 (CYD-4) strains. Advantageously, a vaccine composition of the present invention, e.g. a chimeric dengue virus, of serotype 2 may comprise prM-E sequences having at least 90%, at least 95%, at least 98% or at least 99% identity to the prM-E sequences from the serotype 2 strains LAV-2, BID-V585, PR/DB023 or MD1280 as described in the examples or may comprise prM-E sequences having at least 90%, at least 95%, at least 98% or at least 99% identity to the prM-E sequence shown in SEQ ID NO: 2. Advantageously, a vaccine composition, e.g. a chimeric dengue virus, of serotype 2 for use in the method of the present invention may comprise prM-E sequences from the serotype 2 strains LAV-2, BID-V585, PR/DB023 or MD1280 or the prM-E sequence from SEQ ID NO: 2 as described in the examples. When the recipient genomic backbone of such chimeric dengue viruses is derived from YF-VAX^{®}, such strains are referred to herein as CYD-LAV, CYD-BID, CYD-PR and CYD-MD. A vaccine composition of the present invention comprising chimeric dengue virus of serotype 2 generated using the prM-E sequences of the serotype 2 strains LAV-2 (SEQ ID NO: 8), BID-V585 (SEQ ID NO: 9), PR/DB023 (SEQ ID NO: 10), MD1280 (SEQ ID NO: 11) or SEQ ID NO: 2, or generated using prM-E sequences having at least 90%, at least 95%, at least 98% or at least 99% identity to the prM-E sequences from the serotype 2 strains LAV-2, BID-V585, PR/DB023, MD1280 or the prM-E sequence from SEQ ID NO: 2 may advantageously be used in combination with CYD-1, CYD-3 and CYD-4 in a vaccine composition according to the present invention.

An alternative embodiment of chimeric dengue virus usable in the method of protection of the invention is a recipient flavivirus in which the genetic backbone has been modified by exchanging (i) the sequence encoding the E protein of the recipient flavivirus by the corresponding sequence of a dengue virus and (ii) the sequence encoding the prM protein of the recipient flavivirus by the corresponding sequence of a non-dengue flavivirus, e.g. a JEV virus. Typically, the said chimeric virus may be a live attenuated virus or an inactivated virus. Examples of such chimeric dengue viruses are described in WO2011/138586.

A vaccinal dengue virus of serotype 1 for use in a vaccine composition of the present invention may, for example, be the strain VDV1, CYD-1 or a YF17D/DEN-1 chimeric virus comprising the prM and E amino acid sequences of the DEN-1 16007/PDK13 strain. A vaccinal dengue virus of serotype 2 for use in the method of the present invention may, for example, be the strain VDV2, CYD-2, a YF17D/DEN-2 chimeric virus comprising the prM and E amino acid sequences of the DEN-2 16681/PDK53 strain, a chimeric virus comprising the prM and E amino acid sequences of the DEN-2 strains LAV-2, BID-V585, PR/DB023 or MD1280 or a chimeric virus comprising prM-E sequences having at least 90%, at least 95%, at least 98% or at least 99% identity to the prM-E sequences from the serotype 2 strains LAV-2, BID-V585, PR/DB023 or MD1280 or at least 90%, at least 95%, at least 98% or at least 99% identity to the prM-E sequence in SEQ ID NO: 2. A vaccinal dengue virus of serotype 3 for use in the method of present invention may, for example, be CYD-3 or an alternative YF17D/DEN-3 chimeric virus. An example of a vaccinal dengue virus of serotype 4 is CYD-4 or an alternative YF17D/DEN-4 chimeric virus.

A composition of the present invention comprises at least one dengue antigen. Typically a composition of the present invention comprises a dengue antigen, e.g. a vaccinal dengue virus, of each of serotypes 1, 2, 3 and 4. Dengue antigens, e.g. vaccinal dengue viruses, of the present invention of each serotype may be as described herein. For instance, a composition of the present invention may advantageously comprise any one of the following combinations of dengue antigens: i) a dengue antigen comprising the prM and E sequences of CYD-1, a dengue antigen comprising the prM and E sequences of CYD-LAV, a chimeric dengue virus comprising the prM and E amino acid sequences of CYD-3 and a dengue antigen comprising the prM and E sequences of CYD-4; ii) a dengue antigen comprising the prM and E sequences of CYD-1, a dengue antigen comprising the prM and E sequences of CYD-BID, a dengue antigen comprising the prM and E sequences of CYD-3 and a dengue antigen comprising the prM and E sequences of CYD-4; (iii) a dengue antigen comprising the prM and E sequences of CYD-1, a dengue antigen comprising the prM and E sequences of CYD-PR, a dengue antigen comprising the prM and E sequences of CYD-3 and a dengue antigen comprising the prM and E sequences of CYD-4; (iv) a dengue antigen comprising the prM and E sequences of CYD-1, a dengue antigen comprising the prM and E sequences of CYD-MD, a dengue antigen comprising the prM and E sequences of CYD-3 and a dengue antigen comprising the prM and E sequences of CYD-4;. For instance, a composition of the present invention may also advantageously comprise any one of the following combinations of dengue antigens: i) CYD-1, CYD-LAV, CYD-3 and CYD-4; ii) CYD-1, CYD-BID, CYD-3 and CYD-4; (iii) CYD-1, CYD-PR, CYD-3 and CYD-4 or (iv) CYD-1, CYD-MD, CYD-3 and CYD-4. A composition of the present invention may also advantageously comprise the following combination of dengue antigens: i) a dengue antigen comprising the prM and E sequences of CYD-1, VDV2, a dengue antigen comprising the prM and E sequences of CYD-3 and a dengue antigen comprising the prM and E sequences of CYD-4. For instance, a composition of the present invention may advantageously comprise CYD-1, VDV-2, CYD-3 and CYD-4. A composition of the present invention, as described herein, may advantageously comprise a dengue antigen of serotype 2 which comprises the prM-E sequence of CYD-LAV (SEQ ID NO: 8), CYD-BID (SEQ ID NO: 9), CYD-PR (SEQ ID NO: 10) CYD-MD (SEQ ID NO: 11) or SEQ ID NO: 2. A composition of the present invention, as described herein, may advantageously comprise a dengue antigen of serotype 2 which comprises a sequence having at least 90% identity to the prM-E sequence of CYD-LAV (SEQ ID NO: 8), CYD-BID (SEQ ID NO: 9), CYD-PR (SEQ ID NO: 10) CYD-MD (SEQ ID NO: 11) or SEQ ID NO: 2. For example, said sequence may be at least 91%, at least 92%, at least 93%, at least 94%, at least 95%, at least 96%, at least 97%, at least 98% or at least 99% identical to the prm-E sequence of CYD-LAV (SEQ ID NO: 8), CYD-BID (SEQ ID NO: 9), CYD-PR (SEQ ID NO: 10) CYD-MD (SEQ ID NO: 11) or SEQ ID NO: 2.

The term "virus-like particles or VLPs", as used herein, refers to virus particles that do not contain replicative genetic material but present at their surface a dengue E protein in a repetitive ordered array similar to the virion structure. Typically, dengue VLPs also contain dengue prM and/or M, and E proteins. VLPs may be produced *in vitro* (Zhang et al, J. Virol. (2011) 30 (8):333). VLPs may also be produced *in vivo.* To that end, nucleic acid constructs (e.g. DNA or RNA constructs) encoding prM and E dengue proteins may be introduced into a cell of a subject, e.g. a human subject, via methods known in the art, e.g. via use of a viral vector. Any viral vector may be used provided it is able to contain and express both prM and E dengue virus sequences. Non-limiting examples of viral vectors that may be used in the method of the present invention include the poxviruses (e.g. the attenuated pox Ankara virus) and the measles virus. For use in the present invention, a particular category of viral vector expressing VLPs *in vivo* includes replication-deficient pseudoinfectious (PIV) viruses, e.g. according to the Replivax^{™} technology. (Rumyantsev AA, et al. Vaccine. 2011 Jul 18;29(32):5184-94).

The term "replication-defective pseudo-infectious virus", as used herein, refers to a virion particle that is replication-defective *in vivo,* owing to the absence in their genome of an essential sequence of the replicative cycle, for example the sequence encoding a capsid protein. However, the virion particles can propagate in a culture of helper cells that provide for the essential sequence(s) *in trans.* Replication-deficient pseudoinfectious viruses for use in the present invention include any virus according to the above definition which is capable of expressing the prM and E proteins of a dengue virus of any serotype. Examples include replication defective flavivirus / dengue chimeras such as replication defective West Nile virus / dengue, Japanese Encephalitis virus / dengue and YF / dengue chimeras.

The ability of a vaccine composition of the present invention to provoke an immune response in a subject (i.e. induce the production of neutralizing antibodies) can be assessed, for example, by measuring the neutralizing antibody titre raised against the dengue virus serotype(s) comprised within the composition. The neutralizing antibody titre may be measured by the Plaque Reduction Neutralization Test (PRNT₅₀) test. Briefly, neutralizing antibody titre is measured in sera collected from vaccinated subjects at least 28 days following administration of a vaccine composition of the present invention. Serial, two-fold dilutions of sera (previously heat-inactivated) are mixed with a constant challenge-dose of each dengue virus of serotype 1, 2, 3 or 4 as appropriate (expressed as PFU/mL). The mixtures are inoculated into wells of a microplate with confluent Vero cell monolayers. After adsorption, cell monolayers are incubated for a few days. The presence of dengue virus infected cells is indicated by the formation of infected foci and a reduction in virus infectivity due to the presence of neutralising antibodies in the serum samples can thus be detected. The reported value (end point neutralization titre) represents the highest dilution of serum at which ≥ 50 % of dengue challenge virus (in foci counts) is neutralized when compared to the mean viral focus count in the negative control wells (which represents the 100% virus load). The end point neutralization titres are presented as continuous values. The lower limit of quantification (LLOQ) of the assay is 10 (1/dil). It is commonly considered that seroconversion occurs when the titer is superior or equal to 10 (1/dil). As PRNT tests may slightly vary from a laboratory to another the LLOQ may also slightly vary. Accordingly, in a general manner, it is considered that seroconversion occurs when the titre is superior or equal to the LLOQ of the test. Neutralising antibody titres were considered in the following references, but the authors did not establish a correlate of protection (Guirakhoo et al, J. Virol. (2004) 78 (9): 4761; Libraty et al, PLoS Medicine (2009) 6 (10); Gunther et al, Vaccine (2011) 29: 3895) and Endy et al, J. Infect. Dis. (2004), 189(6): 990-1000).

The term "CCID₅₀" refers to the quantity of virus (e.g. vaccinal virus) infecting 50% of the cell culture. The CCID₅₀ assay is a limit dilution assay with statistical titer calculation *(*Morrison D et al J Infect Dis. 2010; 201(3):370-7*)).*

The term "human subject" is intended to mean males and females of various ages. Preferably a human subject according to the present invention is less than 18 years of age or less than 12 years of age. For example, a human subject according to the present invention may be 0-17 years of age, 0-11 years of age, 4-17 years of age, 4-11 years of age, 4-6 years of age, 6-8 years of age, 8-10 years of age, 2-8 years of age, 2-11 years of age, 2-14 years of age, 9-16 years of age, 12-17 years of age or 18-45 years of age. More preferably, a human subject according to the present invention is 4-11 years of age, 2-14 years of age or 9-16 years of age. A human subject according to the present invention may be at least 9 months old or less than 9 months old. For instance a human subject according to the present invention may be 9 months to 16 years of age, 9 months to 14 years of age, 9 months to 11 years of age or 9 months to 8 years of age. A human subject according to the present invention may be at least 9 months old, with no history of severe allergy to any component of the vaccine composition as defined herein, no congenital or acquired immune deficiency, no symptomatic HIV infection and said subject should not be pregnant or breast feeding.

As used herein, the expression "flavivirus-naïve subject" refers to a subject who has not been infected by a flavivirus nor previously immunized with a flavivirus vaccine, i.e. a serum sample taken from said subject will produce a negative result in a flavivirus ELISA or PRNT assay.

As used herein, the expression "dengue-naïve subject" refers to a subject who has not been infected by a dengue virus nor previously immunized with a dengue vaccine, i.e. a serum sample taken from said subject will produce a negative result in a dengue ELISA or PRNT assay.

As used herein, the expression "flavivirus-immune subject" refers to a subject who has been infected or immunized by a flavivirus before administration of the vaccine composition of the invention, i.e. a serum sample taken from said subject will produce a positive result in a flavivirus ELISA or PRNT assay.

As used herein, the expression "dengue-immune subject" refers to a subject who has been infected by a dengue virus or immunized by a dengue vaccine before administration of the vaccine composition of the present invention, i.e. a serum sample taken from said subject will produce a positive result in a dengue ELISA or PRNTassay.

In accordance with the present invention, a "method of protecting", as used herein, results in a reduction in the severity or in the likelihood of developing dengue disease in a human subject exposed to a dengue virus. Advantageously, said reduction is statistically significant. For example, a method of protecting, according to the present invention, may result in a reduction in at least one symptom of dengue disease as defined herein or a reduction in a combination of any two or more of those symptoms. The protection may result in any one or more of the following:
(i) a statistically significant reduction in the incidence or likelihood of, e.g. the prevention of, symptomatic virologically-confirmed dengue disease caused by dengue virus of any serotype;
(ii) a statistically significant reduction in the incidence or likelihood of, e.g. the prevention of, symptomatic virologically-confirmed dengue disease caused by dengue virus of any one of serotypes 1, 3 or 4;
(iii) a statistically significant reduction in the incidence or likelihood of, e.g. the prevention of, symptomatic dengue disease caused by dengue virus of any serotype;
(iv) a statistically significant reduction in the incidence or likelihood of, e.g. the prevention of, symptomatic dengue disease caused by dengue virus of any one of serotypes 1, 3 or 4;
(v) a statistically significant reduction in the incidence or likelihood of, e.g. the prevention of, severe virologically-confirmed dengue caused by dengue virus of any serotype;
(vi) a statistically significant reduction in the incidence or likelihood of, e.g. the prevention of, severe dengue disease caused by dengue virus of any serotype;
(vii) a statistically significant reduction in the incidence or likelihood of, e.g. the prevention of, dengue hemorrhagic fever cases of Grades I to IV caused by dengue virus of any serotype;
(viii) a statistically significant reduction in the incidence or likelihood of, e.g. the prevention of, DHF cases of Grade I caused by dengue virus of any serotype;
(ix) a statistically significant reduction in the incidence or likelihood of, e.g. the prevention of, DHF cases of Grade II caused by dengue virus of any serotype;
(x) a statistically significant reduction in the incidence or likelihood of, e.g. the prevention of, DHF cases of Grade III caused by dengue virus of any serotype;
(xi) a statistically significant reduction in the incidence or likelihood of, e.g. the prevention of, DHF cases of Grade IV caused by dengue virus of any serotype;
(xii) a statistically significant reduction in the incidence or likelihood of, e.g. the prevention of, fever or a reduction in the mean duration and/or intensity of fever;
(xiii) a statistically significant reduction in the incidence or likelihood of, e.g. the prevention of, plasma leakage as defined by a change in haematocrit or a reduction in the mean value for plasma leakage as defined by a change in haematocrit;
(xiv) a statistically significant reduction in the incidence or likelihood of, e.g. the prevention of, thrombocytopenia or a reduction in the mean value for thrombocytopenia;
(xv) a statistically significant reduction in the incidence or likelihood of, e.g. the prevention of, increases in the level of liver enzymes including alanine aminotransferase (ALT) and aspartate aminotransferase (AST);
(xvi) a statistically significant reduction in the incidence or likelihood of, e.g. the prevention of, hospitalization due to virologically-confirmed dengue disease caused by dengue virus of any serotype;
(xvii) a statistically significant reduction in the incidence or likelihood of, e.g. the prevention of, hospitalization due to dengue disease caused by dengue virus of any serotype;
(xviii) a statistically significant reduction in the length of hospital stay due to virologically-confirmed dengue disease.
(xix) a statistically significant reduction in the length of hospital stay due to dengue disease.

The duration and intensity of fever are monitored and recorded according to standard hospital procedures. In a human subject, a fever (i.e. a febrile episode) is defined as the observance of two temperature readings of at least 37.5°C measured twice over an interval of at least 4 hours. Measurements of haematocrit, thrombocytopenia and hepatic enzyme levels are standard tests well-known to the person of skill in the art, for example as described in the pharmacopea.

Protection against dengue disease, for example as defined in points (i) to (xix) above, may be demonstrated in respect of dengue disease caused by a particular dengue virus serotype. For example, protection against dengue disease, as defined herein, may be demonstrated in respect of dengue disease caused by a dengue virus of serotype 1, a dengue virus of serotype 2, a dengue virus of serotype 3 or a dengue virus of serotype 4. Advantageously, protection against dengue disease, as defined herein, may be demonstrated in respect of dengue disease caused by, for example, dengue virus of serotype 1 or serotype 3, dengue virus of serotype 1 or serotype 4, dengue virus of serotype 3 or serotype 4, dengue virus of serotype 1 or serotype 2, dengue virus of serotype 2 or serotype 3, dengue virus of serotype 2 or serotype 4, dengue virus of serotype 1, 2 or 3, dengue virus of serotype 1, 3 or 4, dengue virus of serotype 2, 3 or 4 or dengue virus of serotype 1, 2, 3 or 4.

Protection against dengue disease, as defined herein, may advantageously be demonstrated in particular sub-groups of human subjects. For instance, protection against dengue disease may advantageously be demonstrated in a human subject who is less than 18 years of age or less than 12 years of age. For example, a human subject according to the present invention may be 0-17 years of age, 0-11 years of age, 4-17 years of age, 4-11 years of age, 4-6 years of age, 6-8 years of age, 8-10 years of age, 2-8 years of age, 2-11 years of age, 2-14 years of age, 9-16 years of age, 12-17 years of age or 18-45 years of age. More preferably, a human subject according to the present invention is 4-11 years of age, 2-14 years of age or 9-16 years of age. A human subject according to the present invention may be at least 9 months old or less than 9 months old. For instance a human subject according to the present invention may be 9 months to 16 years of age, 9 months to 14 years of age, 9 months to 11 years of age or 9 months to 8 years of age. A human subject according to the present invention may be at least 9 months old, with no history of severe allergy to any component of the vaccine composition as defined herein, no congenital or acquired immune deficiency, no symptomatic HIV infection and said subject should not be pregnant or breast feeding.

Protection against dengue disease, as defined herein, may advantageously be demonstrated in particular countries, areas or regions of the world. For instance, protection against dengue disease may advantageously be demonstrated in a dengue endemic area. For instance, a dengue endemic area according to the present invention in which protection may be demonstrated may comprise those American countries or parts thereof which fall within the tropics and sub-tropics. A dengue endemic area in which protection may be demonstrated according to the present invention may thus comprise any one or more of the following: Brazil, Venezuela, Colombia, Ecuador, Peru, Bolivia, Paraguay, Panama, Costa Rica, Nicaragua, Honduras, El Salvador, Guatemala, Belize, Mexico, the USA and the islands of the Caribbean. In a particular embodiment, a dengue endemic area of the present invention in which protection may be demonstrated may consist of the following: Brazil, Colombia, Honduras, Mexico and Puerto Rico. A dengue endemic area in which protection may be demonstrated according to the present invention may also include south Asian and Oceania countries within the tropics and sub-tropics. A dengue endemic area according to the present invention in which protection may be demonstrated may thus consist of any one or more of the following: India, Myanmar (Burma), Thailand, Laos, Vietnam, Cambodia, Indonesia, Malaysia, Singapore, the Philippines, Taiwan, Papua New Guinea and Australia. In a dengue endemic area in which protection may be demonstrated according to the present invention, a particular serotype, strain or genotype of wild type dengue virus may be the dominant circulating strain. For example, a dengue virus of serotype 2 may be characterised as having an Asian I or an Asian/American genotype. Asian/American genotype strains are characterised by at least one of, at least two of, at least three of, at least four of, at least five of or all six of the following residues Arg, Asn, Asp, Thr, Gly and His at positions prM-16, E-83, E-203, E-226, E-228 and E-346 respectively (wherein prM-16 designates position 16 of the prM protein and E-83 etc. designates position 83 of the E protein). Asian I genotype strains are characterised by at least one of, at least two of, at least three of, at least four of, at least five of or all six of the following residues Ile, Lys, Asn, Arg, Glu and Tyr at positions prM-16, E-83, E-203, E-226, E-228 and E-346 respectively (see Table 1 of Hang et al., PLoS NTD, 4(7): e757). A preferred dengue endemic area in which protection may be demonstrated according to the present invention is one in which a dengue virus having an Asian/American genotype is the dominant circulating strain, i.e. at least 50%, at least 60%, at least 70%, at least 80%, at least 90%, at least 95% or 100% of the cases of dengue disease in said dengue endemic area are caused by dengue virus having an Asian/American genotype. A preferred dengue endemic area in which protection may be demonstrated according to the present invention is one in which a dengue virus of any one or more of serotypes 1, 3 or 4 is/are the dominant circulating serotype(s), i.e. at least 50%, at least 60%, at least 70%, at least 80%, at least 90%, at least 95% or 100% of the cases of dengue disease are caused by dengue virus of serotypes 1, 3 or 4.

The term "RNA equivalent" of a given DNA sequence, as used herein, refers to a sequence wherein deoxythymidines have been replaced by uridines. Since the DNA sequences in question constitute the cDNA sequences of the dengue viruses, the equivalent RNA sequences constitute the positive strand RNA of those dengue viruses.

### Overview of Several Embodiments

The present inventors have, for the first time, demonstrated the efficacy of a vaccine composition in protecting a human subject against dengue disease.

Accordingly, the present invention relates to a vaccine composition for use in a method of protecting a human subject against dengue disease, wherein said composition comprises:
(i) a dengue antigen selected from the group consisting of:
   (a) a live attenuated dengue virus;
   (b) an inactivated dengue virus;
   (c) a live attenuated or inactivated chimeric dengue virus;
   (d) a dengue virus-like particle (VLP); and
   (e) a combination of two or more of (a) to (d);
   or
(ii) a nucleic acid construct or viral vector which is able to express in a human cell a dengue antigen which is a dengue VLP.

Preferably the dengue disease according to the present invention is virologically-confirmed dengue disease.

Preferably a human subject according to the present invention is less than 18 years of age or less than 12 years of age. For example, a human subject according to the present invention may be 0-17 years of age, 0-11 years of age, 4-17 years of age, 4-11 years of age, 4-6 years of age, 6-8 years of age, 8-10 years of age, 2-8 years of age, 2-11 years of age, 2-14 years of age, 9-16 years of age, 12-17 years of age or 18-45 years of age. More preferably, a human subject according to the present invention is 4-11 years of age, 2-14 years of age or 9-16 years of age. A human subject according to the present invention may be at least 9 months old or less than 9 months old. For instance a human subject according to the present invention may be 9 months to 16 years of age, 9 months to 14 years of age, 9 months to 11 years of age or 9 months to 8 years of age. A human subject according to the present invention may be at least 9 months old, with no history of severe allergy to any component of the vaccine composition as defined herein, no congenital or acquired immune deficiency, no symptomatic HIV infection and said subject should not be pregnant or breast feeding.

A subject to which a vaccine composition of the present invention is to be administered is preferably a person at risk of infection, for instance a person travelling in an area where dengue fever is present, i.e. a dengue endemic area, or a resident of such an area. Dengue endemic areas according to the present invention include most of the tropics and sub-tropics, for instance any country identified as an endemic country by the WHO. For instance, a dengue endemic area according to the present invention may comprise those American countries or parts thereof which fall within the tropics and sub-tropics. A dengue endemic area according to the present invention may thus comprise any one or more of the following: Brazil, Venezuela, Colombia, Ecuador, Peru, Bolivia, Paraguay, Panama, Costa Rica, Nicaragua, Honduras, El Salvador, Guatemala, Belize, Mexico, the USA and the islands of the Caribbean. In a particular embodiment, a dengue endemic area of the present invention may consist of the following: Brazil, Colombia, Honduras, Mexico and Puerto Rico. A dengue endemic area according to the present invention may also include south Asian and Oceania countries within the tropics and sub-tropics. A dengue endemic area according to the present invention may thus consist of any one or more of the following: India, Myanmar (Burma), Thailand, Laos, Vietnam, Cambodia, Indonesia, Malaysia, Singapore, the Philippines, Taiwan, Papua New Guinea and Australia. In a dengue endemic area according to the present invention, a particular serotype, strain or genotype of wild type dengue virus may be the dominant circulating strain. For example, a dengue virus of serotype 2 may be characterised as having an Asian I or an Asian/American genotype. Asian/American genotype strains are characterised by at least one of, at least two of, at least three of, at least four of, at least five of or all six of the following residues Arg, Asn, Asp, Thr, Gly and His at positions prM-16, E-83, E-203, E-226, E-228 and E-346 respectively (wherein prM-16 designates position 16 of the prM protein and E-83 etc. designates position 83 of the E protein). Asian I genotype strains are characterised by at least one of, at least two of, at least three of, at least four of, at least five of or all six of the following residues Ile, Lys, Asn, Arg, Glu and Tyr at positions prM-16, E-83, E-203, E-226, E-228 and E-346 respectively (see Table 1 of Hang et al., PLoS NTD, 4(7): e757). A preferred dengue endemic area according to the present invention is one in which a dengue virus having an Asian/American genotype is the dominant circulating strain, i.e. at least 50%, at least 60%, at least 70%, at least 80%, at least 90%, at least 95% or 100% of the cases of dengue disease in said dengue endemic area are caused by dengue virus having an Asian/American genotype. A preferred dengue endemic area according to the present invention is one in which a dengue virus of any one or more of serotypes 1, 3 or 4 is/are the dominant circulating serotype(s), i.e. at least 50%, at least 60%, at least 70%, at least 80%, at least 90%, at least 95% or 100% of the cases of dengue disease are caused by dengue virus of serotypes 1, 3 or 4.

A vaccine composition of the present invention may be administered to a flavivirus immune subject, for example a dengue-immune subject, or a vaccine composition of the present invention may be administered to a flavivirus-naive subject. Advantageously, a vaccine composition of the present invention is administered to a flavivirus-immune subject, for example a dengue-immune subject.

Preferably, a composition according to the present invention, e.g. a composition for use in a method according to the present invention, reduces the likelihood or severity of DHF. A reduction in the likelihood of DHF (i.e. a reduction in the probability of contracting DHF) may be measured by comparing the number of cases of DHF in a group of subjects who have received a vaccine composition according to the present invention and the number of cases of DHF in a control group of subjects who have not received a vaccine composition according to the present invention. A reduction in the severity of DHF may be determined by calculating the number of subjects displaying DHF of each of Grades I, II, III or IV in a group of subjects who have received a vaccine composition according to the present invention and comparing those numbers to the equivalent numbers from a control group of subjects who have not received a vaccine composition according to the present invention. For instance, a composition for use in a method according to the present invention preferably reduces the number of cases of Grade I DHF, the number of cases of Grade II DHF, the number of cases of Grade III DHF and/or the number of cases of Grade IV DHF in those subjects receiving the vaccine, when compared to the equivalent number of cases Grade I DHF, Grade II DHF, Grade III DHF and Grade IV DHF occurring in a control group of subjects who have not received a vaccine composition according to the present invention.

Preferably, a composition according to the present invention, e.g. a composition for use in a method according to the present invention, reduces the incidence or likelihood of symptomatic virologically-confirmed dengue disease. Advantageously, a composition according to the present invention, e.g. a composition for use in a method according to the present invention, reduces the incidence or likelihood of symptomatic virologically-confirmed dengue disease caused by dengue virus of serotypes 1, 3 or 4. Preferably, a composition according to the present invention, e.g. a composition for use in a method according to the present invention, reduces the rate of hospitalization due to virologically-confirmed dengue disease, i.e. reduces the incidence of hospitalized virologically-confirmed dengue disease. For instance, a composition according to the present invention, e.g. a composition for use in a method according to the present invention, reduces the rate of hospitalization due to virologically-confirmed dengue disease caused by dengue virus of serotypes 1, 3 or 4, i.e. reduces the incidence of hospitalized virologically-confirmed dengue disease caused by dengue virus of serotypes 1, 3 or 4.

Preferably, a composition according to the present invention, e.g. a composition for use in a method according to the present invention, reduces the incidence or likelihood of dengue disease. Advantageously, a composition according to the present invention, e.g. a composition for use in a method according to the present invention, reduces the incidence or likelihood of dengue disease caused by dengue virus of serotypes 1, 3 or 4. Advantageously, a composition according to the present invention, e.g. a composition for use in a method according to the present invention, reduces the incidence or likelihood of dengue disease caused by dengue virus of serotypes 1, 2, 3 or 4. Preferably, a composition according to the present invention, e.g. a composition for use in a method according to the present invention, reduces the rate of hospitalization due to dengue disease, i.e. reduces the incidence of hospitalized dengue disease. For instance, a composition according to the present invention, e.g. a composition for use in a method according to the present invention, reduces the rate of hospitalization due to dengue disease caused by dengue virus of serotypes 1, 3 or 4, i.e. reduces the incidence of hospitalized dengue disease caused by dengue virus of serotypes 1, 3 or 4.

A vaccine composition according to the present invention may be administered in multiple doses. Doses of a vaccine composition according to the present invention may be administered in an initial vaccination regimen followed by booster vaccinations. For example, a vaccine composition according to the present invention may be administered in one, two or three doses or more than three doses, e.g. four doses. Preferably, the first dose and the third dose are to be administered approximately twelve months apart. For example, an initial vaccination regimen according to the present invention is administered in three doses, wherein the first and third doses of said vaccination regimen are to be administered approximately twelve months apart. Advantageously, a vaccine composition according to the present invention is to be administered in a first dose, a second dose and a third dose. In such an embodiment, said first dose and said third dose may be administered approximately twelve months apart. For instance, a vaccine composition of the present invention may be administered in a first dose, a second dose and a third dose, wherein said second dose is to be administered about six months after said first dose and wherein said third dose is to be administered about twelve months after said first dose. Alternatively, the three doses may be administered at zero months, at about three to four months (e.g. at about three-and-a-half months) and at about twelve months (i.e. a regimen wherein the second dose of the composition is administered at about three-and-a-half months after the first dose, and wherein the third dose of the composition is administered at about twelve months after the first dose).

A vaccine composition according to the present invention may be administered in two doses. Preferably, the first dose and the second dose are to be administered approximately about six to twelve months after the first dose months apart. Preferably, the second dose is to be administered at eight months after the first dose. Preferably the second dose is administered at about eight-and-a-half to nine months after the first dose.

A vaccine composition according to the present invention may be administered in a single dose.

Dengue disease, as defined herein, may be caused by any one of two serotypes of a dengue virus. For example, dengue disease is preferably caused by a dengue virus of serotype 1 or serotype 3, a dengue virus of serotype 1 or serotype 4, a dengue virus of serotype 3 or serotype 4, a dengue virus of serotype 1 or serotype 2, a dengue virus of serotype 2 or serotype 3, a dengue virus of serotype 2 or serotype 4. Dengue disease, as defined herein, is preferably caused by any one of three serotypes of a dengue virus. For example, dengue disease is preferably caused by a dengue virus of serotype 1, 2 or 3, a dengue virus of serotype 1, 3 or 4, a dengue virus of serotype 1, 2 or 4, a dengue virus of serotype 2, 3 or 4. In another embodiment, dengue disease is caused by a dengue virus of serotype 1, a dengue virus of serotype 2, a dengue virus of serotype 3 or a dengue virus of serotype 4.

A vaccine composition according to the present invention, e.g. for use in a method according to the present invention preferably comprises a dengue antigen of serotype 1, a dengue antigen of serotype 2, a dengue antigen of serotype 3 and a dengue antigen of serotype 4. Such a composition may be described herein as a tetravalent composition. For instance, a composition of the present invention, e.g. for use in a method of protecting according to the present invention, may advantageously comprise any one of the following combinations of dengue antigens of serotypes 1, 2, 3 and 4: i) a dengue antigen comprising the prM and E sequences of CYD-1, a dengue antigen comprising the prM and E sequences of CYD-LAV, a chimeric dengue virus comprising the prM and E sequences of CYD-3 and a dengue antigen comprising the prM and E sequences of CYD-4; ii) a dengue antigen comprising the prM and E sequences of CYD-1, a dengue antigen comprising the prM and E sequences of CYD-BID, a dengue antigen comprising the prM and E sequences of CYD-3 and a dengue antigen comprising the prM and E sequences of CYD-4; (iii) a dengue antigen comprising the prM and E sequences of CYD-1, a dengue antigen comprising the prM and E sequences of CYD-PR, a dengue antigen comprising the prM and E sequences of CYD-3 and a dengue antigen comprising the prM and E sequences of CYD-4; (iv) a dengue antigen comprising the prM and E sequences of CYD-1, a dengue antigen comprising the prM and E sequences of CYD-MD, a dengue antigen comprising the prM and E sequences of CYD-3 and a dengue antigen comprising the prM and E sequences of CYD-4;. For instance, a composition of the present invention may also advantageously comprise any one of the following combinations of dengue antigens: i) CYD-1, CYD-LAV, CYD-3 and CYD-4; ii) CYD-1, CYD-BID, CYD-3 and CYD-4; (iii) CYD-1, CYD-PR, CYD-3 and CYD-4 or (iv) CYD-1, CYD-MD, CYD-3 and CYD-4. A composition of the present invention may also advantageously comprise the following combination of dengue antigens: i) a dengue antigen comprising the prM and E sequences of CYD-1, VDV2, a dengue antigen comprising the prM and E sequences of CYD-3 and a dengue antigen comprising the prM and E sequences of CYD-4. For instance, a composition of the present invention may advantageously comprise CYD-1, VDV-2, CYD-3 and CYD-4. A composition of the present invention, as described herein, may advantageously comprise a dengue antigen of serotype 2 which comprises the prM-E sequence of CYD-LAV (SEQ ID NO: 8), CYD-BID (SEQ ID NO: 9), CYD-PR (SEQ ID NO: 10) CYD-MD (SEQ ID NO: 11) or SEQ ID NO: 2. A composition of the present invention, as described herein, may advantageously comprise a dengue antigen of serotype 2 which comprises a sequence having at least 90% identity to the prM-E sequence of CYD-LAV (SEQ ID NO: 8), CYD-BID (SEQ ID NO: 9), CYD-PR (SEQ ID NO: 10) CYD-MD (SEQ ID NO: 11) or SEQ ID NO: 2. For example, said sequence may be at least 91%, at least 92%, at least 93%, at least 94%, at least 95%, at least 96%, at least 97%, at least 98% or at least 99% identical to the prm-E sequence of CYD-LAV (SEQ ID NO: 8), CYD-BID (SEQ ID NO: 9), CYD-PR (SEQ ID NO: 10) CYD-MD (SEQ ID NO: 11) or SEQ ID NO: 2.

A vaccine composition according to the present invention, e.g. for use in a method according to the present invention, preferably comprises a dengue antigen of serotype 1, a dengue antigen of serotype 2, a dengue antigen of serotype 3 and a dengue antigen of serotype 4. Such a composition may be described herein as a tetravalent composition. For instance, a composition of the present invention, e.g. for use in a method of protecting according to the present invention, may advantageously comprise any one of the following combinations of dengue antigens of serotypes 1, 2, 3 and 4: i) a dengue antigen comprising the M and E sequences of CYD-1, a dengue antigen comprising the M and E sequences of CYD-LAV, a chimeric dengue virus comprising the M and E sequences of CYD-3 and a dengue antigen comprising the M and E sequences of CYD-4; ii) a dengue antigen comprising the M and E sequences of CYD-1, a dengue antigen comprising the M and E sequences of CYD-BID, a dengue antigen comprising the M and E sequences of CYD-3 and a dengue antigen comprising the M and E sequences of CYD-4; (iii) a dengue antigen comprising the M and E sequences of CYD-1, a dengue antigen comprising the M and E sequences of CYD-PR, a dengue antigen comprising the M and E sequences of CYD-3 and a dengue antigen comprising the M and E sequences of CYD-4; (iv) a dengue antigen comprising the M and E sequences of CYD-1, a dengue antigen comprising the M and E sequences of CYD-MD, a dengue antigen comprising the M and E sequences of CYD-3 and a dengue antigen comprising the M and E sequences of CYD-4;. For instance, a composition of the present invention may also advantageously comprise any one of the following combinations of dengue antigens: i) CYD-1, CYD-LAV, CYD-3 and CYD-4; ii) CYD-1, CYD-BID, CYD-3 and CYD-4; (iii) CYD-1, CYD-PR, CYD-3 and CYD-4 or (iv) CYD-1, CYD-MD, CYD-3 and CYD-4. A composition of the present invention may also advantageously comprise the following combination of dengue antigens: i) a dengue antigen comprising the M and E sequences of CYD-1, VDV2, a dengue antigen comprising the M and E sequences of CYD-3 and a dengue antigen comprising the M and E sequences of CYD-4. For instance, a composition of the present invention may advantageously comprise CYD-1, VDV-2, CYD-3 and CYD-4. A composition of the present invention, as described herein, may advantageously comprise a dengue antigen of serotype 2 which comprises the E sequence of CYD-LAV (SEQ ID NO: 13), CYD-BID (SEQ ID NO: 14), CYD-PR (SEQ ID NO: 15) CYD-MD (SEQ ID NO: 16) or SEQ ID NO: 18. A composition of the present invention, as described herein, may advantageously comprise a dengue antigen of serotype 2 which comprises a sequence having at least 90% identity to the E sequence of CYD-LAV (SEQ ID NO: 13), CYD-BID (SEQ ID NO: 14), CYD-PR (SEQ ID NO: 15) CYD-MD (SEQ ID NO: 16) or SEQ ID NO: 18. For example, said sequence may be at least 91%, at least 92%, at least 93%, at least 94%, at least 95%, at least 96%, at least 97%, at least 98% or at least 99% identical to the E sequence of CYD-LAV (SEQ ID NO: 13), CYD-BID (SEQ ID NO: 14), CYD-PR (SEQ ID NO: 15) CYD-MD (SEQ ID NO: 16) or SEQ ID NO: 18.

A composition of the present invention, as described herein, (e.g. a tetravalent formulation, e.g. for use in a method of the present invention), may advantageously comprise a dengue antigen of serotype 2 which comprises a polypeptide selected from the group consisting of SEQ ID NO: 19, SEQ ID NO: 20, SEQ ID NO: 21, SEQ ID NO: 22 or SEQ ID NO: 23. Preferably said dengue antigen of serotype 2 further comprises a polypeptide selected from the group consisting of SEQ ID NO: 13, SEQ ID NO: 14, SEQ ID NO: 15, SEQ ID NO: 16 or SEQ ID NO: 18. For instance, said dengue antigen of serotype 2 preferably comprises: i) a polypeptide of SEQ ID NO: 13 and a polypeptide of SEQ ID NO: 19; ii) a polypeptide of SEQ ID NO: 14 and a polypeptide of SEQ ID NO: 20; iii) a polypeptide of SEQ ID NO: 15 and a polypeptide of SEQ ID NO: 21; iv) a polypeptide of SEQ ID NO: 16 and a polypeptide of SEQ ID NO: 22; or v) a polypeptide of SEQ ID NO: 18 and a polypeptide of SEQ ID NO: 23.

A composition of the present invention, as described herein (e.g. a tetravalent formulation, e.g. for use in a method of the present invention), may advantageously comprise a dengue antigen of serotype 2 which comprises a polypeptide having at least 90% identity to SEQ ID NO: 19, SEQ ID NO: 20, SEQ ID NO: 21, SEQ ID NO: 22 or SEQ ID NO: 23. Preferably said dengue antigen of serotype 2 further comprises a polypeptide having at least 90% identity to SEQ ID NO: 13, SEQ ID NO: 14, SEQ ID NO: 15, SEQ ID NO: 16 or SEQ ID NO: 18. For instance, said dengue antigen of serotype 2 preferably comprises: i) a polypeptide having at least 90% sequence identity to SEQ ID NO: 13 and a polypeptide having at least 90% sequence identity to SEQ ID NO: 19; ii) a polypeptide having at least 90% sequence identity to SEQ ID NO: 14 and a polypeptide having at least 90% sequence identity to SEQ ID NO: 20; iii) a polypeptide having at least 90% sequence identity to SEQ ID NO: 15 and a polypeptide having at least 90% sequence identity to SEQ ID NO: 21; iv) a polypeptide having at least 90% sequence identity to SEQ ID NO: 16 and a polypeptide having at least 90% sequence identity to SEQ ID NO: 22; or v) a polypeptide having at least 90% sequence identity to SEQ ID NO: 18 and a polypeptide having at least 90% sequence identity to SEQ ID NO: 23. In preferred embodiments, the references herein to at least 90% identity may be read as at least 91%, at least 92%, at least 93%, at least 94%, at least 95%, at least 96%, at least 97%, at least 98% or at least 99% identity to the given sequence.

The dengue antigens of serotype 2 as described in the preceding paragraphs may advantageously be combined with any of the dengue antigens of serotypes 1, 3 and 4 as described elsewhere herein to form a tetravalent formulation comprising a dengue antigen of serotype 1, a dengue antigen of serotype 2, a dengue antigen of serotype 3 and a dengue antigen of serotype 4. For example, said dengue antigens of serotypes 1, 2, 3 and 4 may each be independently selected from a live attenuated dengue virus or a live attenuated chimeric dengue virus. Advantageously, a composition for use in the method of the present invention comprises a dengue antigen of each of serotypes 1, 2, 3 and 4, wherein said dengue antigens of serotypes 1, 2, 3 and 4 are each a live attenuated chimeric dengue virus. Advantageously, a composition for use in the method of the present invention comprises a dengue antigen of each of serotypes 1, 2, 3 and 4, wherein said dengue antigens of serotypes 1, 3 and 4 are each a live attenuated chimeric dengue virus and said dengue antigen of serotype 2 is selected from the group consisting of a live attenuated dengue virus and a live attenuated chimeric dengue virus.

Advantageously, a composition for use in the method of the present invention comprises a dengue antigen of each of serotypes 1, 2, 3 and 4, wherein said dengue antigens of serotypes 1, 3 and 4 are each a live attenuated chimeric dengue virus and said dengue antigen of serotype 2 is a live attenuated dengue virus. For example, said live attenuated chimeric dengue viruses of serotypes 1, 3 and 4 may be dengue/dengue chimeras such as dengue/dengue chimeras based on the DEN-2 16681/PDK53 strain (also known as LAV2). For example said dengue/dengue chimeras of serotypes 1, 3 and 4 may be chimeric viruses in which the genome of the DEN-2 16681/PDK53 strain is modified such that the prM and E genes of the DEN-2 16681/PDK53 strain are replaced with the prM and E genes of a serotype 1 strain, a serotype 2 strain and a serotype 3 strain respectively. Such chimeric dengue/dengue viruses may be referred to as DEN-2/1, DEN-2/3 and DEN-2/4 respectively. In this context, the dengue antigen of serotype 2 which is a live attenuated virus may be the DEN-2 16681/PDK53 strain (also known as LAV2).

It is an aim of the present inventors to provide an optimized tetravalent dengue vaccine composition (i.e. vaccine composition comprising a dengue antigen of each of serotypes 1, 2, 3 and 4) for use in the method of the present invention, which provides an improved neutralising antibody response against dengue virus of serotype 2 when compared with the neutralising antibody response generated by CYD-1, CYD-2, CYD-3 and CYD-4 as defined in Example 1.

Accordingly, in one aspect, the present invention advantageously provides a vaccine composition for use in the method of the present invention wherein said composition comprises a dengue antigen of each of serotypes 1, 2, 3 and 4, wherein said dengue antigens of serotypes 1, 3 and 4 are each a live attenuated chimeric dengue virus and said dengue antigen of serotype 2 is a live attenuated dengue virus which comprises a nucleic acid sequence having at least 90% sequence identity to the sequence as set forth in SEQ ID NO: 24.

Accordingly, in another aspect, the present invention advantageously provides a vaccine composition for use in a method according to the present invention, which comprises a dengue antigen of serotype 1, a dengue antigen of serotype 2, a dengue antigen of serotype 3 and a dengue antigen of serotype 4, wherein:
i) said dengue antigen of serotype 1 is a YF/dengue chimeric dengue virus (i.e. a recipient yellow fever virus in which the genetic backbone of the YF virus has been modified by exchanging the sequences encoding the prM and E proteins of the YF virus by the corresponding sequences of a dengue serotype 1 virus);
ii) said dengue antigen of serotype 2 is a live attenuated dengue virus of serotype 2 which comprises a nucleic acid sequence having at least 90% sequence identity to the sequence as set forth in SEQ ID NO: 24;
iii) said dengue antigen of serotype 3 is a YF/dengue chimeric dengue virus (i.e. a recipient yellow fever virus in which the genetic backbone of the YF virus has been modified by exchanging the sequences encoding the prM and E proteins of the YF virus by the corresponding sequences of a dengue serotype 3 virus) and
iv) said dengue antigen of serotype 4 is a YF/dengue chimeric dengue virus (i.e. a recipient yellow fever virus in which the genetic backbone of the YF virus has been modified by exchanging the sequences encoding the prM and E proteins of the YF virus by the corresponding sequences of a dengue serotype 4 virus).

Preferably, said recipient YF virus (which forms the genetic backbone of the YF/dengue chimeric viruses of serotypes 1, 3 and 4) is an attenuated YF virus. For example, said recipient YF virus may be an attenuated YF virus selected from the group consisting of YF 17D, YF 17DD and YF 17D204. Preferably, the YF/dengue chimeric viruses of serotypes 1, 3 and 4 are respectively a Chimerivax dengue serotype 1 (i.e. a CYD-1), a Chimerivax dengue serotype 3 (i.e. a CYD-3) and a Chimerivax dengue serotype 4 (i.e. a CYD-4).

A reference herein to a nucleic acid sequence having at least 90% sequence identity to the sequence as set forth in SEQ ID NO: 24 may preferably be read as a nucleic acid sequence having at least 92%, at least 94%, at least 96%, at least 98%, at least 99% or 100% sequence identity to the sequence as set forth in SEQ ID NO: 24. Preferably the nucleotides at the positions within said nucleic acid sequences (that have at least 90% sequence identity to the sequence as set forth in SEQ ID NO: 24) which correspond to positions 736, 1619, 4723, 5062, 9191, 10063, 10507, 57, 524, 2055, 2579, 4018, 5547, 6599 and 8571 of SEQ ID NO: 24 are not mutated. Advantageously, a dengue antigen of serotype 2 which is a live attenuated dengue virus for use in a composition of the present invention (for example for use in combination with a dengue antigen of serotypes 1, 3 and 4 as described above and elsewhere herein (e.g. dengue antigens of serotypes 1, 3 and 4 which are live attenuated chimeric dengue viruses, e.g. YF/dengue chimeric dengue viruses)) is a live attenuated dengue virus which comprises a nucleic acid sequence having 100% sequence identity to the sequence as set forth in SEQ ID NO: 24 or a live attenuated dengue virus which comprises at least one and no more than 20 nucleotide substitutions when compared with the sequence as set forth in SEQ ID NO: 24. Preferably said live attenuated dengue virus comprises at least one and no more than 15, 14, 13, 12, 11, 10, 9, 8, 7, 6, 5, 4, 3 or 2 nucleotide substitutions when compared with the sequence as set forth in SEQ ID NO: 24. Preferably the nucleotides at the positions within said nucleic acid sequences which correspond to positions 736, 1619, 4723, 5062, 9191, 10063, 10507, 57, 524, 2055, 2579, 4018, 5547, 6599 and 8571 of SEQ ID NO: 24 are not mutated. Advantageously, a dengue antigen of serotype 2 which is a live attenuated dengue virus for use in a composition of the present invention comprises a nucleic acid sequence that has no more than 20 base mutations, deletions or insertions when compared with the sequence as set forth in SEQ ID NO: 24. In certain cases said live attenuated dengue virus of serotype 2 comprises a nucleic acid sequence that has no more than 15 or even no more than 12, 11, 10, 9, 8, 7, 6, 5, 4, 3, 2 or 1 base mutations, deletions or insertions when compared with the sequence as set forth in SEQ ID NO: 24. Preferably the nucleotides at the positions within said nucleic acid sequence that correspond to positions 736, 1619, 4723, 5062, 9191, 10063, 10507, 57, 524, 2055, 2579, 4018, 5547, 6599 and 8571 of SEQ ID NO: 24 are not mutated.

It is also preferred that a dengue antigen of serotype 2 for use in a vaccine composition of the present invention (e.g. a dengue antigen which is a live attenuated dengue virus or a live attenuated chimeric dengue virus of serotype 2) is capable of inducing neutralizing antibodies in humans and is capable of inducing a balanced immune response when used in the context of a tetravalent dengue vaccine composition. It is also preferred that a dengue antigen of serotype 2 for use in a vaccine composition of the present invention (e.g. a dengue antigen which is a live attenuated dengue virus or a live attenuated chimeric dengue virus of serotype 2) for use in a vaccine composition of the invention results in low or absent viremia in humans. It is also preferred that a dengue antigen of serotype 2 for use in a tetravalent vaccine composition of the present invention (e.g. a dengue antigen which is a live attenuated dengue virus or a live attenuated chimeric dengue virus of serotype 2) provides an improved neutralising antibody response against dengue virus of serotype 2 when compared with the neutralising antibody response generated by CYD-1, CYD-2, CYD-3 and CYD-4 as defined in Example 1.

Advantageously, a vaccine composition for use in the method of the present invention comprises a dengue antigen of each of serotypes 1, 2, 3 and 4, wherein said dengue antigens of serotypes 1, 3 and 4 are each a live attenuated chimeric dengue virus and said dengue antigen of serotype 2 is a live attenuated dengue virus which comprises a nucleic acid sequence having at least 90% sequence identity to the sequence as set forth in SEQ ID NO: 24 and wherein said dengue antigens of serotypes 1, 2, 3 and 4 are not CYD-1, VDV-2, CYD-3 and CYD-4 respectively or are not a dengue antigen comprising the M and E sequences of CYD1, VDV2, a dengue antigen comprising the M and E sequences of CYD3 and a dengue antigen comprising the M and E sequences of CYD4 respectively.

Advantageously, a vaccine composition for use in the method of the present invention comprises a dengue antigen of each of serotypes 1, 2, 3 and 4, wherein: (i) said dengue antigen of serotype 1 is a live attenuated chimeric dengue virus other than CYD-1 or said dengue antigen of serotype 1 is CYD-1; (ii) said dengue antigen of serotype 2 is a live attenuated dengue virus other than VDV-2 or said dengue antigen of serotype 2 is VDV-2; (iii) said dengue antigen of serotype 3 is a live attenuated chimeric dengue virus other than CYD-3 or said dengue antigen of serotype 3 is CYD-3 and (iv) said dengue antigen of serotype 4 is a live attenuated chimeric dengue virus other than CYD-4 or said dengue antigen of serotype 4 is CYD-4. In this context, the VDV-2 strain is the strain derived from the DEN-2 16681/PDK53 strain (LAV2) by subsequent adaptation to Vero cells, wherein said VDV-2 strain has 10 additional mutations in comparison with the DEN-2 16681/PDK53 strain including four silent mutations.

Advantageously, a composition for use in the method of the present invention comprises a dengue antigen of each of serotypes 1, 2, 3 and 4, wherein: (i) said dengue antigen of serotype 1 is a live attenuated chimeric dengue virus other than CYD-1 or said dengue antigen of serotype 1 is CYD-1; (ii) said dengue antigen of serotype 2 is a live attenuated dengue virus other than VDV-2 or said dengue antigen of serotype 2 is VDV-2; (iii) said dengue antigen of serotype 3 is a live attenuated chimeric dengue virus other than CYD-3 or said dengue antigen of serotype 3 is CYD-3 and (iv) said dengue antigen of serotype 4 is a live attenuated chimeric dengue virus other than CYD-4 or said dengue antigen of serotype 4 is CYD-4. In this context, the VDV-2 strain is the strain comprising the nucleic acid sequence as set forth in SEQ ID NO: 24.

A preferred vaccine composition for use in the method of the present invention comprises a dengue antigen of serotype 1, a dengue antigen of serotype 2, a dengue antigen of serotype 3 and a dengue antigen of serotype 4, wherein:
i) said dengue antigen of serotype 1 is a YF/dengue chimeric dengue virus other than a CYD-1 or said dengue antigen of serotype 1 is a CYD-1;
ii) said dengue antigen of serotype 2 is a live attenuated dengue virus of serotype 2 which comprises a nucleic acid sequence having at least 90% sequence identity to the sequence as set forth in SEQ ID NO: 24, wherein said dengue antigen of serotype 2 is not a live attenuated dengue virus of serotype 2 which comprises a nucleic acid sequence having 100% sequence identity to the sequence as set forth in SEQ ID NO: 24 or said dengue antigen of serotype 2 is a live attenuated dengue virus of serotype 2 which comprises a nucleic acid sequence having 100% sequence identity to the sequence as set forth in SEQ ID NO: 24;
iii) said dengue antigen of serotype 3 is a YF/dengue chimeric dengue virus other than a CYD-3 or said dengue antigen of serotype 3 is a CYD-3; and
iv) said dengue antigen of serotype 4 is a YF/dengue chimeric dengue virus other than a CYD-4 or said dengue antigen of serotype 4 is a CYD-4.

Advantageously, a dengue antigen of serotype 2 which is a live attenuated chimeric dengue virus for use in method vaccine composition of the present invention (for example for use in combination with a dengue antigen of serotypes 1, 3 and 4 as described above and elsewhere herein (e.g. dengue antigens of serotypes 1, 3 and 4 which are YF/dengue chimeric dengue viruses)) comprises a nucleic acid sequence having at least 90% identity to the sequence as set forth in SEQ ID NO: 25. Preferably said nucleic acid sequence has at least 92%, at least 94%, at least 96%, at least 98%, at least 99% or 100% sequence identity to the sequence as set forth in SEQ ID NO: 25. Preferably the nucleotides at the positions within said nucleic acid sequence which correspond to positions 524, 736, 1619 and 2055 of SEQ ID NO: 24 are not mutated (i.e. maintain the nucleotide appearing in SEQ ID NO: 24 at those positions).

A composition of the present invention, as described herein, may advantageously comprise a dengue antigen selected from the group consisting of: (a) a live attenuated dengue virus; (b) an inactivated dengue virus; (c) a live attenuated or inactivated chimeric dengue virus and (d) a combination of two or more of (a) to (c), wherein said dengue antigen comprises a nucleotide sequence selected from the group consisting of SEQ ID NO: 4, SEQ ID NO: 5, SEQ ID NO: 6, SEQ ID NO: 7 and SEQ ID NO: 1.

A composition of the present invention, as described herein, may advantageously comprise a dengue antigen selected from the group consisting of: (a) a live attenuated dengue virus; (b) an inactivated dengue virus; (c) a live attenuated or inactivated chimeric dengue virus and (d) a combination of two or more of (a) to (c), wherein said dengue antigen comprises a nucleotide sequence selected from the group consisting of SEQ ID NO: 4, SEQ ID NO: 5, SEQ ID NO: 6 and SEQ ID NO: 7.

A composition of the present invention, as described herein, may advantageously comprise a dengue antigen selected from the group consisting of: (a) a live attenuated dengue virus; (b) an inactivated dengue virus; (c) a live attenuated or inactivated chimeric dengue virus and (d) a combination of two or more of (a) to (c), wherein said dengue antigen comprises a nucleotide sequence encoding M and E sequences as described herein.

For instance, a composition of the present invention, e.g. for use in a method of protecting according to the present invention, may advantageously comprise any one of the following combinations of dengue antigens of serotypes 1, 2, 3 and 4: i) CYD-1, CYD-LAV, CYD-3 and CYD-4; ii) CYD-1, CYD-BID, CYD-3 and CYD-4; (iii) CYD-1, CYD-PR, CYD-3 and CYD-4 or (iv) CYD-1, CYD-MD, CYD-3 and CYD-4. A composition of the present invention may also advantageously comprise the following combination of dengue antigens: i) a dengue antigen comprising the prM and E sequences of CYD-1, VDV2, a dengue antigen comprising the prM and E sequences of CYD-3 and a dengue antigen comprising the prM and E sequences of CYD-4. For instance, a composition of the present invention may advantageously comprise CYD-1, VDV-2, CYD-3 and CYD-4. A composition of the present invention, as described herein, may advantageously comprise a dengue antigen of serotype 2 which comprises the prM-E sequence of CYD-LAV (SEQ ID NO: 8), CYD-BID (SEQ ID NO: 9), CYD-PR (SEQ ID NO: 10) CYD-MD (SEQ ID NO: 11) or SEQ ID NO: 2. A composition of the present invention, as described herein, may advantageously comprise a dengue antigen of serotype 2 which comprises a sequence having at least 90% identity to the prM-E sequence of CYD-LAV (SEQ ID NO: 8), CYD-BID (SEQ ID NO: 9), CYD-PR (SEQ ID NO: 10) CYD-MD (SEQ ID NO: 11) or SEQ ID NO: 2. For example, said sequence may be at least 91%, at least 92%, at least 93%, at least 94%, at least 95%, at least 96%, at least 97%, at least 98% or at least 99% identical to the prm-E sequence of CYD-LAV (SEQ ID NO: 8), CYD-BID (SEQ ID NO: 9), CYD-PR (SEQ ID NO: 10) CYD-MD (SEQ ID NO: 11) or SEQ ID NO: 2.

A vaccine composition for use the present invention, e.g. for use in a method according to the present invention preferably comprises a dengue antigen which is a vaccinal dengue virus. Such vaccinal dengue viruses include, for example, inactivated viruses, live attenuated viruses and live attenuated chimeric dengue viruses. Preferably, the vaccinal dengue viruses are live attenuated chimeric dengue viruses. Preferably, a live attenuated chimeric dengue virus according to the present invention comprises one or more proteins from a dengue virus and one or more proteins from a different flavivirus. Advantageously, said different flavivirus is a yellow fever virus, for example a yellow fever virus of strain YF 17D. Preferably a chimeric dengue virus according to the present invention comprises the prM-E amino acid sequences of a dengue virus, for example a chimeric dengue virus according to the present invention comprises a yellow fever virus genome whose prM-E whose prM-E sequence has been substituted with the prM-E sequence of a dengue virus. Advantageously, a vaccine composition according to the present invention, e.g. for use in a method of the present invention, comprises CYD-1, CYD-2, CYD-3 and CYD-4. A composition of the present invention may advantageously comprise any one of the following combinations of dengue antigens i) a dengue antigen comprising the prM and E sequences of CYD-1, a dengue antigen comprising the prM and E sequences of CYD-LAV, a chimeric dengue virus comprising the prM and E sequences of CYD-3 and a dengue antigen comprising the prM and E sequences of CYD-4; ii) a dengue antigen comprising the prM and E sequences of CYD-1, a dengue antigen comprising the prM and E sequences of CYD-BID, a dengue antigen comprising the prM and E sequences of CYD-3 and a dengue antigen comprising the prM and E sequences of CYD-4; (iii) a dengue antigen comprising the prM and E sequences of CYD-1, a dengue antigen comprising the prM and E sequences of CYD-PR, a dengue antigen comprising the prM and E sequences of CYD-3 and a dengue antigen comprising the prM and E sequences of CYD-4; (iv) a dengue antigen comprising the prM and E sequences of CYD-1, a dengue antigen comprising the prM and E sequences of CYD-MD, a dengue antigen comprising the prM and E sequences of CYD-3 and a dengue antigen comprising the prM and E sequences of CYD-4;. For instance, a composition of the present invention may also advantageously comprise any one of the following combinations of dengue antigens: i) CYD-1, CYD-LAV, CYD-3 and CYD-4; ii) CYD-1, CYD-BID, CYD-3 and CYD-4; (iii) CYD-1, CYD-PR, CYD-3 and CYD-4 or (iv) CYD-1, CYD-MD, CYD-3 and CYD-4. A composition of the present invention may also advantageously comprise the following combination of dengue antigens: i) a dengue antigen comprising the prM and E sequences of CYD-1, VDV2, a dengue antigen comprising the prM and E sequences of CYD-3 and a dengue antigen comprising the prM and E sequences of CYD-4. For instance, a composition of the present invention may advantageously comprise CYD-1, VDV-2, CYD-3 and CYD-4. A composition of the present invention, as described herein, may advantageously comprise a dengue antigen of serotype 2 which comprises the prM-E sequence of CYD-LAV (SEQ ID NO: 8), CYD-BID (SEQ ID NO: 9), CYD-PR (SEQ ID NO: 10) CYD-MD (SEQ ID NO: 11) or SEQ ID NO: 2. A composition of the present invention, as described herein, may advantageously comprise a dengue antigen of serotype 2 which comprises a sequence having at least 90% identity to the prM-E sequence of CYD-LAV (SEQ ID NO: 8), CYD-BID (SEQ ID NO: 9), CYD-PR (SEQ ID NO: 10) CYD-MD (SEQ ID NO: 11) or SEQ ID NO: 2. For example, said sequence may be at least 91%, at least 92%, at least 93%, at least 94%, at least 95%, at least 96%, at least 97%, at least 98% or at least 99% identical to the prm-E sequence of CYD-LAV (SEQ ID NO: 8), CYD-BID (SEQ ID NO: 9), CYD-PR (SEQ ID NO: 10) CYD-MD (SEQ ID NO: 11) or SEQ ID NO: 2.

The exact quantity of a vaccinal dengue virus of the present invention to be administered may vary according to the age and the weight of the patient being vaccinated, the frequency of administration as well as the other ingredients (e.g. adjuvants) in the composition. The quantity of a live attenuated dengue virus comprised in a vaccine composition of the present invention lies within a range of from about 10³ to about 10⁷ CCID₅₀ Generally, the quantity of a live attenuated dengue virus of each of serotypes 1 to 4 comprised in a vaccine composition of the present invention lies within a range of from about 10³ to about 10⁶ CCID₅₀ or of from about 10³ to about 10⁷ CCID₅₀, for example within a range of from about 5 × 10³ to about 5 × 10⁵ CCID₅₀, for example within a range of from about 1 × 10⁴ to about 1 × 10⁵ CCID₅₀, for example about 10⁵ CCID₅₀. The quantity of a live attenuated dengue virus of each of serotypes 1 to 4 comprised in a vaccine composition of the present invention may also lie within a range of from about 10⁴ to about 10⁷ CCID₅₀, for example about 10⁶ CCID₅₀. The quantity of a live attenuated dengue virus of each of serotypes 1 to 4 comprised in a tetravalent composition of the present invention may be equal. For example a tetravalent composition of the present invention may comprise about 10⁵ CCID₅₀ of each live attenuated dengue virus of serotypes 1 to 4. Alternatively, a tetravalent composition of the present invention may comprise about 10⁶ CCID₅₀ of each live attenuated dengue virus of serotypes 1 to 4. The quantity of a chimeric dengue virus (such as a YF/dengue chimera or Chimerivax (CYD) vaccinal virus as described herein) comprised in a vaccine composition of the present invention is about 10⁵ CCID₅₀ or about 10⁶ CCID₅₀. The quantity of live attenuated virus such as VDV-2 comprised in a vaccine composition of the present invention is about 10⁴ CCID₅₀. Generally, the quantity of an inactivated dengue virus of each of serotypes 1 to 4 comprised in a composition of the present invention lies within a range of from about 10⁴ to about 10⁸ CCID₅₀ equivalent, preferably within a range of from about 5 x 10⁴ to about 5 x 10⁷ CCID₅₀ equivalent, preferably within a range of from about 1 × 10⁴ to about 1 × 10⁶ CCID₅₀ equivalent, preferably about 10⁵ CCID₅₀ equivalent. Generally, the quantity of a VLP of each of serotypes 1 to 4 comprised in the composition lies within a range of from about 100ng to about 100µg of VLP, preferably within a range of from about 100ng to about 50µg, preferably within a range of from about 100ng to about 20µg, preferably about 1µg to 10µg. The amount of VLP can be determined by ELISA. Advantageously, a vaccine composition according to the present invention comprises an effective amount of a dengue antigen as defined herein.

A vaccine composition according to the present invention may further comprise a pharmaceutically acceptable carrier or excipient. A pharmaceutically acceptable carrier or excipient according to the present invention means any solvent or dispersing medium etc., commonly used in the formulation of pharmaceuticals and vaccines to enhance stability, sterility and deliverability of the active agent and which does not produce any secondary reaction, for example an allergic reaction, in humans. The excipient is selected on the basis of the pharmaceutical form chosen, the method and the route of administration. Appropriate excipients, and requirements in relation to pharmaceutical formulation, are described in "Remington's Pharmaceutical Sciences" (19th Edition, A.R. Gennaro, Ed., Mack Publishing Co., Easton, PA (1995)). Particular examples of pharmaceutically acceptable excipients include water, phosphate-buffered saline (PBS) solutions and a 0.3% glycine solution. A vaccine composition according to the present invention may advantageously comprise 0.4% saline and 2.5% human serum albumin (HSA).

A vaccine composition for use in a method of the present invention may optionally contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, human serum albumin, essential amino acids, nonessential amino acids, L-arginine hydrochlorate, saccharose, D-trehalose dehydrate, sorbitol, tris (hydroxymethyl) aminomethane and/or urea. In addition, the vaccine composition may optionally comprise pharmaceutically acceptable additives including, for example, diluents, binders, stabilizers, and preservatives. Preferred stabilizers are described in WO 2010/003670.

A vaccine composition of the present invention may comprise a dengue antigen which is a dengue immunoprotein. A dengue immunoprotein, as used herein, is a dengue envelope (E) protein, or derivative or fragment thereof, that when administered to an immunocompetent subject induces neutralizing antibodies against a dengue virus of serotype 1, 2, 3 or 4. Dengue immunoproteins include native and derivatized forms of dengue E proteins, including chemical conjugates, immunological fragments, and fusion proteins thereof.

Dengue immunoproteins, or derivatives or fragments thereof may be conjugated to carrier molecules. Such conjugation may be achieved by chemical conjugation techniques or through the recombinant expression of fusion proteins comprising the dengue immunoproteins or derivatives or fragments thereof and the carrier molecule. Examples of carrier molecules which may be used for preparing conjugates include diphtheria toxoid, tetanus toxoid, fragment C of tetanus toxin, mutants of diphtheria toxin including CRM 197, CRM 176, CRM228, CRM 45, CRM 9, CRM 45, CRM 102, CRM 103 and CRM 107, pneumococcal pneumolysin, OMPC, heat shock proteins, pertussis proteins, pneumococcal surface protein PspA or the toxin A or B of *Clostridium difficile.*

A vaccine composition of the present invention may comprise one or more adjuvants to enhance the immunogenicity of the dengue antigens. Those skilled in the art will be able to select an adjuvant which is appropriate in the context of this invention. An adjuvant is preferably used in a vaccine composition of the invention comprising an inactivated virus or a VLP or a dengue structural protein. An adjuvant may be used in a vaccine composition of the invention comprising a live attenuated virus, as long as said adjuvant does not impact replication.

Suitable adjuvants include an aluminum salt such as aluminum hydroxide gel, aluminum phosphate or alum, but may also be a salt of calcium, magnesium, iron or zinc. Further suitable adjuvants include an insoluble suspension of acylated tyrosine or acylated sugars, cationically or anionically derivatized saccharides, or polyphosphazenes. Alternatively, the adjuvant may be an oil-in-water emulsion adjuvant (EP 0 399 843B), as well as combinations of oil-in-water emulsions and other active agents (WO 95/17210; WO 98/56414; WO 99/12565 and WO 99/11241). Other oil emulsion adjuvants have been described, such as water-in-oil emulsions (U.S. Pat. No. 5,422, 109; EP 0 480 982 B2) and water-in-oil-in-water emulsions (U.S. Pat. No. 5,424,067; EP 0 480 981 B). Examples of such adjuvants include MF59, AF03 (WO 2007/006939), AF04 (WO 2007/080308), AF05, AF06 and derivatives thereof. The adjuvant may also be a saponin, lipid A or a derivative thereof, an immunostimulatory oligonucleotide, an alkyl glucosamide phosphate, an oil in water emulsion or combinations thereof. Examples of saponins include Quil A and purified fragments thereof such as QS7 and QS21.

As appreciated by skilled artisans, a vaccine composition of the present invention is suitably formulated to be compatible with the intended route of administration. Examples of suitable routes of administration include for instance intramuscular, transcutaneous, subcutaneous, intranasal, oral or intradermal. Advantageously, the route of administration is subcutaneous.

The vaccine compositions of the present invention may be administered using conventional hypodermic syringes or safety syringes such as those commercially available from Becton Dickinson Corporation (Franklin Lakes, NJ, USA) or jet injectors. For intradermal administration, conventional hypodermic syringes may be employed using the Mantoux technique or specialized intradermal delivery devices such as the BD Soluvia(TM) microinjection system (Becton Dickinson Corporation, Franklin Lakes, NJ, USA), may be used.

The volume of a vaccine composition of the present invention administered will depend on the method of administration. In the case of subcutaneous injections, the volume is generally between 0.1 and 1.0 ml, preferably approximately 0.5 ml.

Optionally, booster administrations of a vaccine composition according to the present invention may be used, for example between six months and ten years, for example six months, one year, three years, five years or ten years after initial immunization (i.e. after administration of the last dose scheduled in the initial immunization regimen).

According to one embodiment, the invention also provides a kit comprising a vaccine composition of the invention and instructions for the use of said vaccine composition in a method of protecting a human subject against dengue disease. The kit can comprise at least one dose (typically in a syringe) of any vaccine composition contemplated herein. According to one embodiment the kit may comprises a multi-dose formulation (typically in a vial) of any vaccine composition as described herein. The kit further comprises a leaflet mentioning the use of the said vaccine composition for the prevention of dengue disease or the use of the said vaccine for the prophylaxis of dengue disease. The leaflet may further mention the vaccination regimen and the human subject population to be vaccinated.

The efficacy of a vaccine composition of the present invention in reducing the likelihood or severity of dengue disease may be measured in a number of ways. For instance the efficacy of a vaccine composition of the present invention in reducing the likelihood or severity of symptomatic virologically-confirmed dengue disease may be calculated by measuring after the administration of at least one dose of said vaccine composition (e.g. after administration of one, two or three doses of said vaccine composition):
(i) the percentage of symptomatic virologically-confirmed dengue cases caused by dengue virus of any serotype;
(ii) the percentage of severe virologically-confirmed dengue cases caused by dengue virus of any serotype;
(iii) the percentage of dengue hemorrhagic fever cases of Grades I to IV caused by dengue virus of any serotype;
(iv) the percentage of DHF cases of Grade I caused by dengue virus of any serotype;
(v) the percentage of DHF cases of Grade II caused by dengue virus of any serotype;
(vi) the percentage of DHF cases of Grade III caused by dengue virus of any serotype;
(vii) the percentage of DHF cases of Grade IV caused by dengue virus of any serotype;
(viii) the annual incidence rate of hospitalized virologically-confirmed dengue caused by dengue virus of any serotype; and/or
(ix) the length of hospital stay for symptomatic virologically-confirmed dengue cases caused by dengue virus of any serotype;
in a group of subjects that has received said vaccine composition and comparing those measurements with the equivalent measurements from a control group of subjects that has not received said vaccine composition, wherein the subjects in both said groups are resident in a Dengue endemic region. A statistically significant reduction in any one or more of (i) to (ix) in the vaccinated group of subjects when compared with the unvaccinated control group of subjects is indicative of the efficacy of a vaccine composition according to the present invention. In a preferred embodiment, the efficacy of a vaccine composition according to the present invention is demonstrated by a statistically significant reduction of one or more of the measures as described above, wherein the DHF cases or dengue cases are caused by dengue virus of serotypes 1, 3 or 4.

The efficacy of a vaccine composition according to the present invention in reducing the severity or likelihood of dengue disease may also be calculated by measuring after the administration of at least one dose of said vaccine composition (e.g. after administration of one, two or three doses of said vaccine composition):
(i) the mean duration and/or intensity of fever;
(iii) the mean value for plasma leakage as defined by a change in haematocrit;
(iii) the mean value for thrombocytopenia (platelet count); and/or
(iv) the mean value of the level of liver enzymes including alanine aminotransferase (ALT) and aspartate aminotransferase (AST);
in a group of subjects that has received said vaccine composition and who have developed virologically-confirmed dengue disease and comparing those measurements with the equivalent measurements from a control group of subjects that has not received said vaccine composition and who have developed virologically-confirmed dengue disease. A statistically significant reduction in any one or more of (i) to (v) in the vaccinated group of subjects who have developed virologically-confirmed dengue disease when compared with the control group of subjects who have developed virologically-confirmed dengue disease is indicative of the efficacy of a vaccine composition according to the present invention in reducing the severity or likelihood of dengue disease.

Typically the efficacy of the method of protection of the invention against a dengue disease, as measured e.g. by the method described in example 1 (VE=100*(1-ID_{CYD}/ID_{Control}), where ID is the incidence density (i.e., the number of human subjects with virologically-confirmed dengue divided by the number of person-years at risk) in each group), is at least 50%, preferably at least 60%, wherein said dengue disease is caused by serotype 1, 3 or 4. The efficacy of the method of protection being advantageously at least 70%, preferably 80% against a dengue disease caused by serotype 3 or 4. The efficacy of the method of protection being advantageously at least 90% against dengue disease caused by serotype 4.

Percent identity between two amino acid sequences or two nucleotide sequences is determined by standard alignment algorithms such as, for example, Basic Local Alignment Tool (BLAST) described in Altschul et al. (1990) J. Mol. Biol., 215: 403-410, the algorithm of Needleman et al. (1970) J. Mol. Biol., 48: 444-453; the algorithm of Meyers et al. (1988) Comput. Appl. Biosci., 4: 11-17; or Tatusova et al. (1999) FEMS Microbiol. Lett., 174: 247-250, etc. Such algorithms are incorporated into the BLASTN, BLASTP and "BLAST 2 Sequences" programs (see www.ncbi.nim.nih.gov/BLAST). When utilizing such programs, the default parameters can be used. For example, for nucleotide sequences the following settings can be used for "BLAST 2 Sequences" : program BLASTN, reward for match 2, penalty for mismatch-2, open gap and extension gap penalties 5 and 2 respectively, gap x~dropoff 50, expect 10, word size 11, filter ON. For amino acid sequences the following settings can be used for "BLAST 2 Sequences" : program BLASTP, matrix BLOSUM62, open gap and extension gap penalties 11 and 1 respectively, gap x~dropoff 50, expect 10, word size 3, filter ON.

It is understood that the various features and preferred embodiments of the present invention as disclosed herein may be combined together.

Throughout this application, various references are cited. The disclosures of these references are hereby incorporated by reference into the present disclosure.

The present invention will be further illustrated by the following examples. It should be understood however that the invention is defined by the claims, and that these examples are given only by way of illustration of the invention and do not constitute in any way a limitation thereof.

### EXAMPLES

### Example 1: One year follow-up in Thailand of patients vaccinated with a tetravalent dengue vaccine (TDV) composition comprising Chimerivax^{™} DEN-1, DEN-2, DEN-3 and DEN-4

### Methods

### Study design and participants

An observer-blind, randomised, controlled, monocentre, Phase IIb trial of the efficacy of the tetravalent Chimerivax^{™} vaccine (i.e. a tetravalent vaccine comprising the particular CYD-1 strain generated from the prM and E sequences of DEN1 PU0359 (TYP 1 140), the particular CYD-2 strain generated from the prM and E sequences of DEN2 PUO218, the particular CYD-3 strain generated from the prM and E sequences of DEN3 PaH881/88 and the particular CYD-4 strain generated from the prM and E sequences of DEN4 1228 (TVP 980), see WO 03/101397 and Guy et al., Vaccine (2011), 29(42): 7229-41) against virologically-confirmed dengue disease is conducted. 4002 schoolchildren aged 4-11 years who are in good health based on medical history and physical examination are enrolled into the trial. The study is conducted at Ratchaburi Regional Hospital (RRH), Ratchaburi province, Muang district, Thailand. Children with acute febrile illness at enrolment, those with congenital or acquired immunodeficiency, and those receiving immunosuppressive therapy or other prohibited treatments or vaccines are excluded. Participants are randomly assigned 2:1 to receive three doses of dengue vaccine or a control product at Months 0, 6 and 12.

### Products

Each of the chimeric viruses are produced and cultured on Vero cells as described in WO 03/101397, Guy et al, Hum. Vaccines (2010) 6 (9): 696; Guy et al, Vaccine (2010) 28: 632; Guirakhoo et al, J. Virol. (2000) 74 : 5477 ; Guirakhoo et al, J. Virol. (2001) 75 (16) : 7290 ; Guirakhoo et al, Virol. (June 20, 2002) 298: 146; and Guirakhoo et al, J. Virol. (2004) 78 (9): 4761.

The vaccine is presented as a lyophilized powder (previously stored at temperature of between 2°C and 8°C), which is reconstituted with 0.5 mL of solvent for injection (0.4% NaCl containing 2.5% human serum albumin). As reconstituted, each 0.5 mL dose of vaccine contains 5 ± 1 log₁₀ CCID₅₀ of each chimeric dengue serotype (1, 2, 3 and 4) and excipients (essential amino acids, non-essential amino acids, L-arginine chlorhydrate, saccharose, D-trehalose dehydrate, sorbitol, tris (hydroxymethyl) aminoethane and urea). The control product is inactivated rabies vaccine (Verorab^{®}, Sanofi Pasteur, Lyon France) for the first injection of the first 50 children randomised to the control group, and 0·9% NaCl saline placebo for all other injections. All products are injected subcutaneously into the deltoid region of the upper arm.

### Assessments

All children are actively followed to detect acute febrile illness based on daily surveillance of school registers during school terms for absenteeism (followed by phone calls or home visits to absentees), and twice-weekly home visits, phone calls or mobile phone text-messages throughout school holidays. In any case of febrile illness (defined as illness with two temperature readings of ≥37.5°C at least 4 hours apart) parents are asked to take their child to RRH for diagnosis and treatment. The surveillance system also captures spontaneous consultations at RRH. Consecutive febrile episodes separated by a symptom-free interval of ≥14 days are considered as separate episodes. Paired serum samples are collected at presentation (i.e., acute sample, collected no later than 7 days after fever onset) and 7-14 days later (convalescent sample) and sent to Sanofi Pasteur's Global Clinical Immunology (GCI) laboratory (Swiftwater, PA, USA) and to the Centre for Vaccine Development (CVD, Mahidol University, Thailand). Acute samples are screened for the presence of flavivirus using an initial RT-PCR assay which detects the presence of any flavivirus (using primers composed of highly conserved flavivirus sequences). Positive samples are tested for wild-type dengue virus with a serotype-specific quantitative RT-PCR, as described herein. In parallel, all acute samples are tested for the presence of dengue NS1 antigen using commercial ELISA kit (Platelia^{™}, Bio-Rad Laboratories, Marnes-La-Coquette, France). A virologically-confirmed episode of dengue disease is defined as a positive result in either the serotype-specific RT-PCR, or the NS1 antigen ELISA.

Active surveillance is maintained until each participant has been followed for at least 13 months after the third vaccination and until the Independent Data Monitoring Committee (IDMC) confirms that ≥27 cases have occurred in the per-protocol (PP) population.

All serious adverse events (SAE) are documented until the sixth month after the last vaccination, and thereafter any fatal SAE or vaccine-related SAE.

Dengue immune responses are assessed in the first 300 enrolled children at RRH in sera collected at enrolment and 28 days after each injection. Sera are also prospectively collected from all participants on Day 28 after the third injection to assess immune responses in children with virologically-confirmed dengue occurring from this timepoint. Sera are sent to GCI for measurement of serotype-specific neutralizing antibody titres against the CYD parental dengue viruses using the plaque-reduction neutralization test (PRNT₅₀) as described herein. The assay's quantitation limit is 10 (1/dil). Samples below this value are assigned the titre 5 and considered seronegative.

### Statistical analysis

The primary objective is to determine vaccine efficacy (VE) against cases of symptomatic, virologically-confirmed dengue occurring more than 28 days after the third vaccination among children enrolled and vaccinated as planned, according to the equation: VE =100*(1-ID_{CYD}/ID_{Control}), where ID is the incidence density (i.e., the number of children with virologically-confirmed dengue divided by the number of person-years at risk) in each group. With an assumed disease incidence of 1·3%, a true VE of 70%, a minimum follow-up period of 1 year after the third vaccination, and a per protocol (PP) subject attrition rate of 7·5%/year, 4002 subjects assigned with a 2:1 ratio to dengue vaccine or control are needed to demonstrate, with more than 82% power, and 95% confidence, that VE is not nul. Analyses are based on the two-sided 95% confidence interval (CI) of VE, calculated using the Exact method (Breslow NE, Day NE. Statistical Methods in Cancer Research, Volume II ― The Design and Analysis of Cohort Studies. International Agency for Research on Cancer (IARC scientific publication No. 82), Lyon, France). The primary analysis is performed on the PP population, i.e. those who satisfy the enrolment criteria, who correctly receive all three doses of the assigned vaccine at Months 0, 6 (±15 days), and 12 (±30 days), and for whom group allocation is not unmasked. This analysis is repeated on the full analysis set for efficacy, in those who receive three injections. As a secondary objective, VE against dengue is determined before completion of the 3-dose vaccination regimen. In an analysis defined after unblinding, VE against each serotype individually is investigated. Analyses for safety and immunogenicity endpoints are descriptive, using 95%CI.

### Results

Of the 4002 children enrolled, 95.9% complete the vaccinations and 91.8% are included in the per protocol (PP) analysis set for efficacy. Vaccine and control groups are comparable for age and gender. More than 90% of those sampled at baseline are positive for antibodies against dengue or JEV.

### Efficacy

During the study, 131 dengue cases (131 children had 136 episodes) are virologically-confirmed. Of these, 77 occur more than 28 days after the third injection in the PP population and are included in the primary analysis: 45 cases occurred during 2522 person-years at risk in the vaccine group, while 32 cases occurred during 1251 person-years at risk in the control group. The corresponding vaccine efficacy is 30·2% (95%CI: -13·4―56·6). This finding is confirmed in the full analysis set (see Table 1 below). Efficacy after at least one injection is 33-4% (95%CI: 4·1―53·5) and after at least two injections is 35·3% (95%CI: 3·3―56·5).

**Table 1: Serotype-specific and overall efficacy of CYD tetravalent dengue vaccine against virologically-confirmed dengue disease**

| | **Dengue vaccine** | | **Control** | | **Efficacy** | |
|---|---|---|---|---|---|---|
| | Person-years at risk | Cases or Episodes∗ | Person-years at risk | Cases or Episodes^{∗} | % | (95% CI) |
| >**28 days after 3 injections (per-protocol analysis)** | | | | | | |
| Cases | 2522 | 45 | 1251 | 32 | 30·2 | (-13·4-56·6) |
| Serotype 1 episodes | 2536 | 9 | 1251 | 10 | 55·6 | (-21·6-84·0) |
| Serotype 2 episodes | 2510 | 31 | 1250 | 17 | 9·2 | (-75·3-51·3) |
| Serotype 3 episodes | 2541 | 1 | 1257 | 2 | 75·3 | (-375·0-99·6) |
| Serotype 4 episodes | 2542 | 0 | 1263 | 4 | 100 | (24·8-100) |
| NS1 Antigen positive | 2542 | 4 | 1265 | 0 | ND | ND |
| only episodes | | | | | | |

| >**28 days after 3 injections (Full analysis set)** | | | | | | |
|---|---|---|---|---|---|---|
| Cases | 2620 | 46 | 1307 | 34 | 32·5 | (-8·5-57·6) |
| Serotype 1 episodes | 2633 | 9 | 1308 | 10 | 55·3 | (-22·5-83·9) |
| Serotype 2 episodes | 2608 | 32 | 1307 | 19 | 15·6 | (-57·6-53·6) |
| Serotype 3 episodes | 2638 | 1 | 1312 | 2 | 75·1 | (-378-99·6) |
| Serotype 4 episodes | 2641 | 0 | 1320 | 4 | 100 | (-24·3-100) |
| NS1 Antigen positive | 2640 | 4 | 1322 | 0 | ND | ND |
| only episodes | | | | | | |

| >**28 days after at least 1 injection (Full analysis set)** | | | | | | |
|---|---|---|---|---|---|---|
| Cases | 5089 | 75 | 2532 | 56 | 33-4 | (4·1-53·5) |
| Serotype 1 episodes | 5139 | 14 | 2564 | 18 | 61·2 | (17·4-82·1) |
| Serotype 2 episodes | 5107 | 51 | 2560 | 26 | 1·7 | (-64·3-39·8) |
| Serotype 3 episodes | 5144 | 4 | 2565 | 10 | 80·1 | (30·9-95·4) |
| Serotype 4 episodes | 5149 | 1 | 2577 | 5 | 90·0 | (10·5-99·8) |
| NS1 Antigen positive | 5147 | 5 | 2579 | 1 | -150· | (-11750-72·0) |
| only episodes | | | | | 5 | |

| | | | | | | |
|---|---|---|---|---|---|---|
| Data are number except where indicated. ND: not determined. ^{∗}A 'case' was defined as a first episode of dengue fever virologically-confirmed by either serotype-specific PCRs, or NS1 antigen ELISA. Serotype-specific efficacy was calculated including all episodes of that serotype; 5 children with two virologically confirmed dengue episodes during the study were therefore included twice in the serotype-specific analysis. | | | | | | |

Post-hoc analyses reveal differing efficacy by serotype (see Table 1). Efficacy against DENV1, DENV3, and DENV4 after at least one injection is in the range 61·2%― 90·0%, compared with 1·7% against DENV2. Efficacy against DENV1, DENV3, and DENV4 after three injections is in the range 55.3%-100%, compared with 15.6% against DENV2.

In those subjects that acquired virologically-confirmed dengue, a statistically significant reduction in the annual incidence rate of hospitalization was observed in the vaccinated group when compared with the control group. The relative risk (RR) after three doses was 0.523 (see Table 2).

**Table 2: Incidence of hospitalized virologically-confirmed dengue during the trial**

| | CYD Dengue Vaccine Group (N=2666) Annual | | | | Control Group (N=1331) Annual | | | | Relative Risk | |
|---|---|---|---|---|---|---|---|---|---|---|
| Time period | M | Cases | Incidence Rate (95%CI) | n Occurrences | M | Cases | Incidence Rate (95%CI) | n Occurrences | RR | (95%CI) |
| Year 1 | 2666 | 8 | 0.3 (0.1; 0.6) | 8 | 1331 | 7 | 0.5 (0.2; 1.1) | 7 | 0.571 | (0.181, 1.85) |
| Year 2 | 2557 | 24 | 0.9 (0.5; 1.3) | 24 | 1282 | 23 | 1.7 (1.0; 2.5) | 23 | 0.523 | (0.283, 0.970) |

| | | | | | | | | | | |
|---|---|---|---|---|---|---|---|---|---|---|
| Year 1 = D0 to injection 3 ; Year 2 = Injection 3 to the end of Active Phase | | | | | | | | | | |

**Table 3: Rate of hospitalisation by serotype**

| | Vaccinee Group (%) | Control Group (%) |
|---|---|---|
| Serotype 1 | 8/14 (57.1) | 9/18 (50%) |
| Serotype 2 | 20/52 (38.5) | 15/27 (55.6) |
| Serotype 3 | 1/4 (25) | 3/11 (27.3) |
| Serotype 4 | 0/1 | 2/5 (40) |
| No serotype NS1 +ve | 3/5 (60) | 1/1 (100) |
| Total | 32/76 (42.1) | 30/62 (48.4) |

### Immunogenicity

Geometric mean titres (GMT) of neutralising antibodies against dengue serotypes 1-4 on Day 28 after the third injection in the per-protocol analysis set are, respectively, 146 (95%CI: 98·5―217), 310 (224-431), 405 (307-534), and 155 (123-196) in the vaccine group. In the control group these values are 23·9 (14·0―40·9), 52·2 (26·8―102), 48·9 (25·5―93·9), and 19-4 (11·6―32·2). Post one year GMTs are respectively 76.5; 122; 94 and 153 for serotypes 1, 2, 3 and 4.

### Safety

There are 584 SAEs during this phase of the study: 366 are reported by 11.8% (315/2666) of participants in the vaccine group, and 218 are reported by 13.2% (176/1331) of participants in the control group. There are no vaccine-related SAEs in the dengue group and there is one in the control group. SAEs observed are medical conditions consistent with the age group and showed no clustering within the 7- or 28-day post-vaccination periods.

Virologically-confirmed dengue cases occurring as a breakthrough in vaccinees were not more serious than those cases occurring in the control group.

### Sequence of the prM-E region of circulating wild type serotype 2 strain in the trial

The nucleotide and amino acid sequence of the prM-E region of the wild type serotype 2 strain that causes the DEN-2 cases in the trial is determined. These are set out below as SEQ ID NO: 1 and SEQ ID NO: 2 respectively. The E and the M amino acid sequences of the serotype 2 strain that causes the DEN-2 cases in the trial are described in SEQ ID NOs: 18 and 23 respectively.

>nucleotide sequence (SEQ ID NO: 1)
ttccatctaaccacacgcaacggagaaccacacatgatcgtcggtatacaggagaaaggga
aaagtcttctgttcaaaacagaggatggtgtgaacatgtgcaccctcatggctatggacct
tggtgaattgtgtgaagacacaatcacgtacaagtgtcctcttctcaggcagaatgagcca
gaagacatagactgttggtgcaactccacgtccacgtgggtaacctatgggacctgtacca
ctacgggagaacataggagagaaaaagatcagtggcactcgttccacatgtgggaatggg
actggagacgcgaaccgaaacatggatgtcatcagaaggggcttggaaacatgcccagaga
attgaaacttggatcctgagacatccaggcttcaccataatggcagcaatcctggcataca
ccataggaacgacacatttccagagagtcctgattttcatcctactgacagctgtcgctcc
ttcaatgacaatgcgttgcataggaatatcaaatagagactttgtagaaggggtttcagga
ggaagttgggttgacatagtcttagaacatggaagctgtgtgacgacgatggcaaaaaaca
aaccaacattggatttcgaactgataaaaacggaagccaaacagcctgccaccctaaggaa
gtactgcatagaagcaaaactaaccaacacaacaacagaatcccgttgcccaacacaaggg
gaacccagcctaaaagaagagcaggacaagaggttcgtctgcaaacactccatggtagaca
gaggatggggaaatggatgtggattatttggaaagggaggcattgtgacctgtgctatgtt
cacatgcaaaaagaacatggaaggaaaaatcgtgcaaccagaaaacttggaatacaccatt
gtggtaacacctcactcaggggaagagcatgcggtcggaaatgacacaggaaaacacggca
aggaaatcaaagtaacaccacagagttccatcacagaagcagaactgacaggttatggcac
cgtcacgatggagtgctccccgagaacaggcctcgacttcaatgagatggtgttgctgcag
atggaaaataaagcttggctggtgcataggcaatggtttctagacctgccattaccatggc
tgcccggagcggataacaagaatcaaattggatacagaaagaaacattggtcactttcaa
aaatccccatgcgaagaaacaggatgttgttttaggatcccaagaaggggccatgcat
acagcactcacaggagccacagaaatccaaatgtcgtcaggaaacttgctcttcactggac
atctcaagtgcaggctgagaatggacaagctacagcttaaaggaatgtcatactctatgtg
cacaggaaagtttaaagttgtgaaggaaatagcagaaacacaacatggaacgatagttatc
agagtgcaatatgaaggggacggctctccatgtaaaattccttttgagataatggatttgg
aaaaaagatatgtcttaggccgcctgatcacagtcaacccaattgtaacagaaaaagacag
cccagtcaacatagaagcagaacctccattcggagacagttacatcatcataggagtagag
ccgggacaactgaagctcaactggttcaagaaaggaagttctatcggccaaatgtttgaga
caacgatgagagggcgaagagaatggccattttgggtgacacagcctgggacttcggatc
cctgggaggagtgtttacatctataggaaaagctctccaccaagtctttggagcgatctat
ggggctgccttcagtggggtttcatggaccatgaaaatcctcataggagtcattatcacat
ggataggaatgaactcacgcagcacctcactgtctgtgtcactggtactggtgggaattgt
gacactgtatttaggagtcatggtgcaggcc

>amino acid sequence (SEQ ID NO: 2)
FHLTTRNGEPHMIVGIQEKGKSLLFKTEDGVNMCTLMAMDLGELCEDTITYKCPLLRQNEP
EDIDCWCNSTSTWVTYGTCTTTGEHRREKRSVALVPHVGMGLETRTETWMSSEGAWKHAQR
IETWILRHPGFTIMAAILAYTIGTTHFQRVLIFILLTAVAPSMTMRCIGISNRDFVEGVSG
GSWVDIVLEHGSCVTTMAKNKPTLDFELIKTEAKQPATLRKYCIEAKLTNTTTESRCPTQG
EPSLKEEQDKRFVCKHSMVDRGWGNGCGLFGKGGIVTCAMFTCKKNMEGKIVQPENLEYTI
VVTPHSGEEHAVGNDTGKHGKEIKVTPQSSITEAELTGYGTVTMECSPRTGLDFNEMVLLQ
MENKAWLVHRQWFLDLPLPWLPGADKQESNWIQKETLVTFKNPHAKKQDVVVLGSQEGAMH

TALTGATEIQMSSGNLLFTGHLKCRLRMDKLQLKGMSYSMCTGKFKVVKEIAETQHGTIVI
RVQYEGDGSPCKIPFEIMDLEKRYVLGRLITVNPIVTEKDSPVNIEAEPPFGDSYIIIGVE
PGQLKLNWFKKGSSIGQMFETTMRGAKRMAILGDTAWDFGSLGGVFTSIGKALHQVFGAIY
GAAFSGVSWTMKILIGVIITWIGMNSRSTSLSVSLVLVGIVTLYLGVMVQA

>amino acid sequence (SEQ ID NO: 18)
MRCIGISNRDFVEGVSGGSWVDIVLEHGSCVTTMAKNKPTLDFELIKTEAKQPATLRKYCI
EAKLTNTTTESRCPTQGEPSLKEEQDKRFVCKHSMVDRGWGNGCGLFGKGGIVTCAMFTCK
KNMEGKIVQPENLEYTIVVTPHSGEEHAVGNDTGKHGKEIKVTPQSSITEAELTGYGTVTM
ECSPRTGLDFNEMVLLQMENKAWLVHRQWFLDLPLPWLPGADKQESNWIQKETLVTFKNPH
AKKQDVVVLGSQEGAMHTALTGATEIQMSSGNLLFTGHLKCRLRMDKLQLKGMSYSMCTGK
FKVVKEIAETQHGTIVIRVQYEGDGSPCKIPFEIMDLEKRYVLGRLITVNPIVTEKDSPVN
IEAEPPFGDSYIIIGVEPGQLKLNWFKKGSSIGQMFETTMRGAKRMAILGDTAWDFGSLGG
VFTSIGKALHQVFGAIYGAAFSGVSWTMKILIGVIITWIGMNSRSTSLSVSLVLVGIVTLY
LGVMVQA

>amino acid sequence (SEQ ID NO: 23)
SVALVPHVGMGLETRTETWMSSEGAWKHAQRIETWILRHPGFTIMAAILAYTIGTTHFQRV
LIFILLTAVAPSMT

### Discussion

The main finding from this study is that a safe, efficacious vaccine against dengue based on the chimeric CYD viruses is possible. Estimated efficacy against DENV1, 3 and 4 is in a range consistent with the 70% hypothesis and is statistically significant after at least one vaccination. Efficacy in a range consistent with the 70% hypothesis is not observed against DENV2. Since DENV2 is the prevalent serotype in this study, overall vaccine efficacy is diminished in this setting.

The vaccine's safety and reactogenicity profile is good, and no vaccine-related SAEs and no safety signals are identified during the review of AEs and SAEs collected from over two years of active follow-up of more than 2600 vaccinees. Theoretical safety concerns associated with the potential enhancement of the rate or severity of dengue disease by an incomplete immune response against the four serotypes of dengue have previously hampered vaccine development. In this trial, the absence of disease enhancement in the presence of an incomplete immune response against the circulating DENV2 viruses is an important and reassuring finding. For instance, cases in vaccinees do not differ from cases in controls in terms of factors such as the duration of fever or in terms of the classical clinical signs of dengue such as bleeding, plasma leakage or thrombocytopenia. Furthermore, severe dengue was not more frequent among vaccinees than controls at any point during the trial).

It was also demonstrated that, in those subjects that acquired virologically-confirmed dengue, a statistically significant reduction in the annual incidence rate of hospitalization was observed in the vaccinated group when compared with the control group. This reduction was seen in those subjects that acquired virologically-confirmed dengue of serotype 2 (see Table 3).

The results observed in respect of DENV2 may be explained by a number of contributing factors. For instance, there is a possible antigenic mismatch between the CYD2 vaccine virus and the DENV2 virus that causes disease in the trial. In the 1990s, the Asian 1 genotype of DENV2 emerged in South-East Asia, replacing the previously dominant Asian/American lineage of viruses. Several mutations identified in Domain 2 of the E protein (E83, and in particular E226 and E228) are suggestive of changing viral fitness and antigenicity. The donor wild-type virus for the CYD2 vaccine (and the challenge strain used in the PRNT50) was a clinical isolate from Bangkok in 1980 (Guirakhoo F et al., J Virol 2000, 74: 5477-85). While this virus is also classified as belonging to the Asian I genotype, the above-mentioned key amino acid residues in this virus (and thus in CYD2) correspond to those of the Asian/American genotype (Hang et al PLoS Negl Trop Dis. 2010 Jul 20;4(7):e757).

Additionally, there are two extremely rare mutations in the prM-E sequence of the CYD2 vaccine that may also contribute to a mismatched immune response. These mutations are at positions prM24 and E251 (Guirakhoo et al, J. Virol. (2004) 78 (9): 4761).

The results observed against DENV2 are not associated with an absence of immunogenicity in the PRNT₅₀ assay. Neutralising antibody responses after vaccination against DENV2 are higher than those against DENV1 and DENV3.

In conclusion, the present study constitutes the first ever demonstration that a safe and efficacious dengue vaccine is possible and represents a major milestone in dengue vaccine development.

### Example 2: Identification of optimized dengue vaccinal strains of serotype 2

The objective of the present example is to identify dengue virus strains of serotype 2 which provide the basis for generating optimized dengue vaccine compositions against dengue virus of serotype 2, wherein said optimized dengue vaccine compositions provide improved efficacy in comparison to Chimerivax^{™} CYD-2 when used in a method according to the present invention.

Criteria determining the selection of optimized strains for the determination of a universal dengue 2 antigen include: (i) recently circulating strain; (ii) balanced selection between Asian and American strains; (iii) an optimized strain should have a prM-E sequence that is as similar as possible to a calculated global consensus sequence generated by aligning the available prM-E sequences of dengue viruses of serotype 2; (iv) amino acid variations that are predicted to impact antibody recognition should be avoided; (v) rare amino acids at a particular positions in the prM and E sequences should be avoided, especially in the E protein ectodomain (a rare amino acid at a particular position is defined as a amino acid that appears at that position in less than 15% of the aligned sequences); (vi) optimized strains for which some previous laboratory experience exists are preferred and (vii) a dengue antigen that leads to a balanced immune response in a tetravalent composition. Criteria determining the selection of optimized strains for a local dengue 2 antigen (i.e. that is especially effective against a wild type dengue virus circulating in a particular area) are criteria (i) and (vii).

### Methods

### Databases

Sequences are retrieved from the National Center for Biotechnology Information (NCBI) Dengue virus variation database (www.ncbi.nlm.nih.gov/genomes/VirusVariation/Database/nph-select.cgi?tax_id=12637).

### Sequence analyses

Sequence alignments are performed using the MUSCLE algorithm (Edgar, R. C. (2004) MUSCLE: multiple sequence alignment with high accuracy and high throughput. Nucleic Acids Res, 32(5):1792-1797).

Sequence alignment outputs are generated in Vector NTi version 9, module AlignX (Invitrogen). Sequence similarity searches are carried out using the BLAST algorithm (Altschul, S. F., Gish, W., Miller, W., Myers, E. W., and Lipman, D. J. (1990) Basic local alignment search tool. J Mol Biol, 215(3):403-410).

### Sequence numbering for prM-E sequences

The sub-sequences included in the prM-E sequences may be numbered in various ways: (i) the total prM-E protein sequence is numbered from position 1 to position 661, with the preM protein sequence designated as position 1 to position 90/91, the M protein sequence designated as position 91/92 to position 166 and the E protein sequence designated as position 167 to position 661; (ii) the prM and M protein sequences are numbered together, i.e. from position 1 to position 166 of the total sequence and E is numbered separately from position 1 to position 495; (iii) the prM, M and E sequences are numbered separately, i.e. prM is numbered from position 1 to 90/91, M is numbered from 1 to 75/76 and E from position 1 to position 495.

### Results

### Public sequences retrieval

All available dengue virus serotype 2 full length prM and E protein sequences are downloaded from the NCBI Dengue database. Download of sequences takes place on two separate occasions - on 4 October 2010 and in 2011. On the first occasion 669 sequences are downloaded and on the second occasion approximately 3200 sequences are downloaded.

### Global consensus sequence generation

On each occasion, all retrieved protein sequences are aligned to generate a global consensus sequence for the prM and E proteins of dengue virus of serotype 2. By definition, the global consensus sequence is an artificial sequence containing the most frequently encountered amino acid at each position. The global consensus sequences for the 2010 alignment and the 2011 alignment only differ by two amino acids. In the 2010 alignment, the global consensus sequence contains isoleucine and valine at positions 129 and 308 respectively of the E protein (by reference to the 1-495 E sequence numbering) and, by contrast, in the 2011 alignment, the global consensus sequence contains valine and isoleucine at positions 129 and 308 respectively of the E protein (by reference to the 1-495 E sequence numbering). The differences in the 2010 and 2011 global consensus sequences are explained by the fact that the respective percentages of strains containing valine or isoleucine at those positions are close to 50%. The global consensus sequence for the prM-E sequence is therefore represented as follows:

fhlttrngephmivgrqekgksllfktedgvnmctlmaidlgelcedtitykcpllrqnep
edidcwcnststwvtygtctttgehrrekrsvalvphvgmgletrtetwmssegawkhvqr
ietwilrhpgftimaailaytigtthfqralifilltavapsmtMRCIGISNRDFVEGVSG
GSWVDIVLEHGSCVTTMAKNKPTLDFELIKTEAKQPATLRKYCIEAKLTNTTTESRCPTQG
EPSLNEEQDKRFVCKHSMVDRGWGNGCGLFGKGGIVTCAMFTCKKNMEGKXVQPENLEYTI
VITPHSGEEHAVGNDTGKHGKEIKITPQSSITEAELTGYGTVTMECSPRTGLDFNEMVLLQ
MEDKAWLVHRQWFLDLPLPWLPGADTQGSNWIQKETLVTFKNPHAKKQDVVVLGSQEGAMH
TALTGATEIQMSSGNLLFTGHLKCRLRMDKLQLKGMSYSMCTGKFKZVKEIAETQHGTIVI
RVQYEGDGSPCKIPFEIMDLEKRHVLGRLITVNPIVTEKDSPVNIEAEPPFGDSYIIIGVE
PGQLKLNWFKKGSSIGQMFETTMRGAKRMAILGDTAWDFGSLGGVFTSIGKALHQVFGAIY
GAAFSGVSWTMKILIGVIITWIGMNSRSTSLSVSLVLVGVVTLYLGVMVQA (SEQ ID
NO: 3)

The global consensus sequence for the E sequence is represented as follows:

MRCIGISNRDFVEGVSGGSWVDIVLEHGSCVTTMAKNKPTLDFELIKTEAKQPATLRKYCI
EAKLTNTTTESRCPTQGEPSLNEEQDKRFVCKHSMVDRGWGNGCGLFGKGGIVTCAMFTCK
KNMEGKXVQPENLEYTIVITPHSGEEHAVGNDTGKHGKEIKITPQSSITEAELTGYGTVTM
ECSPRTGLDFNEMVLLQMEDKAWLVHRQWFLDLPLPWLPGADTQGSNWIQKETLVTFKNPH
AKKQDVVVLGSQEGAMHTALTGATEIQMSSGNLLFTGHLKCRLRMDKLQLKGMSYSMCTGK
FKZVKEIAETQHGTIVIRVQYEGDGSPCKIPFEIMDLEKRHVLGRLITVNPIVTEKDSPVN
IEAEPPFGDSYIIIGVEPGQLKLNWFKKGSSIGQMFETTMRGAKRMAILGDTAWDFGSLGG
VFTSIGKALHQVFGAIYGAAFSGVSWTMKILIGVIITWIGMNSRSTSLSVSLVLVGVVTLY
LGVMVQA (SEQ ID NO: 12)

In the above sequence, the global consensus prM sequence is shown in lower case letters and the E sequence is shown in upper case letters. The amino acid positions denoted as X (position 129 of the E sequence) and Z (position 308 of the E sequence) are each independently Val or Ile, i.e. the proportion of aligned amino acid sequences including Val or Ile at those positions is close to 50%.

### Determination of minor amino acid residues and analysis of the Chimerivax^{™} CYD2 sequence

A list of variable amino acid positions is established from the global alignment containing all amino acid positions varying in at least 5% of the aligned sequences. In addition, any amino acid from the sequence of the prM and E proteins of Chimerivax^{™} CYD2 that do not match the global consensus sequence are also identified. The results are shown in Table 4 (N.B., in the table, the prM and M protein sequences are numbered together, i.e. from position 1 to position 166 of the total sequence and E is numbered separately from position 1 to position 495).

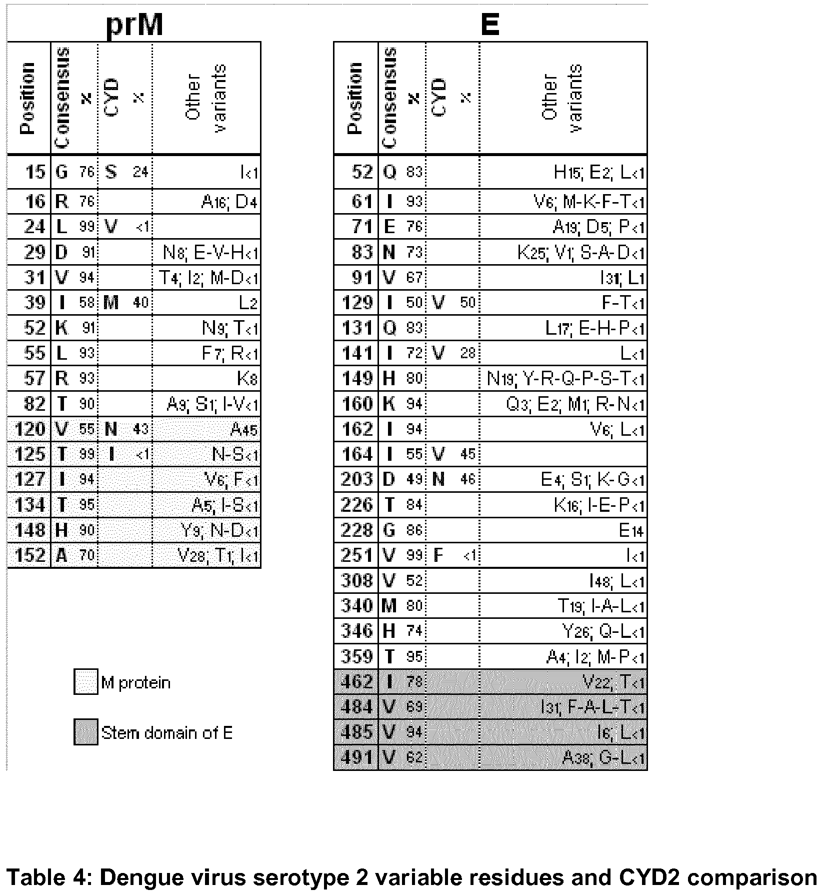

A total of 41 amino acid positions are identified in the prM and E sequences which either vary from the global consensus sequence in at least 5% of the aligned sequences and/or differ from the sequence of the prM and E proteins in CYD2. Ten amino acid positions in the sequence of the prM and E proteins in CYD2 differ from the global consensus sequence (5 positions in E, 2 positions in M and 3 in its precursor part, see Table 4). Five out of the ten differing residues present a variation distribution close to 50:50, suggesting a naturally variable position. Only three positions in the CYD2 prM-E sequence appear as very minor variants (pr-24 Val, M-125 Ile and E-251 Phe).

### Impact analysis of variations in the E and M proteins

To gain further insight into the variable positions, changes in the E protein ectodomain (amino acids 1-395), the most important domain for the seroneutralisation by the immune system are further analysed.

Using information available from a published 3D structure of the soluble ectodomain of the E protein of a dengue virus of serotype 2 (Modis, Y., et al. (2003) Proc Natl Acad Sci USA, 100(12):6986-6991), a 3D model of the Dengue virus particle surface is reconstructed. This allows a fine tuned assessment of the accessibility of each amino acid from the E ectodomain, which in turn is used in association with the variability level and the nature of the amino acid change to assess a potential impact of CYD2 variations on antibody recognition.

The analysis demonstrates that two variations in the Chimerivax^{™} CYD2 sequence from the global consensus sequence (Val 141 and Val 164 of the E protein) are completely buried in the 3D structure and so cannot directly interact with an antibody at the surface of the virion. Position 129 of the E protein is a 50:50 variable amino acid position between Val (Chimerivax^{™} CYD2) and Ile (global consensus sequence) and the substitution is also a fully conservative change. The potential impact of these variations is therefore considered as very limited.

The variation at position 203 of the E protein (Asn in Chimerivax^{™} CYD2 and Asp in the global consensus sequence) could potentially have an impact (well exposed residue, change of charge) but the distribution of the variation among strains is close to 50:50, suggesting a naturally variable position.

The variation at position 251 of the E protein of Chimerivax^{™} CYD2 (Phe in Chimerivax^{™} CYD2 and Val in the global consensus sequence) is extremely rare among retrieved strains. Such a variation could have some impact on recognition by an antibody, as it is rare, rather well exposed at the surface of the virion (29%) and corresponds to a non-conservative amino acid change.

The modeling analysis described above identifies two other position variations in the E protein that could have a potential impact on antibody recognition (positions 226 and 228), although Chimerivax^{™} CYD2 does not vary from the global consensus sequence at those positions. Therefore in identifying optimised serotype 2 strains, variations from the global consensus sequence at those positions (i.e. Thr at position 226 and glycine at position 228) are preferably avoided for a universal dengue 2 vaccine.

Without being bound by theory, the present inventors consider that the impact of amino acid variations can also be assessed using a scoring method which takes into account a number of relevant factors. In particular this method takes into account the genome location of the variation (G), the nature of the amino acid change (B), 3D mapping (M) and known variants at the position in question (DB), wherein the score is calculated as G × B × M × DB. A score of 0 would be classified as no expected impact, a score of >0 to 10 would be classified as a low expected impact, a score of >10 to 25 would be classified as a median expected impact and a score of >25 would be classified as a high expected impact.

The genome location (G) score is 0 if the amino acid is located in the M part of the prM/M protein (i.e. position 92 to 166 of the prM/M sequence) or in position 396 to 495 of the E protein. The genome location score is 1 if the amino acid is located in prM part of the prM/M protein (i.e. position 1 to 91 of the prM/M sequence) or in position 1 to 395 of the E protein.

The score related to the nature of the amino acid change (B) is calculated as B = 100 - [(Blosum95 score + 6) × 10], wherein the Blosum95 score for different amino acid substitutions is as shown in Table 5 below.

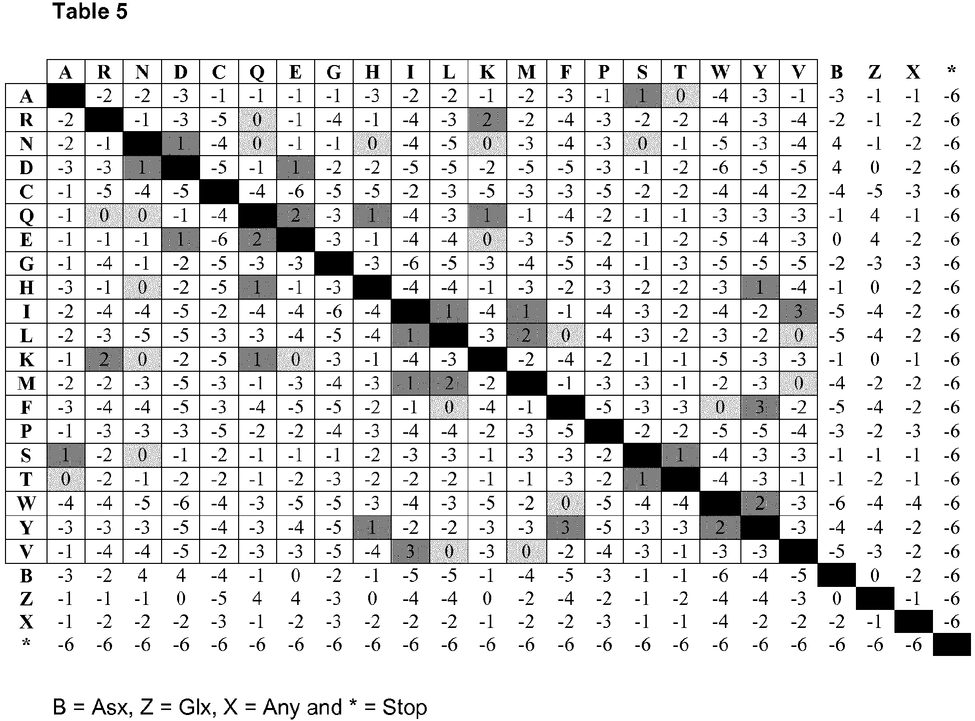

The M value depends on whether the amino acid is or is not located at the prM/E interface. For example, for CYD2 as used in Example 1, the amino acids that are located at the interface are prM residues 6, 7, 39, 40, 46-54, 56, 59-65, 67, 74 and 77 and E residues 64-72, 82-84, 101-104, 106-108 and 244-247. Where an amino acid is located at the interface, M equals 1. Where an amino acid is not located at the interface, M = Y × SAS %. Y is 1 if the amino acid is located in an "up" position (i.e. directed towards the external environment); Y is 0.5 if the amino acid is located on the "side" of the molecule (i.e. the amino acid is neither directed towards the external environment nor towards the capsid) and Y is 0 if the amino acid is located in a "down" position (i.e. directed towards the capsid). The solvent accessibility surface % (SAS %) value is generated using the Discovery Studio 3D modeling software (Accelrys, Inc., CA, USA).

The DB value is 0 when the amino acid substitution results in an amino acid at the substitution position which is the most common amino acid at that position in the dengue sequences present in the GenBank database (http://www.ncbi.nlm.nih.gov). The DB value is 0.25 when the amino acid substitution results in an amino acid at the substitution position which is found in more than 5% of the dengue sequences present in the database (but is not the most common amino acid at that position). The DB value is 0.50 when the amino acid substitution results in an amino acid at the substitution position which is found in less than 5% of the desngue sequences present in the database (except unique substitutions). The DB value is 1 when the substitution amino acid is unique.

During replication, viruses may acquire a mutation leading to an amino acid substitution. The above-mentioned method provides a means to determine the effect of such mutations on the progeny of the mutated viruses.

Preferred sequences (i.e. sequences that are considered to be satisfactorily close to the identified consensus sequence) may have: (i) at most two, preferably one or no high-impact amino acid substitutions; (ii) at most three, preferably two or one, or no median impact amino acid substitutions; and/or (iii) at most five, four, three, two or one low impact amino acid substitutions.

### Identification of optimized serotype 2 strains

Optimised serotype 2 strains are identified on the basis of the selection criteria described above.

A BLAST search is conducted to identify the strain having the closest sequence to the prM-E global consensus sequence in all of the available sequences. No sequence that is 100% identical to the prM-E global consensus sequence is found, but the best hit is a sequence from strain BID-V585 (NCBI Protein ID no. ACA58343; Genome ID no. EU529706; isolated from Puerto Rico in 2006) which shows only one variation from the global consensus sequence, at position 91 (Val in the global consensus sequence and Ile in BID-V585). The BID-V585 prM-E sequence contains 13 variations from the Chimerivax^{™} CYD-2 prM-E sequence.

A further strain selection is made so as to provide geographical balance in strain origin. Therefore a recently isolated Asian strain showing a good score in the BLAST analysis (strain MD-1280; NCBI Protein ID no. CAR65175; Genome ID no. FM21043; isolated from Viet Nam in 2004) is selected. Despite showing 6 variations with the global consensus sequence across prM-E, 3 of the 6 variations are identified as versatile positions naturally varying in more than 30% of the strains. The MD-1280 prM-E sequence contains 15 variations from the Chimerivax^{™} CYD-2 prM-E sequence.

A further strain selection is made on the basis of a large amount of previously accumulated experience with the strain. It is the PDK53-16681 strain, also known as the LAV-2 strain, a live-attenuated virus derived from Dengue serotype 2 16681 strain from Mahidol University (NCBI Protein ID no. AAA73186; Genome ID no. M84728; isolated from Thailand in 1964; Blok, J., et al. (1992); Virology 187 (2), 573-590). The LAV-2 prM-E sequence contains 10 variations from the global consensus sequence and 13 variations from the Chimerivax^{™} CYD-2 prM-E sequence.

A further strain selected on the basis of the above-mentioned criteria is strain PR/DB023 (NCBI Protein ID no. AEN71248; Genome ID no. JF804036; isolated from Puerto Rico in 2007). The PR/DB023 prM-E sequence contains 3 variations from the global consensus sequence and 13 variations from the Chimerivax^{TM} CYD-2 prM-E sequence.

None of the selected strains contain the rare amino acids present in the Chimerivax^{TM} CYD-2 prM-E sequence, i.e. Val at prM-24, Ile at M-125 and Phe at E-251.

### PrM to E nucleotide sequences of the four selected strains

**>LAV-2 prME nucleotide sequence (SEQ ID NO: 4)** 
ttccatttaaccacacgtaacggagaaccacacatgatcgtcagcagacaagagaaggga
aaagtcttctgtttaaaacagaggttggcgtgaacatgtgtaccctcatggccatggacct
tggtgaattgtgtgaagacacaatcacgtacaagtgtccccttctcaggcagaatgagcca
gaagacatagactgttggtgcaactctacgtccacgtgggtaacttatggacgtgtacca
ccatgggagaacatagaagagaaaaagatcagtggcactcgttccacatgtgggaatggg
actggagacacgaactgaaacatggatgtcatcagaaggggcctggaaacatgtccagaga
attgaaacttggatcttgagacatccaggcttcaccatgatggcagcaatcctggcataca
ccataggaacgacacatttccaaagagccctgattttcatcttactgacagctgtcactcc
ttcaatgacaATGCGTTGCATAGGAATGTCAAATAGAGACTTTGTGGAAGGGGTTTCAGGA
GGAAGCTGGGTTGACATAGTCTTAGAACATGGAAGCTGTGTGACGACGATGGCAAAAAACA
AACCAACATTGGATTTTGAACTGATAAAAACAGAAGCCAAACAGCCTGCCACCCTAAGGAA
GTACTGTATAGAGGCAAAGCTAACCAACACAACAACAGAATCTCGCTGCCCAACACAAGGG
GAACCCAGCCTAAATGAAGAGCAGGACAAAAGGTTCGTCTGCAAACACTCCATGGTAGACA
GAGGATGGGGAAATGGATGTGGACTATTTGGAAAGGGAGGCATTGTGACCTGTGCTATGTT
CAGATGCAAAAAGAACATGGAAGGAAAAGTTGTGCAACCAGAAAACTTGGAATACACCATT
GTGATAACACCTCACTCAGGGGAAGAGCATGCAGTCGGAAATGACACAGGAAAACATGGCA
AGGAAATCAAAATAACACCACAGAGTTCCATCACAGAAGCAGAATTGACAGGTTATGGCAC
TGTCACAATGGAGTGCTCTCCAAGAACGGGCCTCGACTTCAATGAGATGGTGTTGCTGCAG
 
ATGGAAAATAAAGCTTGGCTGGTGCACAGGCAATGGTTCCTAGACCTGCCGTTACCATGGT
TGCCCGGAGCGGACACACAAGGGTCAAATTGGATACAGAAAGAGACATTGGTCACTTTCAA
AAATCCCCATGCGAAGAAACAGGATGTTGTTGTTTTAGGATCCCAAGAAGGGGCCATGCAC
ACAGCACTTACAGGGGCCACAGAAATCCAAATGTCATCAGGAAACTTACTCTTCACAGGAC
ATCTCAAGTGCAGGCTGAGAATGGACAAGCTACAGCTCAAAGGAATGTCATACTCTATGTG
CACAGGAAAGTTTAAAGTTGTGAAGGAAATAGCAGAAACACAACATGGAACAATAGTTATC
AGAGTGCAATATGAAGGGGACGGCTCTCCATGCAAGATCCCTTTTGAGATAATGGATTTGG
AAAAAAGACATGTCTTAGGTCGCCTGATTACAGTCAACCCAATTGTGACAGAAAAAGATAG
CCCAGTCAACATAGAAGCAGAACCTCCATTTGGAGACAGCTACATCATCATAGGAGTAGAG
CCGGGACAACTGAAGCTCAACTGGTTTAAGAAAGGAAGTTCTATCGGCCAAATGTTTGAGA
CAACAATGAGGGGGGCGAAGAGAATGGCCATTTTAGGTGACACAGCCTGGGATTTTGGATC
CTTGGGAGGAGTGTTTACATCTATAGGAAAGGCTCTCCACCAAGTCTTTGGAGCAATCTAT
GGAGCTGCCTTCAGTGGGGTTTCATGGACTATGAAAATCCTCATAGGAGTCATTATCACAT
GGATAGGAATGAATTCACGCAGCACCTCACTGTCTGTGACACTAGTATTGGTGGGAATTGT
GACACTGTATTTGGGAGTCATGGTGCAGGCC
 UPPERCASE: E coding sequence; lowercase: prM coding sequence
**> BID/V585 - prME nucleotide sequence (SEQ ID NO: 5)** 
ttccatttaaccacacgtaatggagaaccacacatgatcgttggtaggcaagagaaaggga
aaagtcttctgtttaaaacagaggatggtgttaacatgtgcaccctcatggccatagacct
tggtgaattgtgtgaagatacaatcacgtacaagtgcccccctcctcaggcaaaatgaacca
gaagacatagattgttggtgcaactctacgtccacatgggtaacttatggacatgtacca
ccacaggagaacacagaagagaaaaagatcagtggcactcgttccacatgtgggcatggg
actggagacacgaactgaaacatggatgtcatcagaaggggcctggaaacatgttcagaga
attgaaacctggatcttgagacatccaggctttaccataatggcagcaatcctggcatata
ccataggaacgacacatttccaaagggctctgatcttcatttactgacagccgttgctcc
ttcaatgacaATGCGTTGCATAGGAATATCAAATAGAGACTTCGTAGAAGGGGTTTCAGGA
GGAAGTTGGGTTGACATAGTCTTAGAACATGGAAGTTGTGTGACGACGATGGCAAAAAATA
AACCAACATTGGATTTTGAACTGATAAAAACAGAAGCCAAACAACCTGCCACTCTAAGGAA
GTACTGTATAGAAGCAAAGCTGACCAATACAACAACAGAATCTCGTTGCCCAACACAAGGG
GAACCCAGTCTAAATGAAGAGCAGGACAAAAGGTTCATCTGCAAACACTCCATGGTAGACA
GAGGATGGGGAAATGGATGTGGATTATTTGGAAAGGGAGGCATTGTGACCTGTGCTATGTT
CACATGCAAAAAGAACATGGAAGGAAAAGTCGTGCAGCCAGAAAATCTGGAATACACCATC
GTGATAACACCTCACTCAGGAGAAGAGCACGCTGTAGGTAATGACACAGGAAAGCATGGCA
AGGAAATCAAAATAACACCACAGAGCTCCATCACAGAAGCAGAACTGACAGGCTATGGCAC
TGTCACGATGGAGTGCTCTCCGAGAACGGGCCTCGACTTCAATGAGATGGTACTGCTGCAG
ATGGAAGACAAAGCTTGGCTGGTGCACAGGCAATGGTTCCTAGACCTGCCGTTACCATGGC
TACCCGGAGCGGACACACAAGGATCAAATTGGATACAGAAAGAGACGTTGGTCACTTTCAA
AAATCCCCACGCGAAGAAACAGGACGTCGTTGTTTTAGGATCTCAAGAAGGGGCCATGCAC
ACGGCACTTACAGGGGCCACAGAAATCCAGATGTCATCAGGAAACTTACTGTTCACAGGAC
ATCTCAAGTGTAGGCTGAGAATGGACAAATTACAGCTTAAAGGAATGTCATACTCTATGTG
TACAGGAAAGTTTAAAATTGTGAAGGAAATAGCAGAAACACAACATGGAACAATAGTTATC
AGAGTACAATATGAAGGGGACGGCTCTCCATGTAAGATTCCTTTTGAGATAATGGATTTGG
AAAAAAGACACGTCCTAGGTCGCCTGATTACAGTGAACCCAATCGTAACAGAAAAAGATAG
CCCAGTCAACATAGAAGCAGAACCTCCATTCGGAGACAGCTACATCATCATAGGAGTAGAG
CCGGGACAATTGAAACTCAATTGGTTCAAGAAGGGAAGTTCCATTGGCCAAATGTTTGAGA
CAACAATGAGAGGAGCGAAGAGAATGGCCATTTTAGGTGACACAGCCTGGGATTTTGGATC
CCTGGGAGGAGTGTTTACATCTATAGGAAAGGCTCTCCACCAAGTTTTCGGAGCAATCTAT
GGGGCTGCTTTTAGTGGGGTCTCATGGACTATGAAAATCCTCATAGGAGTTATTATCACAT
 
GGATAGGAATGAATTCACGTAGCACCTCACTGTCTGTGTCACTAGTATTGGTGGGAGTCGT
GACACTGTACTTGGGGGTTATGGTGCAGGCT
**>PR/DB023 prME nucleotide sequence (SEQ ID NO: 6)** 
ttccatttaaccacacgtaatggagaaccacacatgatcgttggtaggcaagagaaaggga
aaagtcttctgttcaaaacagaggatggtgttaacatgtgtaccctcatggccatagacct
tggtgaattgtgtgaagatacaatcacgtacaagtgcccccctcctcaggcaaaatgaacca
gaagacatagattgttggtgcaactctacgtccacatgggtaacttatggacatgtacca
ccacaggagaacacagaagagaaaaagatcagtggcactcgttccacatgtgggcatggg
actggagacacgaactgaaacatggatgtcatcagaaggggcctggaaacatgttcagaga
attgaaacctggatattgagacatccaggctttaccataatggcagcaatcctggcatata
ccataggaacgacacatttccaaagggctctgatcttcatttactgacagccgtcgctcc
ttcaatgacaATGCGTTGCATAGGAATATCAAATAGAGACTTCGTAGAAGGGGTTTCAGGA
GGAAGTTGGGTTGACATAGTCTTAGAACATGGAAGTTGTGTGACGACGATGGCAAAAAATA
AACCAACATTGGATTTTGAACTGATAAAAACAGAAGCCAAACAACCTGCCACTCTAAGGAA
GTACTGTATAGAAGCAAAGCTGACCAATACAACAACAGAATCTCGTTGCCCAACACAAGGG
GAACCCAGTCTAAATGAAGAGCAGGACAAAAGGTTCATCTGCAAACACTCCATGGTAGACA
GAGGATGGGGAAATGGATGTGGATTATTTGGAAAAGGAGGCATTGTAACCTGTGCTATGTT
CACATGCAAAAAGAACATGGAAGGAAAAGTTGTGCTGCCAGAAAATCTGGAATACACCATC
GTGATAACACCTCACTCAGGAGAAGAGCACGCTGTAGGTAATGACACAGGAAAACATGGCA
AGGAAATTAAAATAACACCACAGAGTTCCATCACAGAAGCAGAACTGACAGGCTATGGCAC
TGTCACGATGGAGTGCTCTCCGAGAACGGGCCTCGACTTCAATGAGATGGTGCTGCTGCAG
ATGGAAGACAAAGCCTGGCTGGTGCACAGGCAATGGTTCCTAGATCTGCCGTTACCATGGC
TACCCGGAGCGGACACACAAGGATCAAATTGGATACAGAAAGAGACGTTGGTCACTTTCAA
AAATCCCCACGCGAAGAAACAGGACGTCGTTGTTTTAGGATCTCAAGAAGGGGCCATGCAC
ACGGCACTTACAGGGGCCACAGAAATCCAGATGTCATCAGGAAACTTACTGTTCACAGGAC
ATCTCAAGTGTAGGCTGAGAATGGACAAATTACAGCTTAAAGGAATGTCATACTCTATGTG
TACAGGAAAGTTTAAAATTGTGAAGGAAATAGCAGAAACACAACATGGAACAATAGTTATC
AGAGTACAATATGAAGGGGACGGCTCTCCATGTAAGATTCCTTTTGAGATAATGGATTTAG
AAAAAAGACACGTCCTAGGTCGCCTGATTACAGTGAACCCAATCGTAACAGAAAAAGATAG
CCCAGTCAACATAGAAGCAGAACCTCCATTCGGAGACAGCTACATCATCATAGGAGTAGAG
CCGGGACAATTGAAACTCAATTGGTTCAAGAAGGGAAGTTCCATTGGCCAAATGTTTGAGA
CAACAATGAGAGGAGCGAAGAGAATGGCCATTTTAGGTGACACAGCCTGGGATTTTGGATC
CCTGGGAGGAGTGTTTACATCTATAGGAAAGGCTCTCCACCAAGTTTTCGGAGCAATCTAT
GGGGCTGCTTTTAGTGGGGTCTCATGGACTATGAAAATCCTCATAGGAGTTATCATCACAT
GGATAGGAATGAATTCACGTAGCACCTCACTGTCTGTGTCACTAGTATTGGTGGGAGTCGT
GACACTGTACTTGGGGGTTATGGTGCAGGCT
>MD1280 prME nucleotide sequence (SEQ ID NO: 7)

ttccatttaaccacacgaaatggagaaccacacatgatcgttggcagacaagagaaaggga
aaagccttctgtttaaaacagaggatggtgtgaacatgtgtaccctcatggccattgatct
tggtgaattgtgtgaagatacaatcacgtacaagtgccccctcctcaggcagaatgaacca
gaagatatagattgttggtgcaactccacgtccacatgggtaacttatgggacgtgtacca
ccacaggagaacacagaagagaaaaaagatcagtggcactcgttccacatgtgggtatggg
actggagacacgaactgaaacatggatgtcgtcagaagggcctggaaacacgctcagaga
attgaaacttggatcttgagacatccaggctttaccataatggcagcaatcctggcatata
ccgtaggaacgacacatttccaaaggccctgatttcatcttactggcagctgtcgctcc
ttcaatgacaATGCGTTGCATAGGAATATCAAATAGAGACTTTGTAGAAGGGGTTTCAGGA

GGAAGCTGGGTTGACATAGTCTTAGAACATGGAAGTTGTGACGACAATGGCAAAAAATA
AACCAACACTGGATTTTGAACTGATAAAAACAGAAGCCAAACAACCTGCCACTCTAAGGAA
GTACTGTATAGAGGCAAAGCTGACCAATACAACAACAGAATCTCGTTGCCCAACACAAGGG
GAACCCAGTCTAAATGAAGAGCAGGACAAAAGGTTCGTCTGCAAACACTCCATGGTAGACA
GAGGATGGGGAAATGGATGTGGATTATTTGGAAAGGGAGGCATTGTGACCTGTGCTATGTT
CACATGCAAAAAGAACATGGAAGGAAAAATCGTGCAACCAGAAAATTTGGAATACACCATC
GTGATAACACCTCACTCAGGAGAAGAGCACGCTGTAGGTAATGACACAGGAAAACATGGTA
AGGAAATTAAAATAACACCACAGAGTTCCATCACAGAAGCAGAACTGACAGGCTATGGCAC
AGTCACGATGGAGTGCTCTCCGAGAACGGGCCTTGACTTCAATGAGATGGTGCTGCTGCAG
ATGGAAGATAAAGCTTGGCTGGTGCACAGGCAATGGTTCCTAGACCTGCCGTTACCATGGC
TACCCGGAGCGGACACACAAGGATCAAATTGGATACAGAAAGAGACATTGGTCACTTTCAA
AAATCCCCACGCGAAGAAGCAGGATGTCGTTGTTTTAGGATCTCAAGAAGGAGCCATGCAC
ACGGCACTCACAGGGGCCACAGAAATCCAGATGTCATCAGGAAACTTACTATTCACAGGAC
ATCTCAAATGCAGGCTGAGAATGGACAAACTACAGCTCAAAGGAATGTCATACTCTATGTG
TACAGGAAAGTTTAAAATTGTGAAGGAAATAGCAGAAACACAACATGGAACAATAGTTATC
AGAGTACAATATGAAGGAGACGGCTCTCCATGTAAGATCCCTTTTGAAATAATGGATTTGG
AAAAAAGACATGTCTTAGGTCGCCTGATTACAGTTAATCCGATCGTAACAGAAAAAGATAG
CCCAGTCAACATAGAAGCAGAACCTCCATTCGGAGACAGCTACATCATTATAGGAGTAGAG
CCGGGACAATTGAAACTCAACTGGTTCAAGAAAGGAAGTTCCATCGGCCAAATGTTTGAGA
CGACAATGAGAGGAGCAAAGAGAATGGCCATTTTAGGTGACACAGCCTGGGATTTTGGATC
TCTGGGAGGAGTGTTTACATCTATAGGAAAGGCTCTCCACCAAGTTTTCGGAGCAATCTAT
GGGGCTGCCTTTAGTGGGGTTTCATGGACTATGAAAATCCTCATAGGAGTCATCATCACAT
GGATAGGAATGAATTCACGTAGCACCTCACTGTCTGTGTCACTAGTATTGGTGGGAATCAT
AACACTGTACTTGGGAGCTATGGTGCAGGCT

### Corresponding protein prM to E sequences of the four selected strains

**>LAV2 prME protein sequence (SEQ ID NO: 8)** 
fhlttrngephmivsrqekgksllfktevgvnmctlmamdlgelcedtitykcpllrqnep
edidcwcnststwvtygtcttmgehrrekrsvalvphvgmgletrtetwmssegawkhvqr
ietwilrhpgftmmaailaytigtthfqralifilltavtpsmtMRCIGMSNRDFVEGVSG
GSWVDIVLEHGSCVTTMAKNKPTLDFELIKTEAKQPATLRKYCIEAKLTNTTTESRCPTQG
EPSLNEEQDKRFVCKHSMVDRGWGNGCGLFGKGGIVTCAMFRCKKNMEGKVVQPENLEYTI
VITPHSGEEHAVGNDTGKHGKEIKITPQSSITEAELTGYGTVTMECSPRTGLDFNEMVLLQ
MENKAWLVHRQWFLDLPLPWLPGADTQGSNWIQKETLVTFKNPHAKKQDVVVLGSQEGAMH
TALTGATEIQMSSGNLLFTGHLKCRLRMDKLQLKGMSYSMCTGKFKVVKEIAETQHGTIVI
RVQYEGDGSPCKIPFEIMDLEKRHVLGRLITVNPIVTEKDSPVNIEAEPPFGDSYIIIGVE
PGQLKLNWFKKGSSIGQMFETTMRGAKRMAILGDTAWDFGSLGGVFTSIGKALHQVFGAIY
GAAFSGVSWTMKILIGVIITWIGMNSRSTSLSVTLVLVGIVTLYLGVMVQA
>LAV2 E protein sequence (SEQ ID NO: 13) 
MRCIGMSNRDFVEGVSGGSWVDIVLEHGSCVTTMAKNKPTLDFELIKTEAKQPATLRKYCI
EAKLTNTTTESRCPTQGEPSLNEEQDKRFVCKHSMVDRGWGNGCGLFGKGGIVTCAMFRCK
KNMEGKVVQPENLEYTIVITPHSGEEHAVGNDTGKHGKEIKITPQSSITEAELTGYGTVTM
ECSPRTGLDFNEMVLLQMENKAWLVHRQWFLDLPLPWLPGADTQGSNWIQKETLVTFKNPH
AKKQDVVVLGSQEGAMHTALTGATEIQMSSGNLLFTGHLKCRLRMDKLQLKGMSYSMCTGK
FKVVKEIAETQHGTIVIRVQYEGDGSPCKIPFEIMDLEKRHVLGRLITVNPIVTEKDSPVN
IEAEPPFGDSYIIIGVEPGQLKLNWFKKGSSIGQMFETTMRGAKRMAILGDTAWDFGSLGG
 
VFTSIGKALHQVFGAIYGAAFSGVSWTMKILIGVIITWIGMNSRSTSLSVTLVLVGIVTLY
LGVMVQA
**>LAV2 M protein sequence (SEQ ID NO: 19)** 
svalvphvgmgletrtetwmssegawkhvqrietwilrhpgftmmaailaytigtthfqra
lifilltavtpsmt
**>BID/V585 prME protein sequence (SEQ ID NO: 9)** 
fhlttrngephmivgrqekgksllfktedgvnmctlmaidlgelcedtitykcpllrqnep
edidcwcnststwvtygtctttgehrrekrsvalvphvgmgletrtetwmssegawkhvqr
ietwilrhpgftimaailaytigtthfqralifilltavapsmtMRCIGISNRDFVEGVSG
GSWVDIVLEHGSCVTTMAKNKPTLDFELIKTEAKQPATLRKYCIEAKLTNTTTESRCPTQG
EPSLNEEQDKRFICKHSMVDRGWGNGCGLFGKGGIVTCAMFTCKKNMEGKVVQPENLEYTI
VITPHSGEEHAVGNDTGKHGKEIKITPQSSITEAELTGYGTVTMECSPRTGLDFNEMVLLQ
MEDKAWLVHRQWFLDLPLPWLPGADTQGSNWIQKETLVTFKNPHAKKQDVVVLGSQEGAMH
TALTGATEIQMSSGNLLFTGHLKCRLRMDKLQLKGMSYSMCTGKFKIVKEIAETQHGTIVI
RVQYEGDGSPCKIPFEIMDLEKRHVLGRLITVNPIVTEKDSPVNIEAEPPFGDSYIIIGVE
PGQLKLNWFKKGSSIGQMFETTMRGAKRMAILGDTAWDFGSLGGVFTSIGKALHQVFGAIY
GAAFSGVSWTMKILIGVIITWIGMNSRSTSLSVSLVLVGVVTLYLGVMVQA
**>BID/V585 E protein sequence (SEQ ID NO: 14)** 
MRCIGISNRDFVEGVSGGSWVDIVLEHGSCVTTMAKNKPTLDFELIKTEAKQPATLRKYCI
EAKLTNTTTESRCPTQGEPSLNEEQDKRFICKHSMVDRGWGNGCGLFGKGGIVTCAMFTCK
KNMEGKVVQPENLEYTIVITPHSGEEHAVGNDTGKHGKEIKITPQSSITEAELTGYGTVTM
ECSPRTGLDFNEMVLLQMEDKAWLVHRQWFLDLPLPWLPGADTQGSNWIQKETLVTFKNPH
AKKQDVVVLGSQEGAMHTALTGATEIQMSSGNLLFTGHLKCRLRMDKLQLKGMSYSMCTGK
FKIVKEIAETQHGTIVIRVQYEGDGSPCKIPFEIMDLEKRHVLGRLITVNPIVTEKDSPVN
IEAEPPFGDSYIIIGVEPGQLKLNWFKKGSSIGQMFETTMRGAKRMAILGDTAWDFGSLGG
VFTSIGKALHQVFGAIYGAAFSGVSWTMKILIGVIITWIGMNSRSTSLSVSLVLVGVVTLY
LGVMVQA
**>BID/V585 M protein sequence (SEQ ID NO: 20)** 
svalvphvgmgletrtetwmssegawkhvqrietwilrhpgftimaailaytigtthfqra
lifilltavapsmt
**>PR/DB023 prME protein sequence (SEQ ID NO: 10)** 
fhlttrngephmivgrqekgksllfktedgvnmctlmaidlgelcedtitykcpllrqnep
edidcwcnststwvtygtctttgehrrekrsvalvphvgmgletrtetwmssegawkhvqr
ietwilrhpgftimaailaytigtthfqralifilltavapsmtMRCIGISNRDFVEGVSG
GSWVDIVLEHGSCVTTMAKNKPTLDFELIKTEAKQPATLRKYCIEAKLTNTTTESRCPTQG
EPSLNEEQDKRFICKHSMVDRGWGNGCGLFGKGGIVTCAMFTCKKNMEGKVVLPENLEYTI
VITPHSGEEHAVGNDTGKHGKEIKITPQSSITEAELTGYGTVTMECSPRTGLDFNEMVLLQ
MEDKAWLVHRQWFLDLPLPWLPGADTQGSNWIQKETLVTFKNPHAKKQDVVVLGSQEGAMH
TALTGATEIQMSSGNLLFTGHLKCRLRMDKLQLKGMSYSMCTGKFKIVKEIAETQHGTIVI
RVQYEGDGSPCKIPFEIMDLEKRHVLGRLITVNPIVTEKDSPVNIEAEPPFGDSYIIIGVE
 
PGQLKLNWFKKGSSIGQMFETTMRGAKRMAILGDTAWDFGSLGGVFTSIGKALHQVFGAIY
GAAFSGVSWTMKILIGVIITWIGMNSRSTSLSVSLVLVGVVTLYLGVMVQA
**>PR/DB023 E protein sequence (SEQ ID NO: 15)** 
MRCIGISNRDFVEGVSGGSWVDIVLEHGSCVTTMAKNKPTLDFELIKTEAKQPATLRKYCI
EAKLTNTTTESRCPTQGEPSLNEEQDKRFICKHSMVDRGWGNGCGLFGKGGIVTCAMFTCK
KNMEGKVVLPENLEYTIVITPHSGEEHAVGNDTGKHGKEIKITPQSSITEAELTGYGTVTM
ECSPRTGLDFNEMVLLQMEDKAWLVHRQWFLDLPLPWLPGADTQGSNWIQKETLVTFKNPH
AKKQDVVVLGSQEGAMHTALTGATEIQMSSGNLLFTGHLKCRLRMDKLQLKGMSYSMCTGK
FKIVKEIAETQHGTIVIRVQYEGDGSPCKIPFEIMDLEKRHVLGRLITVNPIVTEKDSPVN
IEAEPPFGDSYIIIGVEPGQLKLNWFKKGSSIGQMFETTMRGAKRMAILGDTAWDFGSLGG
VFTSIGKALHQVFGAIYGAAFSGVSWTMKILIGVIITWIGMNSRSTSLSVSLVLVGVVTLY
LGVMVQA
**>PR/DB023 M protein sequence (SEQ ID NO : 21)** 
svalvphvgmgletrtetwmssegawkhvqrietwilrhpgftimaailaytigtthfqra
lifillltavapsmt
**>MD1280 prME protein sequence (SEQ ID NO: 11)** 
fhlttrngephmivgrqekgksllfktedgvnmctlmaidlgelcedtitykcpllrqnep
edidcwcnststwvtygtctttgehrrekrsvalvphvgmgletrtetwmssegawkhaqr
ietwilrhpgftimaailaytvgtthfqralifillaavapsmtMRCIGISNRDFVEGVSG
GSWVDIVLEHGSCVTTMAKNKPTLDFELIKTEAKQPATLRKYCIEAKLTNTTTESRCPTQG
EPSLNEEQDKRFVCKHSMVDRGWGNGCGLFGKGGIVTCAMFTCKKNMEGKIVQPENLEYTI
VITPHSGEEHAVGNDTGKHGKEIKITPQSSITEAELTGYGTVTMECSPRTGLDFNEMVLLQ
MEDKAWLVHRQWFLDLPLPWLPGADTQGSNWIQKETLVTFKNPHAKKQDVVVLGSQEGAMH
TALTGATEIQMSSGNLLFTGHLKCRLRMDKLQLKGMSYSMCTGKFKIVKEIAETQHGTIVI
RVQYEGDGSPCKIPFEIMDLEKRHVLGRLITVNPIVTEKDSPVNIEAEPPFGDSYIIIGVE
PGQLKLNWFKKGSSIGQMFETTMRGAKRMAILGDTAWDFGSLGGVFTSIGKALHQVFGAIY
GAAFSGVSWTMKILIGVIITWIGMNSRSTSLSVSLVLVGIITLYLGAMVQA
**>MD1280 E protein sequence (SEQ ID NO: 16)** 
MRCIGISNRDFVEGVSGGSWVDIVLEHGSCVTTMAKNKPTLDFELIKTEAKQPATLRKYCI
EAKLTNTTTESRCPTQGEPSLNEEQDKRFVCKHSMVDRGWGNGCGLFGKGGIVTCAMFTCK
KNMEGKIVQPENLEYTIVITPHSGEEHAVGNDTGKHGKEIKITPQSSITEAELTGYGTVTM
ECSPRTGLDFNEMVLLQMEDKAWLVHRQWFLDLPLPWLPGADTQGSNWIQKETLVTFKNPH
AKKQDVVVLGSQEGAMHTALTGATEIQMSSGNLLFTGHLKCRLRMDKLQLKGMSYSMCTGK
FKIVKEIAETQHGTIVIRVQYEGDGSPCKIPFEIMDLEKRHVLGRLITVNPIVTEKDSPVN
IEAEPPFGDSYIIIGVEPGQLKLNWFKKGSSIGQMFETTMRGAKRMAILGDTAWDFGSLGG
VFTSIGKALHQVFGAIYGAAFSGVSWTMKILIGVIITWIGMNSRSTSLSVSLVLVGIITLY
LGAMVQA
**>MD1280 M protein sequence (SEQ ID NO: 22)** 
svalvphvgmgletrtetwmssegawkhaqrietwilrhpgftimaailaytvgtthfqra
lifillaavapsmt
**>Consensus M sequence (SEQ ID NO: 17)**

svalvphvgmgletrtetwmssegawkhvqrietwilrhpgftimaailaytigtthfqra
lifillltavapsmt

### Example 3: Construction of the cDNA clones corresponding to the optimized serotype 2 chimeric viruses and production of the encoded viruses

Construction of chimeric dengue viruses corresponding to the optimized serotype 2 strains is achieved using the Chimerivax^{™} technology substantially in accordance with the teaching of Chambers, et al. (1999, J.Virology 73(4):3095-3101). Reference may also be made to international patent applications WO 98/37911, WO 03/101397, WO 07/021672, WO 08/007021, WO 08/047023 and WO 08/065315, which detail the analogous processes used to construct CYD-1, CYD2, CYD-3 and CYD-4. Briefly, however, chimeric dengue viruses corresponding to the optimized serotype 2 strains are constructed as follows (N.B. the optimized chimeric dengue viruses are constructed using the genomic backbone of YF strain YF17D204 (YF-VAX(R), Sanofi-Pasteur, Swiftwater, PA, USA).

### Construction of plasmid pSP1101

### Construction of the YF-VAX cDNA clone - pJSY2284.1 (pACYC YF-Vax 5-3)

A full-length infectious cDNA clone of YF-VAX is constructed. The full-length infectious cDNA clone is based on the sequence of YF-VAX. A low copy number plasmid pACYC177 (New England Biolabs, Inc., Ipswich, MA, USA) is used to assemble the full-length cDNA clone.

A DNA sequence named as SP6 YF-Vax 5-3 is synthesized by GeneArt^{®}. The sequence of SP6 YF-Vax 5-3 is designed in a way to facilitate an easy assembly of a full-length YF-Vax cDNA clone. The sequence is 2897 bp long and comprises the Xma I-SP6 promoter, the YF-Vax 5'UTR, the capsid, prM, M, part of E which extends to the Apa I site followed by unique sites MIu I-Sap I-Ngo MI-Aat II-Cla I for assembly, part of NS5 and further extended to 3' UTR followed by an Nru I site, which is used for run-off. This synthesized DNA sequence is flanked by EcoR V and Xho I sites. After digestion with EcoR V/Xho I, this DNA fragment is then cloned into the Aat II/Xho I sites of low copy number plasmid pACYC177 to replace the 1615bp Aat II/Xho I fragment. The resulting plasmid pJSY2284.1 (pACYC YF-Vax 5-3) is confirmed by sequence analysis.

### RT-PCR and cloning of the YF-Vax cDNA fragments spanning from the sites Apa I, Mlu I, Sap I, Ngo MI, Aat II and Cla I and assembly of a full-length infectious cDNA clone of YF-vax (pJSY2374.5)

The yellow fever vaccine YF-VAX is grown in Vero cells, and the virus particles are concentrated. The viral RNA of YF-VAX is extracted from the concentrated virus and the cDNA copy is made by reverse transcriptase. Five cDNA fragments as shown herein are PCR amplified, TOPO cloned, sequenced and compared to the sequence of YF-VAX 2003. The PCR errors found in each fragment are corrected by either site-directed mutagenesis or fragment switching. There are too many sequence differences found in Ngo MI-Aat II fragment after TOPO cloning, and therefore, this fragment is synthesized by GeneArt^{®}. After final sequence confirmation, the five DNA fragments; Apa I-MIu I, MIu I-Sap I, Sap I-Ngo M1, Ngo MI-Aat II, and Aat II-CIa I are isolated and stepwise cloned into the unique sites Apa I, MIu I, Sap I, Ngo MI, Aat II and CIa I in the plasmid pJSY2284.1 to obtain plasmid pJSY2374.5, which is confirmed to contain the correct sequence of YF-VAX full-length cDNA.

### Construction of cDNA for optimized chimeric dengue virus derived from the LAV2 strain (pSP1101)

The strategy is to replace the prM and E genes of the YF-VAX^{®} vaccine strain in the pJSY2374.5 plasmid containing the YF-VAX genome with those of the LAV2 strain, as done previously to build the CYD-1, CYD-2, CYD-3 and CYD-4 dengue vaccines, using the Chimerivax^{™} technology. The resulting plasmid is pSP1101.

In pJSY2374, restriction sites used for cloning are Xma I and Mlu I. These sites are located upstream and downstream of a 3000 bp fragment which contains: the SP6 promoter, YF17D 5'UTR, YF17D-capsid, YF17D-prM, YF17D-E and the N terminus of YF17D-NS1. A sequence corresponding to this fragment but instead containing the prM and E genes of LAV2 flanked by Xma I and Mlu I sites is synthesized by GeneArt^{®} and cloned into plasmid pMK-RQ (GeneArt^{®}, Life Technologies Ltd, Paisley, U.K.) to create plasmid pMK-RQ-Seq1. Plasmid pJSY2374.5 and pMK-RQ-Seq1 are digested by Xma I and MIu 1. The Xma I-MIu I fragment from pMK-RQ-Seq1 is then inserted into plasmid pJSY2374.5 to form plasmid pSP1101. XL-10 Gold Ultracompetent bacteria (Agilent Technologies, CA, USA) are used for transformation, as they are suitable for large plasmids. In a second step, positive clones are transferred into One Shot^{®} TOP10 E. *coli* (Life Technologies Ltd, Paisley, U.K.), which allows the amplification of large size plasmids in significant amounts.

Plasmid pSP1101 thus allows the expression of LAV2 strain prM and E proteins with a YF-VAX replication engine. The resulting chimeric virus is designated CYD-LAV. Sequencing analysis shows no mutation as compared to the original sequences.

### Construction of corresponding plasmids for strains BID-V585, PR/DB023 and MD1280

An analogous strategy to that described above is used to build the plasmids corresponding to the serotype 2 strains BID-V585, PR/DB023 and MD1280. These plasmids are designated pSP1102 (BID-V585), pSP1103 (PR/DB023) and pSP1104 (MD1280). The resulting chimeric viruses generated from those plasmids are designated CYD-BID, CYD-PR and CYD-MD. Sequence analysis of the generated plasmids shows no mutations compared to the original sequences.

### Generation of chimeric viruses from plasmids pSP1101, pSP1102, pSP1103 and pSP1104

*In vitro* transcription of RNA and generation of viruses is carried out as previously described (Guirakhoo F et al. J. Virol. 2001; 75:7290-304).

### Example 4. Assessment of tetravalent dengue vaccine formulations in flavivirusnaïve adults in Mexico

The objective of the present study was to compare the immunogenicity and viremia of a blended tetravalent dengue vaccine comprising CYD-1 (i.e. the particular Chimerivax dengue serotype 1 (CYD-1) strain generated from the prM and E sequences of DEN1 PU0359 (TYP 1 140)), VDV2, CYD-3 (i.e. the particular Chimerivax dengue serotype 3 (CYD-3) strain generated from the prM and E sequences of DEN3 PaH881/88) and CYD-4 (i.e. the particular Chimerivax dengue serotype 4 (CYD-4) strain generated from the prM and E sequences of DEN4 1228 (TVP 980)) with the immunogenicity and viremia of a tetravalent dengue vaccine comprising CYD-1, CYD-2 (i.e. the particular Chimerivax dengue serotype 2 (CYD-2) strain generated from the prM and E sequences of DEN2 PUO218), CYD-3 and CYD-4. See Example 1 for more detail concerning the particular CYD-1, CYD-2, CYD-3 and CYD-4 used in this study.

The relevant nucleotide and protein sequences of the VDV2 strain are as follows:
**>VDV2 nucleotide sequence (SEQ ID NO: 24)** 
AGUUGUUAGUCUACGUGGACCGACAAAGACAGAUUCUUUGAGGAGCUAAGCUCAAUGUAG
UUCUAACAGUUUUUAAUUAGAGAGCAGAUCUCUGAUGAAUAACCAACGGAAAAAGGCGAA
AAACACGCCUUUCAAUAUGCUGAAACGCGAGAGAAACCGCGUGUCGACUGUGCAACAGCUG
ACAAAGAGAUUCUCACUUGGAAUGCUGCAGGGACGAGGACCAUUAAAACUGUUCAUGGCCC
UGGUGGCGUUCCUUCGUUUCCUAACAAUCCCACCAACAGCAGGGAUAUUGAAGAGAUGGGG
AACAAUUAAAAAAUCAAAAGCUAUUAAUGUUUUGAGAGGGUUCAGGAAAGAGAUUGGAAGG
AUGCUGAACAUCUUGAAUAGGAGACGCAGAUCUGCAGGCAUGAUCAUUAUGCUGAUUCCAA
CAGUGAUGGCGUUCCAUUUAACCACACGUAACGGAGAACCACACAUGAUCGUCAGCAGACA
AGAGAAAGGGAAAAGUCUUCUGUUUAAAACAGAGGUUGGCGUGAACAUGUGUACCCUCAUG
GCCAUGGACCUUGGUGAAUUGUGUGAAGACACAAUCACGUACAAGUGUCCCCUUCUCAGGC
 
AGAAUGAGCCAGAAGACAUAGACUGUUGGUGCAACUCUACGUCCACGUGGGUAACUUAUGG
GACGUGUACCACCAUGGGAGAACAUAGAAGAGAAAAAGAUCAGUGGCACUCGUUCCACAU
GUGCGAAUGGGACUGGAGACACGAACUGAAACAUGGAUGUCAUCAGAAGGGGCCUGGAAAC
AUGUCCAGAGAAUUGAAACUUGGAUCUUGAGACAUCCAGGCUUCACCAUGAUGGCAGCAAU
CCUGGCAUACACCAUAGGAACGACACAUUUCCAAAGAGCCCUGAUUUUCAUCUUACUGACA
GCUGUCACUCCUUCAAUGACAAUGCGUUGCAUAGGAAUGUCAAAUAGAGACUUUGUGGAAG
GGGUUUCAGGAGGAAGCUGGGUUGACAUAGUCUUAGAACAUGGAAGCUGUGUGACGACGAU
GGCAAAAAACAAACCAACAUUGGAUUUUGAACUGAUAAAAACAGAAGCCAAACAGCCUGCC
ACCCUAAGGAAGUACUGUAUAGAGGCAAAGCUAACCAACACAACAACAGAAUCUCGCUGCC
CAACACAAGGGGAACCCAGCCUAAAUGAAGAGCAGGACAAAAGGUUCGUCUGCAAACACUC
CAUGGUAGACAGAGGAUGGGGAAAUGGAUGUGGACUAUUUGGAAAGGGAGGCAUUGUGACC
UGUGCUAUGUUCAGAUGCAAAAAGAACAUGGAAGGAAAAGUUGUGCAACCAGAAAACUUGG
AAUACACCAUUGUGAUAACACCUCACUCAGGGGAAGAGCAUGCAGUCGGAAAUGACACAGG
AAAACAUGGCAAGGAAAUCAAAAUAACACCACAGAGUUCCAUCACAGAAGCAGAAUUGACA
GGUUAUGGCACUGUCACAAUGGAGUGCUCUCCAAGAACGGGCCUCGACUUCAAUGAGAUGG
UGUUGCUGCAGAUGGAAAAUAAAGCUUGGCUGGUGCACAGGCAAUGGUUCCUAGACCUGCC
GUUACCAUGGUUGCCCGGAGCGGACACACAAGAGUCAAAUUGGAUACAGAAGGAGACAUUG
GUCACUUUCAAAAAUCCCCAUGCGAAGAAACAGGAUGUUGUUGUUUUAGGAUCCCAAGAAG
GGGCCAUGCACACAGCACUUACAGGGGCCACAGAAAUCCAAAUGUCAUCAGGAAACUUACU
CUUCACAGGACAUCUCAAGUGCAGGCUGAGAAUGGACAAGCUACAGCUCAAAGGAAUGUCA
UACUCUAUGUGCACAGGAAAGUUUAAAGUUGUGAAGGAAAUAGCAGAAACACAACAUGGAA
CAAUAGUUAUCAGAGUGCAAUAUGAAGGGGACGGCUCUCCAUGCAAGAUCCCUUUUGAGAU
AAUGGAUUUGGAAAAAAGACAUGUCUUAGGUCGCCUGAUUACAGUCAACCCAAUUGUGACA
GAAAAAGAUAGCCCAGUCAACAUAGAAGCAGAACCUCCAUUUGGAGACAGCUACAUCAUCA
UAGGAGUAGAGCCGGGACAACUGAAGCUCAACUGGUUUAAGAAAGGAAGUUCUAUCGGCCA
AAUGUUGAGACAACAAUGAGGGGGGCGAAGAGAAUGGCCAUUUUAGGUGACACAGCCUGG
GAUUUUGGAUCCUUGGGAGGAGUGUUUACAUCUAUAGGAAAGGCUCUCCACCAAGUCUUUG
GAGCAAUCUAUGGAGCUGCCUUCAGUGGGGUUUCAUGGACUAUGAAAAUCCUCAUAGGAGU
CAUUAUCACAUGGAUAGGAAUGAAUUCACGCAGCACCUCACUGUCUGUGACACUAGUAUUG
GUGGGAAUUGUGACACUGUAUUUGGGAGUCAUGGUGCAGGCCGAUAGUGGUUGCGUUGUGA
GCUGGAAAAACAAAGAACUGAAAUGUGGCAGUGGGAUUUUCAUCACAGACAACGUGCACAC
AUGGACAGAACAAUACAAAUUCCAACCAGAAUCCCCUUCAAAACUAGCUUCAGCUAUCCAG
AAAGCCCAUGAAGAGGACAUUUGUGGAAUCCGCUCAGUAACAAGACUGGAGAAUCUGAUGU
GGAAACAAAUAACACCAGAAUUGAAUCACAUUCUAUCAGAAAAUGAGGUGAAGUUAACUAU
UAUGACAGGAGACAUCAAAGGAAUCAUGCAGGCAGGAAAACGAUCUCUGCGGCCUCAGCCC
ACUGAGCUGAAGUAUUCAUGGAAAACAUGGGGCAAAGCAAAAAUGCUCUCUACAGAGUCUC
AUAACCAGACCUUUCUCAUUGAUGGCCCCGAAACAGCAGAAUGCCCCAACACAAAUAGAGC
UUGGAAUUCGUUGGAAGUUGAAGACUAUGGCUUUGGAGUAUUCACCACCAAUAUAUGGCUA
AAAUUGAAAGAAAAACAGGAUGUAUUCUGCGACUCAAAACUCAUGUCAGCGGCCAUAAAAG
ACAACAGAGCCGUCCAUGCCGAUAUGGGUUAUUGGAUAGAAAGUGCACUCAAUGACACAUG
GAAGAUAGAGAAAGCCUCUUUCAUUGAAGUUAAAAACUGCCACUGGCCAAAAUCACACACC
CUCUGGAGCAAUGGAGUGCUAGAAAGUGAGAUGAUAAUUCCAAAGAAUCUCGCUGGACCAG
UGUCUCAACACAACUAUAGACCAGGCUACCAUACACAAAUAACAGGACCAUGGCAUCUAGG
UAAGCUUGAGAUGGACUUUGAUUUCUGUGAUGGAACAACAGUGGUAGUGACUGAGGACUGC
GGAAAUAGAGGACCCUCUUUGAGAACAACCACUGCCUCUGGAAAACUCAUAACAGAAUGGU
GCUGCCGAUCUUGCACAUUACCACCGCUAAGAUACAGAGGUGAGGAUGGGUGCUGGUACGG
GAUGGAAAUCAGACCAUUGAAGGAGAAAGAAGAGAAUUUGGUCAACUCCUUGGUCACAGCU
GGACAUGGGCAGGUCGACAACUUUUCACUAGGAGUCUUGGGAAUGGCAUUGUUCCUGGAGG
AAAUGCUUAGGACCCGAGUAGGAACGAAACAUGCAAUACUACUAGUUGCAGUUUCUUUUGU
GACAUUGAUCACAGGGAACAUGUCCUUUAGAGACCUGGGAAGAGUGAUGGUUAUGGUAGGC
 
GCCACUAUGACGGAUGACAUAGGUAUGGGCGUGACUUAUCUUGCCCUACUAGCAGCCUUCA
AAGUCAGACCAACUUUUGCAGCUGGACUACUCUUGAGAAAGCUGACCUCCAAGGAAUUGAU
GAUGACUACUAUAGGAAUUGUACUCCUCUCCCAGAGCACCAUACCAGAGACCAUUCUUGAG
UUGACUGAUGCGUUAGCCUUAGGCAUGAUGGUCCUCAAAAUGGUGAGAAAUAUGGAAAAGU
AUCAAUUGGCAGUGACUAUCAUGGCUAUCUUGUGCGUCCCAAACGCAGUGAUAUUACAAAA
CGCAUGGAAAGUGAGUUGCACAAUAUUGGCAGUGGUGUCCGUUUCCCCACUGUUCUUAACA
UCCUCACAGCAAAAAACAGAUUGGAUACCAUUAGCAUUGACGAUCAAAGGUCUCAAUCCAA
CAGCUAUUUUUCUAACAACCCUCUCAAGAACCAGCAAGAAAAGGAGCUGGCCAUUAAAUGA
GGCUAUCAUGGCAGUCGGGAUGGUGAGCAUUUUAGCCAGUUCUCUCCUAAAAAAUGAUAUU
CCCAUGACAGGACCAUUAGUGGCUGGAGGGCUCCUCACUGUGUGCUACGUGCUCACUGGAC
GAUCGGCCGAUUUGGAACUGGAGAGAGCAGCCGAUGUCAAAUGGGAAGACCAGGCAGAGAU
AUCAGGAAGCAGUCCAAUCCUGUCAAUAACAAUAUCAGAAGAUGGUAGCAUGUCGAUAAAA
AAUGAAGAGGAAGAACAAACACUGACCAUACUCAUUAGAACAGGAUUGCUGGUGAUCUCAG
GACUUUUUCCUGUAUCAAUACCAAUCACGGCAGCAGCAUGGUACCUGUGGGAAGUGAAGAA
ACAACGGGCCGGAGUAUUGUGGGAUGUUCCUUCACCCCCACCCAUGGGAAAGGCUGAACUG
GAAGAUGGAGCCUAUAGAAUUAAGCAAAAAGGGAUUCUUGGAUAUUCCCAGAUCGGAGCCG
GAGUUUACAAAGAAGGAACAUUCCAUACAAUGUGGCAUGUCACACGUGGCGCUGUUCUAAU
GCAUAAAGGAAAGAGGAUUGAACCAACAUGGGCGGACGUCAAGAAAGACCUAAUAUCAUAU
GGAGGAGGCUGGAAGUUAGAAGGAGAAUGGAAGGAAGGAGAAGAAGUCCAGGUAUUGGCAC
UGGAGCCUGGAAAAAAUCCAAGAGCCGUCCAAACGAAACCUGGUCUUUUCAAAACCAACGC
CGGAACAAUAGGUGCUGUAUCUCUGGACUUUUCUCCUGGAACGUCAGGAUCUCCAAUUAUC
GACAAAAAAGGAAAAGUUGUGGGUCUUUAUGGUAAUGGUGUUGUUACAAGGAGUGGAGCAU
AUGUGAGUGCUAUAGCCCAGACUGAAAAAAGCAUUGAAGACAACCCAGAGAUCGAAGAUCA
CAUUUUCCGAAAGAGAAGACUGACCAUCAUGGACCUCCACCCAGGAGCGGGAAAGACGAAG
AGAUACCUUCCGGCCAUAGUCAGAGAAGCUAUAAAACGGGGUUUGAGAACAUUAAUCUUGG
CCCCCACUAGAGUUGUGGCAGCUGAAAUGGAGGAAGCCCUUAGAGGACUUCCAAUAAGAUA
CCAGACCCCAGCCAUCAGAGCUGAGCACACCGGGCGGGAGAUUGUGGACCUAAUGUGUCAU
GCCACAUUUACCAUGAGGCUGCUAUCACCAGUUAGAGUGCCAAACUACAACCUGAUUAUCA
UGGACGAAGCCCAUUUCACAGACCCAGCAAGUAUAGCAGCUAGAGGAUACAUCUCAACUCG
AGUGGAGAUGGGUGAGGCAGCUGGGAUUUUUAUGACAGCCACUCCCCCGGGAAGCAGAGAC
CCAUUUCCUCAGAGCAAUGCACCAAUCAUAGAUGAAGAAAGAGAAAUCCCUGAACGCUCGU
GGAAUUCCGGACAUGAAUGGGUCACGGAUUUUAAAGGGAAGACUGUUUGGUUCGUUCCAAG
UAUAAAAGCAGGAAAUGAUAUAGCAGCUUGCCUGAGGAAAAAUGGAAAGAAAGUGAUACAA
CUCAGUAGGAAGACCUUUGAUUCUGAGUAUGUCAAGACUAGAACCAAUGAUUGGGACUUCG
UGGUUACAACUGACAUUUCAGAAAUGGGUGCCAAUUUCAAGGCUGAGAGGGUUAUAGACCC
CAGACGCUGCAUGAAACCAGUCAUACUAACAGAUGGUGAAGAGCGGGUGAUUCUGGCAGGA
CCUAUGCCAGUGACCCACUCUAGUGCAGCACAAAGAAGAGGGAGAAUAGGAAGAAAUCCAA
AAAAUGAGAAUGACCAGUACAUAUACAUGGGGGAACCUCUGGAAAAUGAUGAAGACUGUGC
ACACUGGAAAGAAGCUAAAAUGCUCCUAGAUAACAUCAACACGCCAGAAGGAAUCAUUCCU
AGCAUGUUCGAACCAGAGCGUGAAAAGGUGGAUGCCAUUGAUGGCGAAUACCGCUUGAGAG
GAGAAGCAAGGAAAACCUUUGUAGACUUAAUGAGAAGAGGAGACCUACCAGUCUGGUUGGC
CUACAGAGUGGCAGCUGAAGGCAUCAACUACGCAGACAGAAGGUGGUGUUUUGAUGGAGUC
AAGAACAACCAAAUCCUAGAAGAAAACGUGGAAGUUGAAAUCUGGACAAAAGAAGGGGAAA
GGAAGAAAUUGAAACCCAGAUGGUUGGAUGCUAGGAUCUAUUCUGACCCACUGGCGCUAAA
AGAAUUUAAGGAAUUUGCAGCCGGAAGAAAGUCUCUGACCCUGAACCUAAUCACAGAAAUG
GGUAGGCUCCCAACCUUCAUGACUCAGAAGGCAAGAGACGCACUGGACAACUUAGCAGUGC
UGCACACGGCUGAGGCAGGUGGAAGGGCGUACAACCAUGCUCUCAGUGAACUGCCGGAGAC
CCUGGAGACAUUGCUUUUACUGACACUUCUGGCUACAGUCACGGGAGGGAUCUUUUUAUUC
UUGAUGAGCGCAAGGGGCAUAGGAAGAUGACCCUGGGAAUGUGCUGCAUAAUCACGGCUA
GCAUCCUCCUAUGGUACGCACAAAUACAGCCACACUGGAUAGCAGCUUCAAUAAUACUGGA
 
GUUUUUCUCAUAGUUUUGCUUAUUCCAGAACCUGAAAAACAGAGAACACCCCAAGACAAC
CAACUGACCUACGUUGUCAUAGCCAUCCUCACAGUGGUGGCCGCAACCAUGGCAAACGAGA
UGGGUUUCCUAGAAAAAACGAAGAAAGAUCUCGGAUUGGAAGCAUUGCAACCCAGCAACC
CGAGAGCAACAUCCUGGACAUAGAUCUACGUCCUGCAUCAGCAUGGACGCUGUAUGCCGUG
GCCACAACAUUUGUUACACCAAUGUUGAGACAUAGCAUUGAAAAUUCCUCAGUGAAUGUGU
CCCUAACAGCUAUAGCCAACCAAGCCACAGUGUUAAUGGGUCUCGGGAAAGGAUGGCCAUU
GUCAAAGAUGGACAUCGGAGUUCCCCUUCUCGCCAUUGGAUGCUACUCACAAGUCAACCCC
AUAACUCUCACAGCAGCUCUUUUCUUAUUGGUAGCACAUUAUGCCAUCAUAGGGCCAGGAC
UCCAAGCAAAAGCAACCAGAGAAGCUCAGAAAAGAGCAGCGGCGGGCAUCAUGAAAAACCC
AACUGUCGAUGGAAUAACAGUGAUUGACCUAGAUCCAAUACCUUAUGAUCCAAAGUUUGAA
AAGCAGUUGGGACAAGUAAUGCUCCUAGUCCUCUGCGUGACUCAAGUAUUGAUGAUGAGGA
CUACAUGGGCUCUGUGUGAGGCUUUAACCUUAGCUACCGGGCCCAUCUCCACAUUGUGGGA
AGGAAAUCCAGGGAGGUUUUGGAACACUACCAUUGCGGUGUCAAUGGCUAACAUUUUUAGA
GGGAGUUACUUGGCCGGAGCUGGACUUCUCUUUUCUAUUAUGAAGAACACAACCAACACAA
GAAGGGGAACUGGCAACAUAGGAGAGACGCUUGGAGAGAAAUGGAAAAGCCGAUUGAACGC
AUUGGGAAAAAGUGAAUUCCAGAUCUACAAGAAAAGUGGAAUCCAGGAAGUGGAUAGAACC
UUAGCAAAAGAAGGCAUUAAAAGAGGAGAAACGGACCAUCACGCUGUGUCGCGAGGCUCAG
CAAAACUGAGAUGGUUCGUUGAGAGAAACAUGGUCACACCAGAAGGGAAAGUAGUGGACCU
CGGUUGUGGCAGAGGAGGCUGGUCAUACUAUUGUGGAGGACUAAAGAAUGUAAGAGAAGUC
AAAGGCCUAACAAAAGGAGGACCAGGACACGAAGAACCCAUCCCCAUGUCAACAUAUGGGU
GGAAUCUAGUGCGUCUUCAAAGUGGAGUUGACGUUUUCUUCAUCCCGCCAGAAAAGUGUGA
CACAUUAUUGUGUGACAUAGGGGAGUCAUCACCAAAUCCCACAGUGGAAGCAGGACGAACA
CUCAGAGUCCUUAACUUAGUAGAAAAUUGGUUGAACAACACUCAAUUUUGCAUAAAGG
UUCUCAACCCAUAUAUGCCCUCAGUCAUAGAAAAAAUGGAAGCACUACAAAGGAAAUAUGG
AGGAGCCUUAGUGAGGAAUCCACUCUCACGAAACUCCACACAUGAGAUGUACUGGGUAUCC
AAUGCUUCCGGGAACAUAGUGUCAUCAGUGAACAUGAUUUCAAGGAUGUUGAUCAACAGAU
UUACAAUGAGAUACAAGAAAGCCACUUACGAGCCGGAUGUUGACCUCGGAAGCGGAACCCG
UAACAUCGGGAUUGAAAGUGAGAUACCAAACCUAGAUAUAAUUGGGAAAAGAAUAGAAAAA
AUAAAGCAAGAGCAUGAAACAUCAUGGCACUAUGACCAAGACCACCCAUACAAAACGUGGG
CAUACCAUGGUAGCUAUGAAACAAAACAGACUGGAUCAGCAUCAUCCAUGGUCAACGGAGU
GGUCAGGCUGCUGACAAAACCUUGGGACGUUGUCCCCAUGGUGACACAGAUGGCAAUGACA
GACACGACUCCAUUUGGACAACAGCGCGUUUUUAAAGAGAAAGUGGACACGAGAACCCAAG
AACCGAAAGAAGGCACGAAGAAACUAAUGAAAAUAACAGCAGAGUGGCUUUGGAAAGAAUU
AGGGAAGAAAAAGACACCCAGGAUGUGCACCAGAGAAGAAUUCACAAGAAAGGUGAGAAGC
AAUGCAGCCUUGGGGGCCAUAUUCACUGAUGAGAACAAGUGGAAGUCGGCACGUGAGGCUG
UUGAAGAUAGUAGGUUUUGGGAGCUGGUUGACAAGGAAAGGAAUCUCCAUCUUGAAGGAAA
GUGUGAAACAUGUGUGUACAACAUGAUGGGAAAAAGAGAGAAGAAGCUAGGGGAAUUCGGC
AAGGCAAAAGGCAGCAGAGCCAUAUGGUACAUGUGGCUUGGAGCACGCUUCUUAGAGUUUG
AAGCCCUAGGAUUCUUAAAUGAAGAUCACUGGUUCUCCAGAGAGAACUCCCUGAGUGGAGU
GGAAGGAGAAGGGCUGCACAAGCUAGGUUACAUUCUAAGAGACGUGAGCAAGAAAGAGGGA
GGAGCAAUGUAUGCCGAUGACACCGCAGGAUGGGAUACAAAAAUCACACUAGAAGACCUAA
AAAAUGAAGAGAUGGUAACAAACCACAUGGAAGGAGAACACAAGAAACUAGCCGAGGCCAU
UUUCAAACUAACGUACCAAAACAAGGUGGUGCGUGUGCAAAGACCAACACCAAGAGGCACA
GUAAUGGACAUCAUAUCGAGAAGAGACCAAAGAGGUAGUGGACAAGUUGGCACCUAUGGAC
UCAAUACUUUCACCAAUAUGGAAGCCCAACUAAUCAGACAGAUGGAGGGAGAAGGAGUCUU
UAAAAGCAUUCAGCACCUAACAAUCACAGAAGAAAUCGCUGUGCAAAACUGGUUAGCAAGA
GUGGGGCGCGAAAGGUUAUCAAGAAUGGCCAUCAGUGGAGAUGAUUGUGUUGUGAAACCUU
UAGAUGACAGGUUCGCAAGCGCUUUAACAGCUCUAAAUGACAUGGGAAAGAUUAGGAAAGA
CAUACAACAAUGGGAACCUUCAAGAGGAUGGAAUGAUUGGACACAAGUGCCCUUCUGUUCA
CACCAUUUCCAUGAGUUAAUCAUGAAAGACGGUCGCGUACUCGUUGUUCCAUGUAGAAACC
 
AAGAUGAACUGAUUGGCAGAGCCCGAAUCUCCCAAGGAGCAGGGUGGUCUUUGCGGAGAC
GGCCUGUUUGGGGAAGUCUUACGCCCAAAUGUGGAGCUUGAUGUACUUCCACAGACGCGAC
CUCAGGCUGGCGGCAAAUGCUAUUUGCUCGGCAGUACCAUCACAUUGGGUUCCAACAAGUC
GAACAACCUGGUCCAUACAUGCUAAACAUGAAUGGAUGACAACGGAAGACAUGCUGACAGU
CUGGAACAGGGUGUGGAUUCAAGAAAACCCAUGGAUGGAAGACAAAACUCCAGUGGAAACA
UGGGAGGAAAUCCCAUACUUGGGGAAAAGAGAAGACCAAUGGUGCGGCUCAUUGAUUGGGU
UAACAAGCAGGGCCACCUGGGCAAAGAACAUCCAAGCAGCAAUAAAUCAAGUUAGAUCCCU
UAUAGGCAAUGAAGAAUACACAGAUUACAUGCCAUCCAUGAAAAGAUUCAGAAGAGAAGAG
GAAGAAGCAGGAGUUCUGUGGUAGAAAGCAAAACUAACAUGAAACAAGGCUAGAAGUCAGG
UCGGAUUAAGCCAUAGUACGGAAAAAACUAUGCUACCUGUGAGCCCCGUCCAAGGACGUUA
AAAGAAGUCAGGCCAUCAUAAAUGCCAUAGCUUGAGUAAACUAUGCAGCCUGUAGCUCCAC
CUGAGAAGGUGUAAAAAAUCCGGGAGGCCACAAACCAUGGAAGCUGUACGCAUGGCGUAGU
GGACUAGCGGUUAGGGGAGACCCCUCCCUUACAAAUCGCAGCAACAAUGGGGGCCCAAGGC
GAGAUGAAGCUGUAGUCUCGCUGGAAGGACUAGAGGUUAGAGGAGACCCCCCCGAAACAAA
AAACAGCAUAUUGACGCUGGGAAAGACCAGAGAUCCUGCUGUCUCCUCAGCAUCAUUCCAG
GCACAGAACGCCAGAAAAUGGAAUGGUGCUGUUGAAUCAACAGGUUCU
**>VDV2 prME nucleotide sequence (SEQ ID NO: 25)** 
UUCCAUUUAACCACACGUAACGGAGAACCACACAUGAUCGUCAGCAGACAAGAGAAAGGA
AAAGUCUUCUGUUUAAAACAGAGGUUGGCGUGAACAUGUGUACCCUCAUGGCCAUGGACCU
UGGUGAAUUGUGUGAAGACACAAUCACGUACAAGUGUCCCCUUCUCAGGCAGAAUGAGCCA
GAAGACAUAGACUGUUGGUGCAACUCUACGUCCACGUGGGUAACUUAUGGGACGUGUACCA
CCAUGGGAGAACAUAGAAGAGAAAAAAGAUCAGUGGCACUCGUUCCACAUGUGCGAAUGGG
ACUGGAGACACGAACUGAAACAUGGAUGUCAUCAGAAGGGGCCUGGAAACAUGUCCAGAGA
AUUGAAACUUGGAUCUUGAGACAUCCAGGCUUCACCAUGAUGGCAGCAAUCCUGGCAUACA
CCAUAGGAACGACACAUUUCCAAAGAGCCCUGAUUUUCAUCUUACUGACAGCUGUCACUCC
UUCAAUGACAAUGCGUUGCAUAGGAAUGUCAAAUAGAGACUUUGUGGAAGGGGUUUCAGGA
GGAAGCUGGGUUGACAUAGUCUUAGAACAUGGAAGCUGUGUGACGACGAUGGCAAAAAACA
AACCAACAUUGGAUUUUGAACUGAUAAAAACAGAAGCCAAACAGCCUGCCACCCUAAGGAA
GUACUGUAUAGAGGCAAAGCUAACCAACACAACAACAGAAUCUCGCUGCCCAACACAAGGG
GAACCCAGCCUAAAUGAAGAGCAGGACAAAAGGUUCGUCUGCAAACACUCCAUGGUAGACA
GAGGAUGGGGAAAUGGAUGUGGACUAUUUGGAAAGGGAGGCAUUGUGACCUGUGCUAUGUU
CAGAUGCAAAAAGAACAUGGAAGGAAAAGUUGUGCAACCAGAAAACUUGGAAUACACCAUU
GUGAUAACACCUCACUCAGGGGAAGAGCAUGCAGUCGGAAAUGACACAGGAAAACAUGGCA
AGGAAAUCAAAAUAACACCACAGAGUUCCAUCACAGAAGCAGAAUUGACAGGUUAUGGCAC
UGUCACAAUGGAGUGCUCUCCAAGAACGGGCCUCGACUUCAAUGAGAUGGUGUUGCUGCAG
AUGGAAAAUAAAGCUUGGCUGGUGCACAGGCAAUGGUUCCUAGACCUGCCGUUACCAUGGU
UGCCCGGAGCGGACACACAAGAGUCAAAUUGGAUACAGAAGGAGACAUUGGUCACUUUCAA
AAAUCCCCAUGCGAAGAAACAGGAUGUUGUUGUUUAGGAUCCCAAGAAGGGGCCAUGCAC
ACAGCACUUACAGGGGCCACAGAAAUCCAAAUGUCAUCAGGAAACUUACUCUUCACAGGAC
AUCUCAAGUGCAGGCUGAGAAUGGACAAGCUACAGCUCAAAGGAAUGUCAUACUCUAUGUG
CACAGGAAAGUUUAAAGUUGUGAAGGAAAUAGCAGAAACACAACAUGGAACAAUAGUUAUC
AGAGUGCAAUAUGAAGGGGACGGCUCUCCAUGCAAGAUCCCUUUUGAGAUAAUGGAUUUGG
AAAAAAGACAUGUCUUAGGUCGCCUGAUUACAGUCAACCCAAUUGUGACAGAAAAAGAUAG
CCCAGUCAACAUAGAAGCAGAACCUCCAUUUGGAGACAGCUACAUCAUCAUAGGAGUAGAG
CCGGGACAACUGAAGCUCAACUGGUUUAAGAAAGGAAGUUCUAUCGGCCAAAUGUUUGAGA
CAACAAUGAGGGGGGCGAAGAGAAUGGCCAUUUUAGGUGACACAGCCUGGGAUUUUGGAUC
CUUGGGAGGAGUGUUUACAUCUAUAGGAAAGGCUCUCCACCAAGUCUUUGGAGCAAUCUAU
GGAGCUGCCUUCAGUGGGGUUUCAUGGACUAUGAAAAUCCUCAUAGGAGUCAUUAUCACAU
 
GGAUAGGAAUGAAUUCACGCAGCACCUCACUGUCUGUGACACUAGUAUUGGUGGGAAUUGU
GACACUGUAUUUGGGAGUCAUGGUGCAGGCC
**>VDV2 E protein sequence (SEQ ID NO: 26)** 
MRCIGMSNRDFVEGVSGGSWVDIVLEHGSCVTTMAKNKPTLDFELIKTEAKQPATLRKYCI
EAKLTNTTTESRCPTQGEPSLNEEQDKRFVCKHSMVDRGWGNGCGLFGKGGIVTCAMFRCK
KNMEGKVVQPENLEYTIVITPHSGEEHAVGNDTGKHGKEIKITPQSSITEAELTGYGTVTM
ECSPRTGLDFNEMVLLQMENKAWLVHRQWFLDLPLPWLPGADTQESNWIQKETLVTFKNPH
AKKQDVVVLGSQEGAMHTALTGATEIQMSSGNLLFTGHLKCRLRMDKLQLKGMSYSMCTGK
FKVVKEIAETQHGTIVIRVQYEGDGSPCKIPFEIMDLEKRHVLGRLITVNPIVTEKDSPVN
IEAEPPFGDSYIIIGVEPGQLKLNWFKKGSSIGQMFETTMRGAKRMAILGDTAWDFGSLGG
VFTSIGKALHQVFGAIYGAAFSGVSWTMKILIGVIITWIGMNSRSTSLSVTLVLVGIVTLY
LGVMVQA
**>VDV2 M protein sequence (SEQ ID NO: 27)**

SVALVPHVRMGLETRTETWMSSEGAWKHVQRIETWILRHPGFTMMAAILAYTIGTTHFQRA
LIFILLTAVTPSMT

### Study design

In an open, randomised, controlled, phase IIa trial, 150 healthy adults aged 18-45 years were enrolled at two centres in Mexico City, which is a dengue non-endemic area. Main exclusion criteria were: pregnancy or breast-feeding, human immunodeficiency virus, hepatitis B or C seropositivity, immunodeficiency or any other chronic illness that could interfere with the results, previous residence in or travel of >2 weeks to areas with high dengue endemicity, a history of flavivirus infection or previous vaccination against flavivirus disease. Women who were capable of conceiving were required to use an effective method of contraception or abstinence for at least 4 weeks before the first injection until at least four weeks after the last injection.

Participants were randomised into two groups and vaccinations were performed on Day 0 and Day 105 (±15 days). The groups received the following formulations:
**Group 1:** Blended CYD/VDV2 tetravalent formulation, i.e. a formulation comprising CYD-1, CYD-3, CYD4 and VDV2.
**Group 2:** Control tetravalent formulation (CYD-TDV), i.e. CYD-1, CYD-2, CYD-3 and CYD-4.

The formulations contained 10⁵ CCID₅₀ of each serotype of the CYD viruses and the formulation administered to Group 1 contained 10⁴ CCID₅₀ of the VDV-2 virus.

### Viremia

To evaluate the safety of the vaccines, the presence of CYD-1―4 or VDV-2 was assessed in serum collected 7, 14 and 21 days after each injection. Analyses were performed by the Global Clinical Immunology laboratory (Sanofi Pasteur, Swiftwater, PA, USA).

Analyses for CYD-1―4 viremia were performed in two steps, as previously described in Poo et al., Pediatr Infect Dis J (2011) 30: e9. Briefly, a first, non-serotype-specific, reverse transcriptase-polymerase chain reaction (RT-PCR) was used to detect the presence of any of the four CYD viruses. Samples that were positive in this first test were then analysed using four CYD serotype-specific quantitative RT-PCRs. In the non-serotype-specific RT-PCR, RNA was extracted from the serum using a commercial kit and an RT-PCR was carried out with primers from the yellow fever core gene sequence. In the serotype-specific RT-PCRs, RNA was again extracted from the serum using a commercial kit and an RT-PCR was carried out with serotype-specific primers from the envelope non-structural protein 1 junction gene sequence for each serotype. A dengue RT-PCR for serotype 2 was performed in group 1 since the tetravalent blending formulation administered to this group contained the VDV-2 virus.

### Immunogenicity

Antibody levels to each of the four dengue virus serotypes were determined by 50% plaque reduction neutralisation test on serum collected 28 days after each injection as well as on day 365 after the first injection. Briefly, serial 2-fold dilutions of heat-inactivated serum were mixed with a constant challenge dose of each dengue serotype DEN-1, -2, -3, or -4 (expressed as plaque forming unit [PFU]/mL). The mixtures were inoculated into wells of a 24-well plate of confluent VERO cell monolayers. After incubation for several days, dengue virus infection is indicated by formation of plaques. The neutralising antibody titre is calculated as the highest reciprocal dilution (1/dil) of serum at which ≥50% reduction in viral plaque count is observed (PRNT50). The lower limit of quantitation of the dengue PRNT50 is 10; samples with titres ≥10 were considered seropositive.

### Results

Formulations were administered to participants in Groups 1 and 2 on day 0 and day 105 of the study. There were no marked differences between the two groups with regard to the injection site or systemic reactogenicity after either the first or the second vaccination. Viremia was assessed in serum collected 7, 14 and 21 days after each injection (Table 6). The neutralising antibody titres were measured 28 days after each injection and on day 365 after the first injection (Table 7).

**Table 6. Vaccine virus viremia 7, 14, or 21 days after first and second injections (n (%) with detectable and quantifiable viremia)**

| | First injection | | Second injection | |
|---|---|---|---|---|
| | Group 1 Blended CYD/VDV | Group 2 Tetravalent CYD-TDV | Group 1 Blended CYD/VDV | Group 2 Tetravalent CYD-TDV |
| Non-serotype specific | | | | |
| N | 29 | 31 | 28 | 29 |
| Detectable viraemia | 27 (93%) | 25 (81%) | 1 (4%) | 1 (3%) |
| Quantifiable viraemia | 1 (3%) | 2 (6%) | 0 | 0 |
| DENV-1 | | | | |
| Detectable viraemia | 1 (3%) | 4 (13%) | 0 | 0 |
| Quantifiable viraemia | 0 | 2 (7%) | 0 | 0 |

| DENV-2 | | | | |
|---|---|---|---|---|
| Detectable viraemia | 0 | 2 (6%) | 0 | 0 |
| Quantifiable viraemia | 0 | 0 | 0 | 0 |

| DENV-3 | | | | |
|---|---|---|---|---|
| Detectable viraemia | 8 (28%) | 7 (23%) | 1 (4%) | 0 |
| Quantifiable viraemia | 0 | 0 | 0 | 0 |

| DENV-4 | | | | |
|---|---|---|---|---|
| Detectable viraemia | 24 (83%) | 21 (68%) | 0 | 0 |
| Quantifiable viraemia | 0 | 3 (1%) | 0 | 0 |

After the first injection, detectable viremia, as determined by the non-serotype specific RT-PCR test, was observed in a similar proportion of participants in both groups (see Table 6). In the majority of cases, viremia was below the lower limit of quantitation. Analysis with the serotype-specific assays showed that CYD-4 was the most commonly detected serotype, followed by CYD-3. After the second injection of the blended CYD/VDV vaccine in Group 1 or the CYD-TDV vaccine in Group 2, viremia was only detected in one participant per group by the non-serotype-specific assay.

Accordingly, there was no significant difference between the levels of viremia induced by the blended CYD/VDV and CYD-TDV.

**Table 7. Geometric mean titres (95% confidence interval) of dengue antibodies 28 days after the first and second injections and 365 days after the first injection**

| | **Group 1 CYD/VDV blended** | **Group 2 CYD-TDV** |
|---|---|---|
| **First injection** | | |
| Serotype 1 | 15 (9;28) | 17 (10;31) |
| Serotype 2 | 17 (8;33) | 32 (16;65) |
| Serotype 3 | 64 (31;133) | 23 (13;39) |
| Serotype 4 | 552 (299;1019) | 468 (226;968) |

| **Second injection** | | |
|---|---|---|
| Serotype 1 | 54 (30;96) | 28 (15;50) |
| Serotype 2 | 152 (79;293) | 43 (23;79) |
| Serotype 3 | 127 (71;229) | 46 (29;73) |
| Serotype 4 | 246 (159;382) | 173 (97;307) |

| **365 days post-dose 1** | | |
|---|---|---|
| Serotype 1 | 14 (9;22) | 18 (10;30) |
| Serotype 2 | 55 (32;94) | 16 (9;29) |
| Serotype 3 | 36 (20;64) | 11 (7;16) |
| Serotype 4 | 103 (69;155) | 72 (44;117) |

It can be seen from Table 7 that the second injection of the blended CYD/VDV vaccine (Group 1) induced higher GMTs against serotype 2 of dengue virus than the CYD-TDV vaccine (Group 2). An improved response to serotype 2 in the blended CYD/VDV group was also observed 365 days after the first dose.

Furthermore, the second injection of the blended CYD/VDV vaccine (Group 1) resulted in an improved neutralising antibody response against all serotypes of dengue virus when compared with the group receiving the CYD-TDV vaccine (Group 2). Importantly, the blended CYD/VDV formulation group demonstrated a more persistent neutralising antibody response against dengue virus than the CYD-TDV group on day 365 after the first injection.

The example therefore shows that, overall, the blended CYD-1, 3, 4/VDV2 vaccine formulation induces stronger and longer lasting immune responses against the dengue virus serotypes than the CYD-TDV vaccine while showing a similar safety profile, as determined by the levels of viremia.

### Sequence Listing

| SEQ ID NO. | Sequence |
|---|---|
| 1 | prM+E CYD23 circulating strain nucleotide sequence |
| 2 | prM+E CYD23 circulating strain protein sequence |
| 3 | prM+E consensus serotype 2 protein sequence |
| 4 | prM+E LAV2 nucleotide sequence |
| 5 | prM+E BID/V585 nucleotide sequence |
| 6 | prM+E PR/DB023 nucleotide sequence |
| 7 | prM+E MD1280 nucleotide sequence |
| 8 | prM+E LAV2 protein sequence |
| 9 | prM+E BID/V585 protein sequence |
| 10 | prM+E PR/DB023 protein sequence |
| 11 | prM+E MD1280 protein sequence |
| 12 | E consensus serotype 2 protein sequence |
| 13 | E LAV2 protein sequence |
| 14 | E BID/V585 protein sequence |
| 15 | E PR/DB023 protein sequence |
| 16 | E MD1280 protein sequence |
| 17 | M consensus serotype 2 protein sequence |
| 18 | E CYD23 circulating strain protein sequence |
| 19 | M LAV2 protein sequence |
| 20 | M BID/V585 protein sequence |
| 21 | M PR/DB023 protein sequence |
| 22 | M MD1280 protein sequence |
| 23 | M CYD23 circulating strain protein sequence |
| 24 | Entire nucleotide sequence of VDV2 (RNA equivalent) |
| 25 | prM+E VDV2 nucleotide sequence (RNA equivalent) |
| 26 | E VDV2 protein sequence |
| 27 | M VDV2 protein sequence |

In the listed nucleotide sequences, where a nucleotide sequence is DNA, the nucleotide T may be replaced with the nucleotide U to give the RNA equivalent of that DNA sequence. Similarly, where a nucleotide sequence is RNA, the nucleotide U may be replaced by the nucleotide T to give the equivalent DNA sequence. The DNA sequences listed above constitute the cDNA sequences of the noted dengue viruses and therefore the equivalent RNA sequences constitute the positive strand RNA of those dengue viruses.

### Embodiments

Also described herein are the following embodiments.
1. A vaccine composition for use in a method of protecting a human subject against dengue disease, wherein said composition comprises:
   (i) a dengue antigen selected from the group consisting of:
      (a) a live attenuated dengue virus;
      (b) an inactivated dengue virus;
      (c) a live attenuated or inactivated chimeric dengue virus;
      (d) a dengue virus-like particle (VLP); and
      (e) a combination of two or more of (a) to (d);
      or
   (ii) a nucleic acid construct or viral vector which is able to express in a human cell a dengue antigen which is a dengue VLP.
2. A composition for use according to embodiment 1, wherein said human is 4-11 years of age.
3. A composition for use according to any preceding embodiment, wherein said subject resides in a dengue endemic area.
4. A composition for use according to any preceding embodiment, wherein said subject is flavivirus immune.
5. A composition for use according to any one of embodiments 1 to 4, wherein said subject is flavivirus naïve.
6. A composition for use in a method according to any preceding embodiment, wherein said method reduces the incidence or likelihood of dengue disease.
7. A composition for use in a method according to any preceding embodiment, wherein dengue disease is virologically-confirmed.
8. A composition for use in a method according to any preceding embodiment, wherein said dengue disease is caused by a dengue virus of serotype 1, serotype 3 or serotype 4.
9. A composition for use according to any preceding embodiment, wherein said method comprises administering said composition in multiple doses.
10. A composition for use according to embodiment 8, wherein said method comprises administering said composition in three or more doses.
11. A composition for use according to embodiment 8 or embodiment 9, wherein a first and a third dose are to be administered approximately twelve months apart.
12. A composition for use according to any one of embodiments 8 to 10, wherein said method comprises administering said composition in a first dose, a second dose and a third dose and wherein said second dose is to be administered about six months after said first dose and wherein said third dose is to be administered about twelve months after said first dose.
13. A composition for use according to any preceding embodiment, wherein said composition comprises a dengue antigen of serotype 1, a dengue antigen of serotype 2, a dengue antigen of serotype 3 and a dengue antigen of serotype 4.
14. A composition for use according to any preceding embodiment, wherein said composition comprises a live attenuated dengue virus or a live attenuated chimeric dengue virus.
15. A composition for use according to embodiment 13, wherein said dengue antigens of serotypes 1, 2, 3 and 4 are each independently selected from a live attenuated dengue virus or a live attenuated chimeric dengue virus.
16. A composition for use according to embodiment 15, wherein said dengue antigens of serotypes 1, 3 and 4 are each a live attenuated chimeric dengue virus and said dengue antigen of serotype 2 is selected from the group consisting of a live attenuated dengue virus and a live attenuated chimeric dengue virus, preferably a live attenuated dengue virus.
17. A composition for use according to embodiment 16, wherein said dengue antigen of serotype 2 is a live attenuated dengue virus which comprises a nucleic acid sequence having at least 90% sequence identity to the sequence as set forth in SEQ ID NO: 24.
18. A composition for use according to embodiment 15, wherein said dengue antigens of serotypes 1, 2, 3 and 4 are each a live attenuated chimeric dengue virus.
19. A composition for use according to any one of embodiments 1 to 18, wherein said composition comprises a live attenuated chimeric dengue virus.
20. A composition for use according to any preceding embodiment, wherein said chimeric dengue virus comprises one or more proteins from a dengue virus and one or more proteins from a different flavivirus.
21. A composition for use according to any one of embodiments 1 to 19, wherein said chimeric dengue virus comprises one or more proteins from a dengue virus of one serotype and one or more proteins from a dengue virus of a different serotype.
22. A composition for use according to embodiment 19 or 20, wherein the different flavivirus is a yellow fever virus.
23. A composition for use according to embodiment 21 or 22, wherein the yellow fever virus is strain YF17D.
24. A composition for use according to any one of embodiments 13 to 23, wherein said chimeric dengue virus comprises the prM-E sequence of a dengue virus.
25. A composition for use according to any one of embodiments 13 to 20 and 22 to 24, wherein the chimeric dengue virus comprises a yellow fever virus genome whose prM-E sequence has been substituted with the prM-E sequence of a dengue virus.
26. A composition for use according to any one of embodiments 13 to 25, wherein said composition comprises the prM-E sequences of CYD-1, CYD-2, CYD-3 and CYD-4.
27. A composition for use according to any one of embodiments 13 to 20 and 22 to 26, wherein said composition comprises CYD-1, CYD-2, CYD-3 and CYD-4.
28. A composition for use according to any one of embodiments 13 to 17, 19 to 20 and 22 to 25 and 21 to 25, wherein said composition comprises CYD-1, VDV-2, CYD-3 and CYD-4.
29. A composition for use according to any one of embodiments 13 to 28, wherein at least one serotype of said dengue virus is present in an amount of from about 10³ to about 10⁶ CCID₅₀.
30. A composition for use according to embodiments 13 to 28, wherein at least one serotype of said dengue virus is present in an amount of from about 10³ to about 10⁷ CCID₅₀.
31. A composition for use according to embodiment 27 or embodiment 29, wherein said composition comprises all four serotypes of dengue virus and each of said serotypes is present in an amount of from about 10³ to about 10⁶ CCID₅₀.
32. A composition for use according to embodiment 30, wherein said composition comprises all four serotypes of dengue virus and each of said serotypes is present in an amount of from about 10³ to about 10⁷ CCID₅₀.
33. A composition for use according to any one of embodiments 13 to 32, wherein at least one serotype of said dengue virus is present in an amount of about 10⁵ CCID₅₀.
34. A composition for use according to any one of embodiments 13 to 32, wherein at least one serotype of said dengue virus is present in an amount of about 10⁶ CCID₅₀.
35. A composition for use according to embodiment 31, wherein said composition comprises all four serotypes of dengue virus and each of said serotypes is present in an amount of about 10⁵ CCID₅₀.
36. A composition for use according to embodiment 32, where said composition comprises all four serotypes of dengue virus and each of said serotypes is present in an amount of about 10⁶ CCID₅₀.
37. A composition for use according to any preceding embodiment, wherein said composition is lyophilized.
38. A composition for use according to any preceding embodiment, wherein said method comprises administering said composition to said human subject via a subcutaneous route.
39. A composition for use according to any preceding embodiment, wherein said composition comprises a pharmaceutically acceptably carrier or excipient.
40. A method of protecting a human subject against dengue disease, wherein said method comprises administering to said human subject an effective amount of a composition according to any one of embodiments 1 to 39.
41. A kit comprising a composition according to any one of embodiments 1 to 39 and instructions for the use of said composition in a method of protecting a human subject against dengue disease.

## Claims

1. A vaccine composition for use in a method of protecting a human subject against dengue disease, wherein said composition comprises a dengue antigen of serotype 1, a dengue antigen of serotype 2, a dengue antigen of serotype 3 and a dengue antigen of serotype 4; wherein said dengue antigens of serotypes 1, 2, 3 and 4 are each independently selected from a live attenuated dengue virus or a live attenuated chimeric dengue virus and wherein said chimeric dengue virus comprises the prM-E sequence of a dengue virus.

2. A composition for use according to claim 1, wherein said human is 2-45 years of age.

3. A composition for use according to any claim 1 or claim 2, wherein said subject is dengue immune.

4. A composition for use in a method according to any preceding claim, wherein said method reduces the incidence or likelihood of dengue disease.

5. A composition for use according to any preceding claim, wherein said method comprises administering said composition in three doses.

6. A composition for use according to any one of claims 1 to 4, wherein said method comprises administering said composition in two doses.

7. A composition for use according to any one of claims 1 to 4, wherein said method comprises administering said composition in a first dose and a second dose and wherein said second dose is to be administered approximately six to twelve months after the first dose.

8. A composition for use according to any one of claims 1 to 5, wherein said method comprises administering said composition in a first dose, a second dose and a third dose and wherein said second dose is to be administered about six months after said first dose and wherein said third dose is to be administered about twelve months after said first dose.

9. A composition for use according to any preceding claim, wherein said dengue antigens of serotypes 1, 3 and 4 are each a live attenuated chimeric dengue virus and said dengue antigen of serotype 2 is selected from the group consisting of a live attenuated dengue virus and a live attenuated chimeric dengue virus, preferably a live attenuated dengue virus.

10. A composition for use according to any preceding claim, wherein said dengue antigens of serotypes 1, 2, 3 and 4 are each a live attenuated chimeric dengue virus.

11. A composition for use according to any preceding claim, wherein said chimeric dengue virus comprises one or more proteins from a dengue virus and one or more proteins from a different flavivirus.

12. A composition for use according to claim 11, wherein the different flavivirus is a yellow fever virus.

13. A composition for use according to any preceding claim, wherein the chimeric dengue virus comprises a yellow fever virus genome whose prM-E sequence has been substituted with the prM-E sequence of a dengue virus.

14. A composition for use according to any preceding claim, wherein said composition comprises all four serotypes of dengue virus and each of said serotypes is present in an amount of from about 10³ to about 10⁶ CCID₅₀.

15. A composition for use according to any preceding claim, wherein said method comprises administering said composition to said human subject via a subcutaneous route.


SEQUENCE LISTING

<110>  Sanofi Pasteur

<120>  Vaccine Compositions

<130>  P031359EP1

<150>  EP12305912.3
<151>  2012-07-25

<150>  EP12305907.3
<151>  2012-07-24

<160>  27

<170>  PatentIn version 3.5

<210>  1
<211>  1983
<212>  DNA
<213>  Artificial Sequence

<220>
<223>  Clinical trial circulating strain

<400>  1
ttccatctaa ccacacgcaa cggagaacca cacatgatcg tcggtataca ggagaaaggg      60

aaaagtcttc tgttcaaaac agaggatggt gtgaacatgt gcaccctcat ggctatggac     120

cttggtgaat tgtgtgaaga cacaatcacg tacaagtgtc ctcttctcag gcagaatgag     180

ccagaagaca tagactgttg gtgcaactcc acgtccacgt gggtaaccta tgggacctgt     240

accactacgg gagaacatag gagagaaaaa agatcagtgg cactcgttcc acatgtggga     300

atgggactgg agacgcgaac cgaaacatgg atgtcatcag aaggggcttg gaaacatgcc     360

cagagaattg aaacttggat cctgagacat ccaggcttca ccataatggc agcaatcctg     420

gcatacacca taggaacgac acatttccag agagtcctga ttttcatcct actgacagct     480

gtcgctcctt caatgacaat gcgttgcata ggaatatcaa atagagactt tgtagaaggg     540

gtttcaggag gaagttgggt tgacatagtc ttagaacatg gaagctgtgt gacgacgatg     600

gcaaaaaaca aaccaacatt ggatttcgaa ctgataaaaa cggaagccaa acagcctgcc     660

accctaagga agtactgcat agaagcaaaa ctaaccaaca caacaacaga atcccgttgc     720

ccaacacaag gggaacccag cctaaaagaa gagcaggaca agaggttcgt ctgcaaacac     780

tccatggtag acagaggatg gggaaatgga tgtggattat ttggaaaggg aggcattgtg     840

acctgtgcta tgttcacatg caaaaagaac atggaaggga aaatcgtgca accagaaaac     900

ttggaataca ccattgtggt aacacctcac tcaggggaag agcatgcggt cggaaatgac     960

acaggaaaac acggcaagga aatcaaagta acaccacaga gttccatcac agaagcagaa    1020

ctgacaggtt atggcaccgt cacgatggag tgctccccga gaacaggcct cgacttcaat    1080

gagatggtgt tgctgcagat ggaaaataaa gcttggctgg tgcataggca atggtttcta 1140

gacctgccat taccatggct gcccggagcg gataaacaag aatcaaattg gatacagaaa 1200

gaaacattgg tcactttcaa aaatccccat gcgaagaaac aggatgttgt tgttttagga 1260

tcccaagaag gggccatgca tacagcactc acaggagcca cagaaatcca aatgtcgtca 1320

ggaaacttgc tcttcactgg acatctcaag tgcaggctga gaatggacaa gctacagctt 1380

aaaggaatgt catactctat gtgcacagga aagtttaaag ttgtgaagga aatagcagaa 1440

acacaacatg gaacgatagt tatcagagtg caatatgaag gggacggctc tccatgtaaa 1500

attccttttg agataatgga tttggaaaaa agatatgtct taggccgcct gatcacagtc 1560

aacccaattg taacagaaaa agacagccca gtcaacatag aagcagaacc tccattcgga 1620

gacagttaca tcatcatagg agtagagccg ggacaactga agctcaactg gttcaagaaa 1680

ggaagttcta tcggccaaat gtttgagaca acgatgagag gggcgaagag aatggccatt 1740

ttgggtgaca cagcctggga cttcggatcc ctgggaggag tgtttacatc tataggaaaa 1800

gctctccacc aagtctttgg agcgatctat ggggctgcct tcagtggggt ttcatggacc 1860

atgaaaatcc tcataggagt cattatcaca tggataggaa tgaactcacg cagcacctca 1920

ctgtctgtgt cactggtact ggtgggaatt gtgacactgt atttaggagt catggtgcag 1980

gcc 1983

<210> 2
<211> 661
<212> PRT
<213> Artificial Sequence

<220>
<223> Clinical trial circulating strain

<400> 2

Phe His Leu Thr Thr Arg Asn Gly Glu Pro His Met Ile Val Gly Ile
1               5                   10                  15

Gln Glu Lys Gly Lys Ser Leu Leu Phe Lys Thr Glu Asp Gly Val Asn
            20                  25                  30

Met Cys Thr Leu Met Ala Met Asp Leu Gly Glu Leu Cys Glu Asp Thr
        35                  40                  45

Ile Thr Tyr Lys Cys Pro Leu Leu Arg Gln Asn Glu Pro Glu Asp Ile
    50                  55                  60

Asp Cys Trp Cys Asn Ser Thr Ser Thr Trp Val Thr Tyr Gly Thr Cys
65                  70                  75                  80

Thr Thr Thr Gly Glu His Arg Arg Glu Lys Arg Ser Val Ala Leu Val
                85                  90                  95

Pro His Val Gly Met Gly Leu Glu Thr Arg Thr Glu Thr Trp Met Ser
            100                 105                 110

Ser Glu Gly Ala Trp Lys His Ala Gln Arg Ile Glu Thr Trp Ile Leu
        115                 120                 125

Arg His Pro Gly Phe Thr Ile Met Ala Ala Ile Leu Ala Tyr Thr Ile
    130                 135                 140

Gly Thr Thr His Phe Gln Arg Val Leu Ile Phe Ile Leu Leu Thr Ala
145                 150                 155                 160

Val Ala Pro Ser Met Thr Met Arg Cys Ile Gly Ile Ser Asn Arg Asp
                165                 170                 175

Phe Val Glu Gly Val Ser Gly Gly Ser Trp Val Asp Ile Val Leu Glu
            180                 185                 190

His Gly Ser Cys Val Thr Thr Met Ala Lys Asn Lys Pro Thr Leu Asp
        195                 200                 205

Phe Glu Leu Ile Lys Thr Glu Ala Lys Gln Pro Ala Thr Leu Arg Lys
    210                 215                 220

Tyr Cys Ile Glu Ala Lys Leu Thr Asn Thr Thr Thr Glu Ser Arg Cys
225                 230                 235                 240

Pro Thr Gln Gly Glu Pro Ser Leu Lys Glu Glu Gln Asp Lys Arg Phe
                245                 250                 255

Val Cys Lys His Ser Met Val Asp Arg Gly Trp Gly Asn Gly Cys Gly
            260                 265                 270

Leu Phe Gly Lys Gly Gly Ile Val Thr Cys Ala Met Phe Thr Cys Lys
        275                 280                 285

Lys Asn Met Glu Gly Lys Ile Val Gln Pro Glu Asn Leu Glu Tyr Thr
    290                 295                 300

Ile Val Val Thr Pro His Ser Gly Glu Glu His Ala Val Gly Asn Asp
305                 310                 315                 320

Thr Gly Lys His Gly Lys Glu Ile Lys Val Thr Pro Gln Ser Ser Ile
                325                 330                 335

Thr Glu Ala Glu Leu Thr Gly Tyr Gly Thr Val Thr Met Glu Cys Ser
            340                 345                 350

Pro Arg Thr Gly Leu Asp Phe Asn Glu Met Val Leu Leu Gln Met Glu
        355                 360                 365

Asn Lys Ala Trp Leu Val His Arg Gln Trp Phe Leu Asp Leu Pro Leu
    370                 375                 380

Pro Trp Leu Pro Gly Ala Asp Lys Gln Glu Ser Asn Trp Ile Gln Lys
385                 390                 395                 400

Glu Thr Leu Val Thr Phe Lys Asn Pro His Ala Lys Lys Gln Asp Val
                405                 410                 415

Val Val Leu Gly Ser Gln Glu Gly Ala Met His Thr Ala Leu Thr Gly
            420                 425                 430

Ala Thr Glu Ile Gln Met Ser Ser Gly Asn Leu Leu Phe Thr Gly His
        435                 440                 445

Leu Lys Cys Arg Leu Arg Met Asp Lys Leu Gln Leu Lys Gly Met Ser
    450                 455                 460

Tyr Ser Met Cys Thr Gly Lys Phe Lys Val Val Lys Glu Ile Ala Glu
465                 470                 475                 480

Thr Gln His Gly Thr Ile Val Ile Arg Val Gln Tyr Glu Gly Asp Gly
                485                 490                 495

Ser Pro Cys Lys Ile Pro Phe Glu Ile Met Asp Leu Glu Lys Arg Tyr
            500                 505                 510

Val Leu Gly Arg Leu Ile Thr Val Asn Pro Ile Val Thr Glu Lys Asp
        515                 520                 525

Ser Pro Val Asn Ile Glu Ala Glu Pro Pro Phe Gly Asp Ser Tyr Ile
    530                 535                 540

Ile Ile Gly Val Glu Pro Gly Gln Leu Lys Leu Asn Trp Phe Lys Lys
545                 550                 555                 560

Gly Ser Ser Ile Gly Gln Met Phe Glu Thr Thr Met Arg Gly Ala Lys
                565                 570                 575

Arg Met Ala Ile Leu Gly Asp Thr Ala Trp Asp Phe Gly Ser Leu Gly
            580                 585                 590

Gly Val Phe Thr Ser Ile Gly Lys Ala Leu His Gln Val Phe Gly Ala
        595                 600                 605

Ile Tyr Gly Ala Ala Phe Ser Gly Val Ser Trp Thr Met Lys Ile Leu
    610                 615                 620

Ile Gly Val Ile Ile Thr Trp Ile Gly Met Asn Ser Arg Ser Thr Ser
625                 630                 635                 640

Leu Ser Val Ser Leu Val Leu Val Gly Ile Val Thr Leu Tyr Leu Gly
                645                 650                 655

Val Met Val Gln Ala
            660

<210>  3
<211>  661
<212>  PRT
<213>  Artificial Sequence

<220>
<223>  prM+E universal serotype 2

<220>
<221>  Variant
<222>  (295)..(295)
<223>  Xaa can be Val or Ile

<220>
<221>  Variant
<222>  (474)..(474)
<223>  Xaa can be Val or Ile

<400>  3

Phe His Leu Thr Thr Arg Asn Gly Glu Pro His Met Ile Val Gly Arg
1               5                   10                  15

Gln Glu Lys Gly Lys Ser Leu Leu Phe Lys Thr Glu Asp Gly Val Asn
            20                  25                  30

Met Cys Thr Leu Met Ala Ile Asp Leu Gly Glu Leu Cys Glu Asp Thr
        35                  40                  45

Ile Thr Tyr Lys Cys Pro Leu Leu Arg Gln Asn Glu Pro Glu Asp Ile
    50                  55                  60

Asp Cys Trp Cys Asn Ser Thr Ser Thr Trp Val Thr Tyr Gly Thr Cys
65                  70                  75                  80

Thr Thr Thr Gly Glu His Arg Arg Glu Lys Arg Ser Val Ala Leu Val
                85                  90                  95

Pro His Val Gly Met Gly Leu Glu Thr Arg Thr Glu Thr Trp Met Ser
            100                 105                 110

Ser Glu Gly Ala Trp Lys His Val Gln Arg Ile Glu Thr Trp Ile Leu
        115                 120                 125

Arg His Pro Gly Phe Thr Ile Met Ala Ala Ile Leu Ala Tyr Thr Ile
    130                 135                 140

Gly Thr Thr His Phe Gln Arg Ala Leu Ile Phe Ile Leu Leu Thr Ala
145                 150                 155                 160

Val Ala Pro Ser Met Thr Met Arg Cys Ile Gly Ile Ser Asn Arg Asp
                165                 170                 175

Phe Val Glu Gly Val Ser Gly Gly Ser Trp Val Asp Ile Val Leu Glu
            180                 185                 190

His Gly Ser Cys Val Thr Thr Met Ala Lys Asn Lys Pro Thr Leu Asp
        195                 200                 205

Phe Glu Leu Ile Lys Thr Glu Ala Lys Gln Pro Ala Thr Leu Arg Lys
    210                 215                 220

Tyr Cys Ile Glu Ala Lys Leu Thr Asn Thr Thr Thr Glu Ser Arg Cys
225                 230                 235                 240

Pro Thr Gln Gly Glu Pro Ser Leu Asn Glu Glu Gln Asp Lys Arg Phe
                245                 250                 255

Val Cys Lys His Ser Met Val Asp Arg Gly Trp Gly Asn Gly Cys Gly
            260                 265                 270

Leu Phe Gly Lys Gly Gly Ile Val Thr Cys Ala Met Phe Thr Cys Lys
        275                 280                 285

Lys Asn Met Glu Gly Lys Xaa Val Gln Pro Glu Asn Leu Glu Tyr Thr
    290                 295                 300

Ile Val Ile Thr Pro His Ser Gly Glu Glu His Ala Val Gly Asn Asp
305                 310                 315                 320

Thr Gly Lys His Gly Lys Glu Ile Lys Ile Thr Pro Gln Ser Ser Ile
                325                 330                 335

Thr Glu Ala Glu Leu Thr Gly Tyr Gly Thr Val Thr Met Glu Cys Ser
        340                 345                 350

Pro Arg Thr Gly Leu Asp Phe Asn Glu Met Val Leu Leu Gln Met Glu
    355                 360                 365

Asp Lys Ala Trp Leu Val His Arg Gln Trp Phe Leu Asp Leu Pro Leu
    370                 375                 380

Pro Trp Leu Pro Gly Ala Asp Thr Gln Gly Ser Asn Trp Ile Gln Lys
385                 390                 395                 400

Glu Thr Leu Val Thr Phe Lys Asn Pro His Ala Lys Lys Gln Asp Val
                405                 410                 415

Val Val Leu Gly Ser Gln Glu Gly Ala Met His Thr Ala Leu Thr Gly
            420                 425                 430

Ala Thr Glu Ile Gln Met Ser Ser Gly Asn Leu Leu Phe Thr Gly His
        435                 440                 445

Leu Lys Cys Arg Leu Arg Met Asp Lys Leu Gln Leu Lys Gly Met Ser
    450                 455                 460

Tyr Ser Met Cys Thr Gly Lys Phe Lys Xaa Val Lys Glu Ile Ala Glu
465                 470                 475                 480

Thr Gln His Gly Thr Ile Val Ile Arg Val Gln Tyr Glu Gly Asp Gly
                485                 490                 495

Ser Pro Cys Lys Ile Pro Phe Glu Ile Met Asp Leu Glu Lys Arg His
            500                 505                 510

Val Leu Gly Arg Leu Ile Thr Val Asn Pro Ile Val Thr Glu Lys Asp
        515                 520                 525

Ser Pro Val Asn Ile Glu Ala Glu Pro Pro Phe Gly Asp Ser Tyr Ile
    530                 535                 540

Ile Ile Gly Val Glu Pro Gly Gln Leu Lys Leu Asn Trp Phe Lys Lys
545                 550                 555                 560

Gly Ser Ser Ile Gly Gln Met Phe Glu Thr Thr Met Arg Gly Ala Lys
                565                 570                 575

Arg Met Ala Ile Leu Gly Asp Thr Ala Trp Asp Phe Gly Ser Leu Gly
            580                 585                 590

Gly Val Phe Thr Ser Ile Gly Lys Ala Leu His Gln Val Phe Gly Ala
        595                 600                 605

Ile Tyr Gly Ala Ala Phe Ser Gly Val Ser Trp Thr Met Lys Ile Leu
    610                 615                 620

Ile Gly Val Ile Ile Thr Trp Ile Gly Met Asn Ser Arg Ser Thr Ser
625                 630                 635                 640

Leu Ser Val Ser Leu Val Leu Val Gly Val Val Thr Leu Tyr Leu Gly
                645                 650                 655

Val Met Val Gln Ala
            660

<210>  4
<211>  1983
<212>  DNA
<213>  Artificial Sequence

<220>
<223>  prM+E LAV2

<400>  4
ttccatttaa ccacacgtaa cggagaacca cacatgatcg tcagcagaca agagaaaggg      60

aaaagtcttc tgtttaaaac agaggttggc gtgaacatgt gtaccctcat ggccatggac     120

cttggtgaat tgtgtgaaga cacaatcacg tacaagtgtc cccttctcag gcagaatgag     180

ccagaagaca tagactgttg gtgcaactct acgtccacgt gggtaactta tgggacgtgt     240

accaccatgg gagaacatag aagagaaaaa agatcagtgg cactcgttcc acatgtggga     300

atgggactgg agacacgaac tgaaacatgg atgtcatcag aaggggcctg gaaacatgtc     360

cagagaattg aaacttggat cttgagacat ccaggcttca ccatgatggc agcaatcctg     420

gcatacacca taggaacgac acatttccaa agagccctga ttttcatctt actgacagct     480

gtcactcctt caatgacaat gcgttgcata ggaatgtcaa atagagactt tgtggaaggg     540

gtttcaggag gaagctgggt tgacatagtc ttagaacatg gaagctgtgt gacgacgatg     600

gcaaaaaaca aaccaacatt ggattttgaa ctgataaaaa cagaagccaa acagcctgcc     660

accctaagga agtactgtat agaggcaaag ctaaccaaca caacaacaga atctcgctgc     720

ccaacacaag gggaacccag cctaaatgaa gagcaggaca aaaggttcgt ctgcaaacac     780

tccatggtag acagaggatg gggaaatgga tgtggactat ttggaaaggg aggcattgtg     840

acctgtgcta tgttcagatg caaaaagaac atggaaggaa aagttgtgca accagaaaac     900

ttggaataca ccattgtgat aacacctcac tcaggggaag agcatgcagt cggaaatgac     960

acaggaaaac atggcaagga aatcaaaata acaccacaga gttccatcac agaagcagaa 1020

ttgacaggtt atggcactgt cacaatggag tgctctccaa gaacgggcct cgacttcaat 1080

gagatggtgt tgctgcagat ggaaaataaa gcttggctgg tgcacaggca atggttccta 1140

gacctgccgt taccatggtt gcccggagcg gacacacaag ggtcaaattg gatacagaaa 1200

gagacattgg tcactttcaa aaatccccat gcgaagaaac aggatgttgt tgttttagga 1260

tcccaagaag gggccatgca cacagcactt acaggggcca cagaaatcca aatgtcatca 1320

ggaaacttac tcttcacagg acatctcaag tgcaggctga gaatggacaa gctacagctc 1380

aaaggaatgt catactctat gtgcacagga aagtttaaag ttgtgaagga aatagcagaa 1440

acacaacatg gaacaatagt tatcagagtg caatatgaag gggacggctc tccatgcaag 1500

atcccttttg agataatgga tttggaaaaa agacatgtct taggtcgcct gattacagtc 1560

aacccaattg tgacagaaaa agatagccca gtcaacatag aagcagaacc tccatttgga 1620

gacagctaca tcatcatagg agtagagccg ggacaactga agctcaactg gtttaagaaa 1680

ggaagttcta tcggccaaat gtttgagaca acaatgaggg gggcgaagag aatggccatt 1740

ttaggtgaca cagcctggga ttttggatcc ttgggaggag tgtttacatc tataggaaag 1800

gctctccacc aagtctttgg agcaatctat ggagctgcct tcagtggggt ttcatggact 1860

atgaaaatcc tcataggagt cattatcaca tggataggaa tgaattcacg cagcacctca 1920

ctgtctgtga cactagtatt ggtgggaatt gtgacactgt atttgggagt catggtgcag 1980

gcc 1983

<210> 5
<211> 1983
<212> DNA
<213> Artificial Sequence

<220>
<223> prM+E BID/V585

<400> 5
ttccatttaa ccacacgtaa tggagaacca cacatgatcg ttggtaggca agagaaaggg 60

aaaagtcttc tgtttaaaac agaggatggt gttaacatgt gcaccctcat ggccatagac 120

cttggtgaat tgtgtgaaga tacaatcacg tacaagtgcc ccctcctcag gcaaaatgaa 180

ccagaagaca tagattgttg gtgcaactct acgtccacat gggtaactta tgggacatgt 240

accaccacag gagaacacag aagagaaaaa agatcagtgg cactcgttcc acatgtgggc 300

atgggactgg agacacgaac tgaaacatgg atgtcatcag aaggggcctg gaaacatgtt 360

cagagaattg aaacctggat cttgagacat ccaggcttta ccataatggc agcaatcctg 420

gcatatacca taggaacgac acatttccaa agggctctga tcttcatttt actgacagcc 480

gttgctcctt caatgacaat gcgttgcata ggaatatcaa atagagactt cgtagaaggg 540

gtttcaggag gaagttgggt tgacatagtc ttagaacatg gaagttgtgt gacgacgatg 600

gcaaaaaata aaccaacatt ggattttgaa ctgataaaaa cagaagccaa acaacctgcc 660

actctaagga agtactgtat agaagcaaag ctgaccaata caacaacaga atctcgttgc 720

ccaacacaag gggaacccag tctaaatgaa gagcaggaca aaaggttcat ctgcaaacac 780

tccatggtag acagaggatg gggaaatgga tgtggattat ttggaaaggg aggcattgtg 840

acctgtgcta tgttcacatg caaaaagaac atggaaggaa aagtcgtgca gccagaaaat 900

ctggaataca ccatcgtgat aacacctcac tcaggagaag agcacgctgt aggtaatgac 960

acaggaaagc atggcaagga aatcaaaata acaccacaga gctccatcac agaagcagaa 1020

ctgacaggct atggcactgt cacgatggag tgctctccga gaacgggcct cgacttcaat 1080

gagatggtac tgctgcagat ggaagacaaa gcttggctgg tgcacaggca atggttccta 1140

gacctgccgt taccatggct acccggagcg gacacacaag gatcaaattg gatacagaaa 1200

gagacgttgg tcactttcaa aaatccccac gcgaagaaac aggacgtcgt tgttttagga 1260

tctcaagaag gggccatgca cacggcactt acaggggcca cagaaatcca gatgtcatca 1320

ggaaacttac tgttcacagg acatctcaag tgtaggctga gaatggacaa attacagctt 1380

aaaggaatgt catactctat gtgtacagga aagtttaaaa ttgtgaagga aatagcagaa 1440

acacaacatg gaacaatagt tatcagagta caatatgaag gggacggctc tccatgtaag 1500

attccttttg agataatgga tttggaaaaa agacacgtcc taggtcgcct gattacagtg 1560

aacccaatcg taacagaaaa agatagccca gtcaacatag aagcagaacc tccattcgga 1620

gacagctaca tcatcatagg agtagagccg ggacaattga aactcaattg gttcaagaag 1680

ggaagttcca ttggccaaat gtttgagaca acaatgagag gagcgaagag aatggccatt 1740

ttaggtgaca cagcctggga ttttggatcc ctgggaggag tgtttacatc tataggaaag 1800

gctctccacc aagttttcgg agcaatctat ggggctgctt ttagtggggt ctcatggact 1860

atgaaaatcc tcataggagt tattatcaca tggataggaa tgaattcacg tagcacctca 1920

ctgtctgtgt cactagtatt ggtgggagtc gtgacactgt acttgggggt tatggtgcag 1980

gct 1983

<210> 6
<211> 1983
<212> DNA
<213> Artificial Sequence

<220>
<223> prM+E PR/DB023

<400> 6
ttccatttaa ccacacgtaa tggagaacca cacatgatcg ttggtaggca agagaaaggg 60

aaaagtcttc tgttcaaaac agaggatggt gttaacatgt gtaccctcat ggccatagac 120

cttggtgaat tgtgtgaaga tacaatcacg tacaagtgcc ccctcctcag gcaaaatgaa 180

ccagaagaca tagattgttg gtgcaactct acgtccacat gggtaactta tgggacatgt 240

accaccacag gagaacacag aagagaaaaa agatcagtgg cactcgttcc acatgtgggc 300

atgggactgg agacacgaac tgaaacatgg atgtcatcag aaggggcctg gaaacatgtt 360

cagagaattg aaacctggat attgagacat ccaggcttta ccataatggc agcaatcctg 420

gcatatacca taggaacgac acatttccaa agggctctga tcttcatttt actgacagcc 480

gtcgctcctt caatgacaat gcgttgcata ggaatatcaa atagagactt cgtagaaggg 540

gtttcaggag gaagttgggt tgacatagtc ttagaacatg gaagttgtgt gacgacgatg 600

gcaaaaaata aaccaacatt ggattttgaa ctgataaaaa cagaagccaa acaacctgcc 660

actctaagga agtactgtat agaagcaaag ctgaccaata caacaacaga atctcgttgc 720

ccaacacaag gggaacccag tctaaatgaa gagcaggaca aaaggttcat ctgcaaacac 780

tccatggtag acagaggatg gggaaatgga tgtggattat ttggaaaagg aggcattgta 840

acctgtgcta tgttcacatg caaaaagaac atggaaggaa aagttgtgct gccagaaaat 900

ctggaataca ccatcgtgat aacacctcac tcaggagaag agcacgctgt aggtaatgac 960

acaggaaaac atggcaagga aattaaaata acaccacaga gttccatcac agaagcagaa 1020

ctgacaggct atggcactgt cacgatggag tgctctccga gaacgggcct cgacttcaat 1080

gagatggtgc tgctgcagat ggaagacaaa gcctggctgg tgcacaggca atggttccta 1140

gatctgccgt taccatggct acccggagcg gacacacaag gatcaaattg gatacagaaa 1200

gagacgttgg tcactttcaa aaatccccac gcgaagaaac aggacgtcgt tgttttagga 1260

tctcaagaag gggccatgca cacggcactt acaggggcca cagaaatcca gatgtcatca 1320

ggaaacttac tgttcacagg acatctcaag tgtaggctga gaatggacaa attacagctt 1380

aaaggaatgt catactctat gtgtacagga aagtttaaaa ttgtgaagga aatagcagaa 1440

acacaacatg gaacaatagt tatcagagta caatatgaag gggacggctc tccatgtaag 1500

attccttttg agataatgga tttagaaaaa agacacgtcc taggtcgcct gattacagtg 1560

aacccaatcg taacagaaaa agatagccca gtcaacatag aagcagaacc tccattcgga 1620

gacagctaca tcatcatagg agtagagccg ggacaattga aactcaattg gttcaagaag 1680

ggaagttcca ttggccaaat gtttgagaca acaatgagag gagcgaagag aatggccatt 1740

ttaggtgaca cagcctggga ttttggatcc ctgggaggag tgtttacatc tataggaaag 1800

gctctccacc aagttttcgg agcaatctat ggggctgctt ttagtggggt ctcatggact 1860

atgaaaatcc tcataggagt tatcatcaca tggataggaa tgaattcacg tagcacctca 1920

ctgtctgtgt cactagtatt ggtgggagtc gtgacactgt acttgggggt tatggtgcag 1980

gct                                                                 1983

<210>  7
<211>  1983
<212>  DNA
<213>  Artificial Sequence

<220>
<223>  prM+E MD1280

<400>  7
ttccatttaa ccacacgaaa tggagaacca cacatgatcg ttggcagaca agagaaaggg      60

aaaagccttc tgtttaaaac agaggatggt gtgaacatgt gtaccctcat ggccattgat     120

cttggtgaat tgtgtgaaga tacaatcacg tacaagtgcc ccctcctcag gcagaatgaa     180

ccagaagata tagattgttg gtgcaactcc acgtccacat gggtaactta tgggacgtgt     240

accaccacag gagaacacag aagagaaaaa agatcagtgg cactcgttcc acatgtgggt     300

atgggactgg agacacgaac tgaaacatgg atgtcgtcag aaggggcctg gaaacacgct     360

cagagaattg aaacttggat cttgagacat ccaggcttta ccataatggc agcaatcctg     420

gcatataccg taggaacgac acatttccaa agggccctga ttttcatctt actggcagct     480

gtcgctcctt caatgacaat gcgttgcata ggaatatcaa atagagactt tgtagaaggg     540

gtttcaggag gaagctgggt tgacatagtc ttagaacatg gaagttgtgt gacgacaatg     600

gcaaaaaata aaccaacact ggattttgaa ctgataaaaa cagaagccaa acaacctgcc     660

actctaagga agtactgtat agaggcaaag ctgaccaata caacaacaga atctcgttgc     720

ccaacacaag gggaacccag tctaaatgaa gagcaggaca aaaggttcgt ctgcaaacac     780

tccatggtag acagaggatg gggaaatgga tgtggattat ttggaaaggg aggcattgtg     840

acctgtgcta tgttcacatg caaaaagaac atggaaggaa aaatcgtgca accagaaaat     900

ttggaataca ccatcgtgat aacacctcac tcaggagaag agcacgctgt aggtaatgac     960

acaggaaaac atggtaagga aattaaaata acaccacaga gttccatcac agaagcagaa    1020

ctgacaggct atggcacagt cacgatggag tgctctccga gaacgggcct tgacttcaat    1080

gagatggtgc tgctgcagat ggaagataaa gcttggctgg tgcacaggca atggttccta    1140

gacctgccgt taccatggct acccggagcg gacacacaag gatcaaattg gatacagaaa    1200

gagacattgg tcactttcaa aaatccccac gcgaagaagc aggatgtcgt tgttttagga    1260

tctcaagaag gagccatgca cacggcactc acaggggcca cagaaatcca gatgtcatca    1320

ggaaacttac tattcacagg acatctcaaa tgcaggctga gaatggacaa actacagctc    1380

aaaggaatgt catactctat gtgtacagga aagtttaaaa ttgtgaagga aatagcagaa    1440

acacaacatg gaacaatagt tatcagagta caatatgaag gagacggctc tccatgtaag    1500

atcccttttg aaataatgga tttggaaaaa agacatgtct taggtcgcct gattacagtt 1560

aatccgatcg taacagaaaa agatagccca gtcaacatag aagcagaacc tccattcgga 1620

gacagctaca tcattatagg agtagagccg ggacaattga aactcaactg gttcaagaaa 1680

ggaagttcca tcggccaaat gtttgagacg acaatgagag gagcaaagag aatggccatt 1740

ttaggtgaca cagcctggga ttttggatct ctgggaggag tgtttacatc tataggaaag 1800

gctctccacc aagttttcgg agcaatctat ggggctgcct ttagtggggt ttcatggact 1860

atgaaaatcc tcataggagt catcatcaca tggataggaa tgaattcacg tagcacctca 1920

ctgtctgtgt cactagtatt ggtgggaatc ataacactgt acttgggagc tatggtgcag 1980

gct 1983

<210> 8
<211> 661
<212> PRT
<213> Artificial Sequence

<220>
<223> prM+E LAV2

<400> 8

Phe His Leu Thr Thr Arg Asn Gly Glu Pro His Met Ile Val Ser Arg
1               5                   10                  15

Gln Glu Lys Gly Lys Ser Leu Leu Phe Lys Thr Glu Val Gly Val Asn
            20                  25                  30

Met Cys Thr Leu Met Ala Met Asp Leu Gly Glu Leu Cys Glu Asp Thr
        35                  40                  45

Ile Thr Tyr Lys Cys Pro Leu Leu Arg Gln Asn Glu Pro Glu Asp Ile
    50                  55                  60

Asp Cys Trp Cys Asn Ser Thr Ser Thr Trp Val Thr Tyr Gly Thr Cys
65                  70                  75                  80

Thr Thr Met Gly Glu His Arg Arg Glu Lys Arg Ser Val Ala Leu Val
                85                  90                  95

Pro His Val Gly Met Gly Leu Glu Thr Arg Thr Glu Thr Trp Met Ser
            100                 105                 110

Ser Glu Gly Ala Trp Lys His Val Gln Arg Ile Glu Thr Trp Ile Leu
        115                 120                 125

Arg His Pro Gly Phe Thr Met Met Ala Ala Ile Leu Ala Tyr Thr Ile
    130                 135                 140

Gly Thr Thr His Phe Gln Arg Ala Leu Ile Phe Ile Leu Leu Thr Ala
145                 150                 155                 160

Val Thr Pro Ser Met Thr Met Arg Cys Ile Gly Met Ser Asn Arg Asp
                165                 170                 175

Phe Val Glu Gly Val Ser Gly Gly Ser Trp Val Asp Ile Val Leu Glu
            180                 185                 190

His Gly Ser Cys Val Thr Thr Met Ala Lys Asn Lys Pro Thr Leu Asp
        195                 200                 205

Phe Glu Leu Ile Lys Thr Glu Ala Lys Gln Pro Ala Thr Leu Arg Lys
    210                 215                 220

Tyr Cys Ile Glu Ala Lys Leu Thr Asn Thr Thr Thr Glu Ser Arg Cys
225                 230                 235                 240

Pro Thr Gln Gly Glu Pro Ser Leu Asn Glu Glu Gln Asp Lys Arg Phe
                245                 250                 255

Val Cys Lys His Ser Met Val Asp Arg Gly Trp Gly Asn Gly Cys Gly
            260                 265                 270

Leu Phe Gly Lys Gly Gly Ile Val Thr Cys Ala Met Phe Arg Cys Lys
        275                 280                 285

Lys Asn Met Glu Gly Lys Val Val Gln Pro Glu Asn Leu Glu Tyr Thr
    290                 295                 300

Ile Val Ile Thr Pro His Ser Gly Glu Glu His Ala Val Gly Asn Asp
305                 310                 315                 320

Thr Gly Lys His Gly Lys Glu Ile Lys Ile Thr Pro Gln Ser Ser Ile
                325                 330                 335

Thr Glu Ala Glu Leu Thr Gly Tyr Gly Thr Val Thr Met Glu Cys Ser
            340                 345                 350

Pro Arg Thr Gly Leu Asp Phe Asn Glu Met Val Leu Leu Gln Met Glu
        355                 360                 365

Asn Lys Ala Trp Leu Val His Arg Gln Trp Phe Leu Asp Leu Pro Leu
    370                 375                 380

Pro Trp Leu Pro Gly Ala Asp Thr Gln Gly Ser Asn Trp Ile Gln Lys

385                 390                 395                 400

Glu Thr Leu Val Thr Phe Lys Asn Pro His Ala Lys Lys Gln Asp Val
                405                 410                 415

Val Val Leu Gly Ser Gln Glu Gly Ala Met His Thr Ala Leu Thr Gly
            420                 425                 430

Ala Thr Glu Ile Gln Met Ser Ser Gly Asn Leu Leu Phe Thr Gly His
        435                 440                 445

Leu Lys Cys Arg Leu Arg Met Asp Lys Leu Gln Leu Lys Gly Met Ser
    450                 455                 460

Tyr Ser Met Cys Thr Gly Lys Phe Lys Val Val Lys Glu Ile Ala Glu
465                 470                 475                 480

Thr Gln His Gly Thr Ile Val Ile Arg Val Gln Tyr Glu Gly Asp Gly
                485                 490                 495

Ser Pro Cys Lys Ile Pro Phe Glu Ile Met Asp Leu Glu Lys Arg His
            500                 505                 510

Val Leu Gly Arg Leu Ile Thr Val Asn Pro Ile Val Thr Glu Lys Asp
        515                 520                 525

Ser Pro Val Asn Ile Glu Ala Glu Pro Pro Phe Gly Asp Ser Tyr Ile
    530                 535                 540

Ile Ile Gly Val Glu Pro Gly Gln Leu Lys Leu Asn Trp Phe Lys Lys
545                 550                 555                 560

Gly Ser Ser Ile Gly Gln Met Phe Glu Thr Thr Met Arg Gly Ala Lys
                565                 570                 575

Arg Met Ala Ile Leu Gly Asp Thr Ala Trp Asp Phe Gly Ser Leu Gly
            580                 585                 590

Gly Val Phe Thr Ser Ile Gly Lys Ala Leu His Gln Val Phe Gly Ala
        595                 600                 605

Ile Tyr Gly Ala Ala Phe Ser Gly Val Ser Trp Thr Met Lys Ile Leu
    610                 615                 620

Ile Gly Val Ile Ile Thr Trp Ile Gly Met Asn Ser Arg Ser Thr Ser
625                 630                 635                 640

Leu Ser Val Thr Leu Val Leu Val Gly Ile Val Thr Leu Tyr Leu Gly
                645                 650                 655

Val Met Val Gln Ala
            660

<210>  9
<211>  661
<212>  PRT
<213>  Artificial Sequence

<220>
<223>  prM+E BID/V585

<400>  9

Phe His Leu Thr Thr Arg Asn Gly Glu Pro His Met Ile Val Gly Arg
1               5                   10                  15

Gln Glu Lys Gly Lys Ser Leu Leu Phe Lys Thr Glu Asp Gly Val Asn
            20                  25                  30

Met Cys Thr Leu Met Ala Ile Asp Leu Gly Glu Leu Cys Glu Asp Thr
        35                  40                  45

Ile Thr Tyr Lys Cys Pro Leu Leu Arg Gln Asn Glu Pro Glu Asp Ile
    50                  55                  60

Asp Cys Trp Cys Asn Ser Thr Ser Thr Trp Val Thr Tyr Gly Thr Cys
65                  70                  75                  80

Thr Thr Thr Gly Glu His Arg Arg Glu Lys Arg Ser Val Ala Leu Val
                85                  90                  95

Pro His Val Gly Met Gly Leu Glu Thr Arg Thr Glu Thr Trp Met Ser
            100                 105                 110

Ser Glu Gly Ala Trp Lys His Val Gln Arg Ile Glu Thr Trp Ile Leu
        115                 120                 125

Arg His Pro Gly Phe Thr Ile Met Ala Ala Ile Leu Ala Tyr Thr Ile
    130                 135                 140

Gly Thr Thr His Phe Gln Arg Ala Leu Ile Phe Ile Leu Leu Thr Ala
145                 150                 155                 160

Val Ala Pro Ser Met Thr Met Arg Cys Ile Gly Ile Ser Asn Arg Asp
                165                 170                 175

Phe Val Glu Gly Val Ser Gly Gly Ser Trp Val Asp Ile Val Leu Glu

            180                 185                 190

His Gly Ser Cys Val Thr Thr Met Ala Lys Asn Lys Pro Thr Leu Asp
        195                 200                 205

Phe Glu Leu Ile Lys Thr Glu Ala Lys Gln Pro Ala Thr Leu Arg Lys
    210                 215                 220

Tyr Cys Ile Glu Ala Lys Leu Thr Asn Thr Thr Thr Glu Ser Arg Cys
225                 230                 235                 240

Pro Thr Gln Gly Glu Pro Ser Leu Asn Glu Glu Gln Asp Lys Arg Phe
                245                 250                 255

Ile Cys Lys His Ser Met Val Asp Arg Gly Trp Gly Asn Gly Cys Gly
            260                 265                 270

Leu Phe Gly Lys Gly Gly Ile Val Thr Cys Ala Met Phe Thr Cys Lys
        275                 280                 285

Lys Asn Met Glu Gly Lys Val Val Gln Pro Glu Asn Leu Glu Tyr Thr
    290                 295                 300

Ile Val Ile Thr Pro His Ser Gly Glu Glu His Ala Val Gly Asn Asp
305                 310                 315                 320

Thr Gly Lys His Gly Lys Glu Ile Lys Ile Thr Pro Gln Ser Ser Ile
                325                 330                 335

Thr Glu Ala Glu Leu Thr Gly Tyr Gly Thr Val Thr Met Glu Cys Ser
            340                 345                 350

Pro Arg Thr Gly Leu Asp Phe Asn Glu Met Val Leu Leu Gln Met Glu
        355                 360                 365

Asp Lys Ala Trp Leu Val His Arg Gln Trp Phe Leu Asp Leu Pro Leu
    370                 375                 380

Pro Trp Leu Pro Gly Ala Asp Thr Gln Gly Ser Asn Trp Ile Gln Lys
385                 390                 395                 400

Glu Thr Leu Val Thr Phe Lys Asn Pro His Ala Lys Lys Gln Asp Val
                405                 410                 415

Val Val Leu Gly Ser Gln Glu Gly Ala Met His Thr Ala Leu Thr Gly
            420                 425                 430

Ala Thr Glu Ile Gln Met Ser Ser Gly Asn Leu Leu Phe Thr Gly His
        435                 440                 445

Leu Lys Cys Arg Leu Arg Met Asp Lys Leu Gln Leu Lys Gly Met Ser
    450                 455                 460

Tyr Ser Met Cys Thr Gly Lys Phe Lys Ile Val Lys Glu Ile Ala Glu
465                 470                 475                 480

Thr Gln His Gly Thr Ile Val Ile Arg Val Gln Tyr Glu Gly Asp Gly
                485                 490                 495

Ser Pro Cys Lys Ile Pro Phe Glu Ile Met Asp Leu Glu Lys Arg His
            500                 505                 510

Val Leu Gly Arg Leu Ile Thr Val Asn Pro Ile Val Thr Glu Lys Asp
        515                 520                 525

Ser Pro Val Asn Ile Glu Ala Glu Pro Pro Phe Gly Asp Ser Tyr Ile
    530                 535                 540

Ile Ile Gly Val Glu Pro Gly Gln Leu Lys Leu Asn Trp Phe Lys Lys
545                 550                 555                 560

Gly Ser Ser Ile Gly Gln Met Phe Glu Thr Thr Met Arg Gly Ala Lys
                565                 570                 575

Arg Met Ala Ile Leu Gly Asp Thr Ala Trp Asp Phe Gly Ser Leu Gly
            580                 585                 590

Gly Val Phe Thr Ser Ile Gly Lys Ala Leu His Gln Val Phe Gly Ala
        595                 600                 605

Ile Tyr Gly Ala Ala Phe Ser Gly Val Ser Trp Thr Met Lys Ile Leu
    610                 615                 620

Ile Gly Val Ile Ile Thr Trp Ile Gly Met Asn Ser Arg Ser Thr Ser
625                 630                 635                 640

Leu Ser Val Ser Leu Val Leu Val Gly Val Val Thr Leu Tyr Leu Gly
                645                 650                 655

Val Met Val Gln Ala
            660

<210>  10
<211>  661
<212>  PRT

<213>  Artificial Sequence

<220>
<223>  prM+E PR/DB023

<400>  10

Phe His Leu Thr Thr Arg Asn Gly Glu Pro His Met Ile Val Gly Arg
1               5                   10                  15

Gln Glu Lys Gly Lys Ser Leu Leu Phe Lys Thr Glu Asp Gly Val Asn
            20                  25                  30

Met Cys Thr Leu Met Ala Ile Asp Leu Gly Glu Leu Cys Glu Asp Thr
        35                  40                  45

Ile Thr Tyr Lys Cys Pro Leu Leu Arg Gln Asn Glu Pro Glu Asp Ile
    50                  55                  60

Asp Cys Trp Cys Asn Ser Thr Ser Thr Trp Val Thr Tyr Gly Thr Cys
65                  70                  75                  80

Thr Thr Thr Gly Glu His Arg Arg Glu Lys Arg Ser Val Ala Leu Val
                85                  90                  95

Pro His Val Gly Met Gly Leu Glu Thr Arg Thr Glu Thr Trp Met Ser
            100                 105                 110

Ser Glu Gly Ala Trp Lys His Val Gln Arg Ile Glu Thr Trp Ile Leu
        115                 120                 125

Arg His Pro Gly Phe Thr Ile Met Ala Ala Ile Leu Ala Tyr Thr Ile
    130                 135                 140

Gly Thr Thr His Phe Gln Arg Ala Leu Ile Phe Ile Leu Leu Thr Ala
145                 150                 155                 160

Val Ala Pro Ser Met Thr Met Arg Cys Ile Gly Ile Ser Asn Arg Asp
                165                 170                 175

Phe Val Glu Gly Val Ser Gly Gly Ser Trp Val Asp Ile Val Leu Glu
            180                 185                 190

His Gly Ser Cys Val Thr Thr Met Ala Lys Asn Lys Pro Thr Leu Asp
        195                 200                 205

Phe Glu Leu Ile Lys Thr Glu Ala Lys Gln Pro Ala Thr Leu Arg Lys
    210                 215                 220

Tyr Cys Ile Glu Ala Lys Leu Thr Asn Thr Thr Thr Glu Ser Arg Cys
225                 230                 235                 240

Pro Thr Gln Gly Glu Pro Ser Leu Asn Glu Glu Gln Asp Lys Arg Phe
                245                 250                 255

Ile Cys Lys His Ser Met Val Asp Arg Gly Trp Gly Asn Gly Cys Gly
            260                 265                 270

Leu Phe Gly Lys Gly Gly Ile Val Thr Cys Ala Met Phe Thr Cys Lys
        275                 280                 285

Lys Asn Met Glu Gly Lys Val Val Leu Pro Glu Asn Leu Glu Tyr Thr
    290                 295                 300

Ile Val Ile Thr Pro His Ser Gly Glu Glu His Ala Val Gly Asn Asp
305                 310                 315                 320

Thr Gly Lys His Gly Lys Glu Ile Lys Ile Thr Pro Gln Ser Ser Ile
                325                 330                 335

Thr Glu Ala Glu Leu Thr Gly Tyr Gly Thr Val Thr Met Glu Cys Ser
            340                 345                 350

Pro Arg Thr Gly Leu Asp Phe Asn Glu Met Val Leu Leu Gln Met Glu
        355                 360                 365

Asp Lys Ala Trp Leu Val His Arg Gln Trp Phe Leu Asp Leu Pro Leu
    370                 375                 380

Pro Trp Leu Pro Gly Ala Asp Thr Gln Gly Ser Asn Trp Ile Gln Lys
385                 390                 395                 400

Glu Thr Leu Val Thr Phe Lys Asn Pro His Ala Lys Lys Gln Asp Val
                405                 410                 415

Val Val Leu Gly Ser Gln Glu Gly Ala Met His Thr Ala Leu Thr Gly
            420                 425                 430

Ala Thr Glu Ile Gln Met Ser Ser Gly Asn Leu Leu Phe Thr Gly His
        435                 440                 445

Leu Lys Cys Arg Leu Arg Met Asp Lys Leu Gln Leu Lys Gly Met Ser
    450                 455                 460

Tyr Ser Met Cys Thr Gly Lys Phe Lys Ile Val Lys Glu Ile Ala Glu
465                 470                 475                 480

Thr Gln His Gly Thr Ile Val Ile Arg Val Gln Tyr Glu Gly Asp Gly
                485                 490                 495

Ser Pro Cys Lys Ile Pro Phe Glu Ile Met Asp Leu Glu Lys Arg His
            500                 505                 510

Val Leu Gly Arg Leu Ile Thr Val Asn Pro Ile Val Thr Glu Lys Asp
        515                 520                 525

Ser Pro Val Asn Ile Glu Ala Glu Pro Pro Phe Gly Asp Ser Tyr Ile
    530                 535                 540

Ile Ile Gly Val Glu Pro Gly Gln Leu Lys Leu Asn Trp Phe Lys Lys
545                 550                 555                 560

Gly Ser Ser Ile Gly Gln Met Phe Glu Thr Thr Met Arg Gly Ala Lys
                565                 570                 575

Arg Met Ala Ile Leu Gly Asp Thr Ala Trp Asp Phe Gly Ser Leu Gly
            580                 585                 590

Gly Val Phe Thr Ser Ile Gly Lys Ala Leu His Gln Val Phe Gly Ala
        595                 600                 605

Ile Tyr Gly Ala Ala Phe Ser Gly Val Ser Trp Thr Met Lys Ile Leu
    610                 615                 620

Ile Gly Val Ile Ile Thr Trp Ile Gly Met Asn Ser Arg Ser Thr Ser
625                 630                 635                 640

Leu Ser Val Ser Leu Val Leu Val Gly Val Val Thr Leu Tyr Leu Gly
                645                 650                 655

Val Met Val Gln Ala
            660

<210>  11
<211>  661
<212>  PRT
<213>  Artificial Sequence

<220>
<223>  prM+E MD1280

<400>  11

Phe His Leu Thr Thr Arg Asn Gly Glu Pro His Met Ile Val Gly Arg
1               5                   10                  15

Gln Glu Lys Gly Lys Ser Leu Leu Phe Lys Thr Glu Asp Gly Val Asn
            20                  25                  30

Met Cys Thr Leu Met Ala Ile Asp Leu Gly Glu Leu Cys Glu Asp Thr
        35                  40                  45

Ile Thr Tyr Lys Cys Pro Leu Leu Arg Gln Asn Glu Pro Glu Asp Ile
    50                  55                  60

Asp Cys Trp Cys Asn Ser Thr Ser Thr Trp Val Thr Tyr Gly Thr Cys
65                  70                  75                  80

Thr Thr Thr Gly Glu His Arg Arg Glu Lys Arg Ser Val Ala Leu Val
                85                  90                  95

Pro His Val Gly Met Gly Leu Glu Thr Arg Thr Glu Thr Trp Met Ser
            100                 105                 110

Ser Glu Gly Ala Trp Lys His Ala Gln Arg Ile Glu Thr Trp Ile Leu
        115                 120                 125

Arg His Pro Gly Phe Thr Ile Met Ala Ala Ile Leu Ala Tyr Thr Val
    130                 135                 140

Gly Thr Thr His Phe Gln Arg Ala Leu Ile Phe Ile Leu Leu Ala Ala
145                 150                 155                 160

Val Ala Pro Ser Met Thr Met Arg Cys Ile Gly Ile Ser Asn Arg Asp
                165                 170                 175

Phe Val Glu Gly Val Ser Gly Gly Ser Trp Val Asp Ile Val Leu Glu
            180                 185                 190

His Gly Ser Cys Val Thr Thr Met Ala Lys Asn Lys Pro Thr Leu Asp
        195                 200                 205

Phe Glu Leu Ile Lys Thr Glu Ala Lys Gln Pro Ala Thr Leu Arg Lys
    210                 215                 220

Tyr Cys Ile Glu Ala Lys Leu Thr Asn Thr Thr Thr Glu Ser Arg Cys
225                 230                 235                 240

Pro Thr Gln Gly Glu Pro Ser Leu Asn Glu Glu Gln Asp Lys Arg Phe
                245                 250                 255

Val Cys Lys His Ser Met Val Asp Arg Gly Trp Gly Asn Gly Cys Gly
            260                 265                 270

Leu Phe Gly Lys Gly Gly Ile Val Thr Cys Ala Met Phe Thr Cys Lys
        275                 280                 285

Lys Asn Met Glu Gly Lys Ile Val Gln Pro Glu Asn Leu Glu Tyr Thr
    290                 295                 300

Ile Val Ile Thr Pro His Ser Gly Glu Glu His Ala Val Gly Asn Asp
305                 310                 315                 320

Thr Gly Lys His Gly Lys Glu Ile Lys Ile Thr Pro Gln Ser Ser Ile
                325                 330                 335

Thr Glu Ala Glu Leu Thr Gly Tyr Gly Thr Val Thr Met Glu Cys Ser
            340                 345                 350

Pro Arg Thr Gly Leu Asp Phe Asn Glu Met Val Leu Leu Gln Met Glu
        355                 360                 365

Asp Lys Ala Trp Leu Val His Arg Gln Trp Phe Leu Asp Leu Pro Leu
    370                 375                 380

Pro Trp Leu Pro Gly Ala Asp Thr Gln Gly Ser Asn Trp Ile Gln Lys
385                 390                 395                 400

Glu Thr Leu Val Thr Phe Lys Asn Pro His Ala Lys Lys Gln Asp Val
                405                 410                 415

Val Val Leu Gly Ser Gln Glu Gly Ala Met His Thr Ala Leu Thr Gly
            420                 425                 430

Ala Thr Glu Ile Gln Met Ser Ser Gly Asn Leu Leu Phe Thr Gly His
        435                 440                 445

Leu Lys Cys Arg Leu Arg Met Asp Lys Leu Gln Leu Lys Gly Met Ser
    450                 455                 460

Tyr Ser Met Cys Thr Gly Lys Phe Lys Ile Val Lys Glu Ile Ala Glu
465                 470                 475                 480

Thr Gln His Gly Thr Ile Val Ile Arg Val Gln Tyr Glu Gly Asp Gly
                485                 490                 495

Ser Pro Cys Lys Ile Pro Phe Glu Ile Met Asp Leu Glu Lys Arg His
            500                 505                 510

Val Leu Gly Arg Leu Ile Thr Val Asn Pro Ile Val Thr Glu Lys Asp
        515                 520                 525

Ser Pro Val Asn Ile Glu Ala Glu Pro Pro Phe Gly Asp Ser Tyr Ile
    530                 535                 540

Ile Ile Gly Val Glu Pro Gly Gln Leu Lys Leu Asn Trp Phe Lys Lys
545                 550                 555                 560

Gly Ser Ser Ile Gly Gln Met Phe Glu Thr Thr Met Arg Gly Ala Lys
                565                 570                 575

Arg Met Ala Ile Leu Gly Asp Thr Ala Trp Asp Phe Gly Ser Leu Gly
            580                 585                 590

Gly Val Phe Thr Ser Ile Gly Lys Ala Leu His Gln Val Phe Gly Ala
        595                 600                 605

Ile Tyr Gly Ala Ala Phe Ser Gly Val Ser Trp Thr Met Lys Ile Leu
    610                 615                 620

Ile Gly Val Ile Ile Thr Trp Ile Gly Met Asn Ser Arg Ser Thr Ser
625                 630                 635                 640

Leu Ser Val Ser Leu Val Leu Val Gly Ile Ile Thr Leu Tyr Leu Gly
                645                 650                 655

Ala Met Val Gln Ala
            660

<210>  12
<211>  495
<212>  PRT
<213>  Artificial Sequence

<220>
<223>  E universal

<220>
<221>  Variant
<222>  (129)..(129)
<223>  Xaa can be Val or Ile

<220>
<221>  Variant
<222>  (308)..(308)
<223>  Xaa can be Val or Ile

<400>  12

Met Arg Cys Ile Gly Ile Ser Asn Arg Asp Phe Val Glu Gly Val Ser
1               5                   10                  15

Gly Gly Ser Trp Val Asp Ile Val Leu Glu His Gly Ser Cys Val Thr
            20                  25                  30

Thr Met Ala Lys Asn Lys Pro Thr Leu Asp Phe Glu Leu Ile Lys Thr
        35                  40                  45

Glu Ala Lys Gln Pro Ala Thr Leu Arg Lys Tyr Cys Ile Glu Ala Lys
    50                  55                  60

Leu Thr Asn Thr Thr Thr Glu Ser Arg Cys Pro Thr Gln Gly Glu Pro
65                  70                  75                  80

Ser Leu Asn Glu Glu Gln Asp Lys Arg Phe Val Cys Lys His Ser Met
                85                  90                  95

Val Asp Arg Gly Trp Gly Asn Gly Cys Gly Leu Phe Gly Lys Gly Gly
            100                 105                 110

Ile Val Thr Cys Ala Met Phe Thr Cys Lys Lys Asn Met Glu Gly Lys
        115                 120                 125

Xaa Val Gln Pro Glu Asn Leu Glu Tyr Thr Ile Val Ile Thr Pro His
    130                 135                 140

Ser Gly Glu Glu His Ala Val Gly Asn Asp Thr Gly Lys His Gly Lys
145                 150                 155                 160

Glu Ile Lys Ile Thr Pro Gln Ser Ser Ile Thr Glu Ala Glu Leu Thr
                165                 170                 175

Gly Tyr Gly Thr Val Thr Met Glu Cys Ser Pro Arg Thr Gly Leu Asp
            180                 185                 190

Phe Asn Glu Met Val Leu Leu Gln Met Glu Asp Lys Ala Trp Leu Val
        195                 200                 205

His Arg Gln Trp Phe Leu Asp Leu Pro Leu Pro Trp Leu Pro Gly Ala
    210                 215                 220

Asp Thr Gln Gly Ser Asn Trp Ile Gln Lys Glu Thr Leu Val Thr Phe
225                 230                 235                 240

Lys Asn Pro His Ala Lys Lys Gln Asp Val Val Val Leu Gly Ser Gln
                245                 250                 255

Glu Gly Ala Met His Thr Ala Leu Thr Gly Ala Thr Glu Ile Gln Met
            260                 265                 270

Ser Ser Gly Asn Leu Leu Phe Thr Gly His Leu Lys Cys Arg Leu Arg
        275                 280                 285

Met Asp Lys Leu Gln Leu Lys Gly Met Ser Tyr Ser Met Cys Thr Gly
    290                 295                 300

Lys Phe Lys Xaa Val Lys Glu Ile Ala Glu Thr Gln His Gly Thr Ile
305                 310                 315                 320

Val Ile Arg Val Gln Tyr Glu Gly Asp Gly Ser Pro Cys Lys Ile Pro
                325                 330                 335

Phe Glu Ile Met Asp Leu Glu Lys Arg His Val Leu Gly Arg Leu Ile
            340                 345                 350

Thr Val Asn Pro Ile Val Thr Glu Lys Asp Ser Pro Val Asn Ile Glu
        355                 360                 365

Ala Glu Pro Pro Phe Gly Asp Ser Tyr Ile Ile Ile Gly Val Glu Pro
    370                 375                 380

Gly Gln Leu Lys Leu Asn Trp Phe Lys Lys Gly Ser Ser Ile Gly Gln
385                 390                 395                 400

Met Phe Glu Thr Thr Met Arg Gly Ala Lys Arg Met Ala Ile Leu Gly
                405                 410                 415

Asp Thr Ala Trp Asp Phe Gly Ser Leu Gly Gly Val Phe Thr Ser Ile
            420                 425                 430

Gly Lys Ala Leu His Gln Val Phe Gly Ala Ile Tyr Gly Ala Ala Phe
        435                 440                 445

Ser Gly Val Ser Trp Thr Met Lys Ile Leu Ile Gly Val Ile Ile Thr
    450                 455                 460

Trp Ile Gly Met Asn Ser Arg Ser Thr Ser Leu Ser Val Ser Leu Val
465                 470                 475                 480

Leu Val Gly Val Val Thr Leu Tyr Leu Gly Val Met Val Gln Ala
                485                 490                 495

<210>  13
<211>  495
<212>  PRT
<213>  Artificial Sequence

<220>

<223>  E LAV2

<400>  13

Met Arg Cys Ile Gly Met Ser Asn Arg Asp Phe Val Glu Gly Val Ser
1               5                   10                  15

Gly Gly Ser Trp Val Asp Ile Val Leu Glu His Gly Ser Cys Val Thr
            20                  25                  30

Thr Met Ala Lys Asn Lys Pro Thr Leu Asp Phe Glu Leu Ile Lys Thr
        35                  40                  45

Glu Ala Lys Gln Pro Ala Thr Leu Arg Lys Tyr Cys Ile Glu Ala Lys
    50                  55                  60

Leu Thr Asn Thr Thr Thr Glu Ser Arg Cys Pro Thr Gln Gly Glu Pro
65                  70                  75                  80

Ser Leu Asn Glu Glu Gln Asp Lys Arg Phe Val Cys Lys His Ser Met
                85                  90                  95

Val Asp Arg Gly Trp Gly Asn Gly Cys Gly Leu Phe Gly Lys Gly Gly
            100                 105                 110

Ile Val Thr Cys Ala Met Phe Arg Cys Lys Lys Asn Met Glu Gly Lys
        115                 120                 125

Val Val Gln Pro Glu Asn Leu Glu Tyr Thr Ile Val Ile Thr Pro His
    130                 135                 140

Ser Gly Glu Glu His Ala Val Gly Asn Asp Thr Gly Lys His Gly Lys
145                 150                 155                 160

Glu Ile Lys Ile Thr Pro Gln Ser Ser Ile Thr Glu Ala Glu Leu Thr
                165                 170                 175

Gly Tyr Gly Thr Val Thr Met Glu Cys Ser Pro Arg Thr Gly Leu Asp
            180                 185                 190

Phe Asn Glu Met Val Leu Leu Gln Met Glu Asn Lys Ala Trp Leu Val
        195                 200                 205

His Arg Gln Trp Phe Leu Asp Leu Pro Leu Pro Trp Leu Pro Gly Ala
    210                 215                 220

Asp Thr Gln Gly Ser Asn Trp Ile Gln Lys Glu Thr Leu Val Thr Phe
225                 230                 235                 240

Lys Asn Pro His Ala Lys Lys Gln Asp Val Val Val Leu Gly Ser Gln
                245                 250                 255

Glu Gly Ala Met His Thr Ala Leu Thr Gly Ala Thr Glu Ile Gln Met
            260                 265                 270

Ser Ser Gly Asn Leu Leu Phe Thr Gly His Leu Lys Cys Arg Leu Arg
        275                 280                 285

Met Asp Lys Leu Gln Leu Lys Gly Met Ser Tyr Ser Met Cys Thr Gly
    290                 295                 300

Lys Phe Lys Val Val Lys Glu Ile Ala Glu Thr Gln His Gly Thr Ile
305                 310                 315                 320

Val Ile Arg Val Gln Tyr Glu Gly Asp Gly Ser Pro Cys Lys Ile Pro
                325                 330                 335

Phe Glu Ile Met Asp Leu Glu Lys Arg His Val Leu Gly Arg Leu Ile
            340                 345                 350

Thr Val Asn Pro Ile Val Thr Glu Lys Asp Ser Pro Val Asn Ile Glu
        355                 360                 365

Ala Glu Pro Pro Phe Gly Asp Ser Tyr Ile Ile Ile Gly Val Glu Pro
    370                 375                 380

Gly Gln Leu Lys Leu Asn Trp Phe Lys Lys Gly Ser Ser Ile Gly Gln
385                 390                 395                 400

Met Phe Glu Thr Thr Met Arg Gly Ala Lys Arg Met Ala Ile Leu Gly
                405                 410                 415

Asp Thr Ala Trp Asp Phe Gly Ser Leu Gly Gly Val Phe Thr Ser Ile
            420                 425                 430

Gly Lys Ala Leu His Gln Val Phe Gly Ala Ile Tyr Gly Ala Ala Phe
        435                 440                 445

Ser Gly Val Ser Trp Thr Met Lys Ile Leu Ile Gly Val Ile Ile Thr
    450                 455                 460

Trp Ile Gly Met Asn Ser Arg Ser Thr Ser Leu Ser Val Thr Leu Val
465                 470                 475                 480

Leu Val Gly Ile Val Thr Leu Tyr Leu Gly Val Met Val Gln Ala
                485                 490                 495

<210>  14
<211>  495
<212>  PRT
<213>  Artificial Sequence

<220>
<223>  E BID/V585

<400>  14

Met Arg Cys Ile Gly Ile Ser Asn Arg Asp Phe Val Glu Gly Val Ser
1               5                   10                  15

Gly Gly Ser Trp Val Asp Ile Val Leu Glu His Gly Ser Cys Val Thr
            20                  25                  30

Thr Met Ala Lys Asn Lys Pro Thr Leu Asp Phe Glu Leu Ile Lys Thr
        35                  40                  45

Glu Ala Lys Gln Pro Ala Thr Leu Arg Lys Tyr Cys Ile Glu Ala Lys
    50                  55                  60

Leu Thr Asn Thr Thr Thr Glu Ser Arg Cys Pro Thr Gln Gly Glu Pro
65                  70                  75                  80

Ser Leu Asn Glu Glu Gln Asp Lys Arg Phe Ile Cys Lys His Ser Met
                85                  90                  95

Val Asp Arg Gly Trp Gly Asn Gly Cys Gly Leu Phe Gly Lys Gly Gly
            100                 105                 110

Ile Val Thr Cys Ala Met Phe Thr Cys Lys Lys Asn Met Glu Gly Lys
        115                 120                 125

Val Val Gln Pro Glu Asn Leu Glu Tyr Thr Ile Val Ile Thr Pro His
    130                 135                 140

Ser Gly Glu Glu His Ala Val Gly Asn Asp Thr Gly Lys His Gly Lys
145                 150                 155                 160

Glu Ile Lys Ile Thr Pro Gln Ser Ser Ile Thr Glu Ala Glu Leu Thr
                165                 170                 175

Gly Tyr Gly Thr Val Thr Met Glu Cys Ser Pro Arg Thr Gly Leu Asp
            180                 185                 190

Phe Asn Glu Met Val Leu Leu Gln Met Glu Asp Lys Ala Trp Leu Val
        195                 200                 205

His Arg Gln Trp Phe Leu Asp Leu Pro Leu Pro Trp Leu Pro Gly Ala
    210                 215                 220

Asp Thr Gln Gly Ser Asn Trp Ile Gln Lys Glu Thr Leu Val Thr Phe
225                 230                 235                 240

Lys Asn Pro His Ala Lys Lys Gln Asp Val Val Val Leu Gly Ser Gln
                245                 250                 255

Glu Gly Ala Met His Thr Ala Leu Thr Gly Ala Thr Glu Ile Gln Met
            260                 265                 270

Ser Ser Gly Asn Leu Leu Phe Thr Gly His Leu Lys Cys Arg Leu Arg
        275                 280                 285

Met Asp Lys Leu Gln Leu Lys Gly Met Ser Tyr Ser Met Cys Thr Gly
    290                 295                 300

Lys Phe Lys Ile Val Lys Glu Ile Ala Glu Thr Gln His Gly Thr Ile
305                 310                 315                 320

Val Ile Arg Val Gln Tyr Glu Gly Asp Gly Ser Pro Cys Lys Ile Pro
                325                 330                 335

Phe Glu Ile Met Asp Leu Glu Lys Arg His Val Leu Gly Arg Leu Ile
            340                 345                 350

Thr Val Asn Pro Ile Val Thr Glu Lys Asp Ser Pro Val Asn Ile Glu
        355                 360                 365

Ala Glu Pro Pro Phe Gly Asp Ser Tyr Ile Ile Ile Gly Val Glu Pro
    370                 375                 380

Gly Gln Leu Lys Leu Asn Trp Phe Lys Lys Gly Ser Ser Ile Gly Gln
385                 390                 395                 400

Met Phe Glu Thr Thr Met Arg Gly Ala Lys Arg Met Ala Ile Leu Gly
                405                 410                 415

Asp Thr Ala Trp Asp Phe Gly Ser Leu Gly Gly Val Phe Thr Ser Ile
            420                 425                 430

Gly Lys Ala Leu His Gln Val Phe Gly Ala Ile Tyr Gly Ala Ala Phe
        435                 440                 445

Ser Gly Val Ser Trp Thr Met Lys Ile Leu Ile Gly Val Ile Ile Thr
    450                 455                 460

Trp Ile Gly Met Asn Ser Arg Ser Thr Ser Leu Ser Val Ser Leu Val
465                 470                 475                 480

Leu Val Gly Val Val Thr Leu Tyr Leu Gly Val Met Val Gln Ala
                485                 490                 495

<210>  15
<211>  495
<212>  PRT
<213>  Artificial Sequence

<220>
<223>  E PR/DB023

<400>  15

Met Arg Cys Ile Gly Ile Ser Asn Arg Asp Phe Val Glu Gly Val Ser
1               5                   10                  15

Gly Gly Ser Trp Val Asp Ile Val Leu Glu His Gly Ser Cys Val Thr
            20                  25                  30

Thr Met Ala Lys Asn Lys Pro Thr Leu Asp Phe Glu Leu Ile Lys Thr
        35                  40                  45

Glu Ala Lys Gln Pro Ala Thr Leu Arg Lys Tyr Cys Ile Glu Ala Lys
    50                  55                  60

Leu Thr Asn Thr Thr Thr Glu Ser Arg Cys Pro Thr Gln Gly Glu Pro
65                  70                  75                  80

Ser Leu Asn Glu Glu Gln Asp Lys Arg Phe Ile Cys Lys His Ser Met
                85                  90                  95

Val Asp Arg Gly Trp Gly Asn Gly Cys Gly Leu Phe Gly Lys Gly Gly
            100                 105                 110

Ile Val Thr Cys Ala Met Phe Thr Cys Lys Lys Asn Met Glu Gly Lys
        115                 120                 125

Val Val Leu Pro Glu Asn Leu Glu Tyr Thr Ile Val Ile Thr Pro His
    130                 135                 140

Ser Gly Glu Glu His Ala Val Gly Asn Asp Thr Gly Lys His Gly Lys
145                 150                 155                 160

Glu Ile Lys Ile Thr Pro Gln Ser Ser Ile Thr Glu Ala Glu Leu Thr
                165                 170                 175

Gly Tyr Gly Thr Val Thr Met Glu Cys Ser Pro Arg Thr Gly Leu Asp
            180                 185                 190

Phe Asn Glu Met Val Leu Leu Gln Met Glu Asp Lys Ala Trp Leu Val
        195                 200                 205

His Arg Gln Trp Phe Leu Asp Leu Pro Leu Pro Trp Leu Pro Gly Ala
    210                 215                 220

Asp Thr Gln Gly Ser Asn Trp Ile Gln Lys Glu Thr Leu Val Thr Phe
225                 230                 235                 240

Lys Asn Pro His Ala Lys Lys Gln Asp Val Val Val Leu Gly Ser Gln
                245                 250                 255

Glu Gly Ala Met His Thr Ala Leu Thr Gly Ala Thr Glu Ile Gln Met
            260                 265                 270

Ser Ser Gly Asn Leu Leu Phe Thr Gly His Leu Lys Cys Arg Leu Arg
        275                 280                 285

Met Asp Lys Leu Gln Leu Lys Gly Met Ser Tyr Ser Met Cys Thr Gly
    290                 295                 300

Lys Phe Lys Ile Val Lys Glu Ile Ala Glu Thr Gln His Gly Thr Ile
305                 310                 315                 320

Val Ile Arg Val Gln Tyr Glu Gly Asp Gly Ser Pro Cys Lys Ile Pro
                325                 330                 335

Phe Glu Ile Met Asp Leu Glu Lys Arg His Val Leu Gly Arg Leu Ile
            340                 345                 350

Thr Val Asn Pro Ile Val Thr Glu Lys Asp Ser Pro Val Asn Ile Glu
        355                 360                 365

Ala Glu Pro Pro Phe Gly Asp Ser Tyr Ile Ile Ile Gly Val Glu Pro
    370                 375                 380

Gly Gln Leu Lys Leu Asn Trp Phe Lys Lys Gly Ser Ser Ile Gly Gln
385                 390                 395                 400

Met Phe Glu Thr Thr Met Arg Gly Ala Lys Arg Met Ala Ile Leu Gly
                405                 410                 415

Asp Thr Ala Trp Asp Phe Gly Ser Leu Gly Gly Val Phe Thr Ser Ile
            420                 425                 430

Gly Lys Ala Leu His Gln Val Phe Gly Ala Ile Tyr Gly Ala Ala Phe
        435                 440                 445

Ser Gly Val Ser Trp Thr Met Lys Ile Leu Ile Gly Val Ile Ile Thr
    450                 455                 460

Trp Ile Gly Met Asn Ser Arg Ser Thr Ser Leu Ser Val Ser Leu Val
465                 470                 475                 480

Leu Val Gly Val Val Thr Leu Tyr Leu Gly Val Met Val Gln Ala
                485                 490                 495

<210>  16
<211>  495
<212>  PRT
<213>  Artificial Sequence

<220>
<223>  E MD1280

<400>  16

Met Arg Cys Ile Gly Ile Ser Asn Arg Asp Phe Val Glu Gly Val Ser
1               5                   10                  15

Gly Gly Ser Trp Val Asp Ile Val Leu Glu His Gly Ser Cys Val Thr
            20                  25                  30

Thr Met Ala Lys Asn Lys Pro Thr Leu Asp Phe Glu Leu Ile Lys Thr
        35                  40                  45

Glu Ala Lys Gln Pro Ala Thr Leu Arg Lys Tyr Cys Ile Glu Ala Lys
    50                  55                  60

Leu Thr Asn Thr Thr Thr Glu Ser Arg Cys Pro Thr Gln Gly Glu Pro
65                  70                  75                  80

Ser Leu Asn Glu Glu Gln Asp Lys Arg Phe Val Cys Lys His Ser Met
                85                  90                  95

Val Asp Arg Gly Trp Gly Asn Gly Cys Gly Leu Phe Gly Lys Gly Gly
            100                 105                 110

Ile Val Thr Cys Ala Met Phe Thr Cys Lys Lys Asn Met Glu Gly Lys
        115                 120                 125

Ile Val Gln Pro Glu Asn Leu Glu Tyr Thr Ile Val Ile Thr Pro His
    130                 135                 140

Ser Gly Glu Glu His Ala Val Gly Asn Asp Thr Gly Lys His Gly Lys
145                 150                 155                 160

Glu Ile Lys Ile Thr Pro Gln Ser Ser Ile Thr Glu Ala Glu Leu Thr
                165                 170                 175

Gly Tyr Gly Thr Val Thr Met Glu Cys Ser Pro Arg Thr Gly Leu Asp
            180                 185                 190

Phe Asn Glu Met Val Leu Leu Gln Met Glu Asp Lys Ala Trp Leu Val
        195                 200                 205

His Arg Gln Trp Phe Leu Asp Leu Pro Leu Pro Trp Leu Pro Gly Ala
    210                 215                 220

Asp Thr Gln Gly Ser Asn Trp Ile Gln Lys Glu Thr Leu Val Thr Phe
225                 230                 235                 240

Lys Asn Pro His Ala Lys Lys Gln Asp Val Val Val Leu Gly Ser Gln
                245                 250                 255

Glu Gly Ala Met His Thr Ala Leu Thr Gly Ala Thr Glu Ile Gln Met
            260                 265                 270

Ser Ser Gly Asn Leu Leu Phe Thr Gly His Leu Lys Cys Arg Leu Arg
        275                 280                 285

Met Asp Lys Leu Gln Leu Lys Gly Met Ser Tyr Ser Met Cys Thr Gly
    290                 295                 300

Lys Phe Lys Ile Val Lys Glu Ile Ala Glu Thr Gln His Gly Thr Ile
305                 310                 315                 320

Val Ile Arg Val Gln Tyr Glu Gly Asp Gly Ser Pro Cys Lys Ile Pro
                325                 330                 335

Phe Glu Ile Met Asp Leu Glu Lys Arg His Val Leu Gly Arg Leu Ile
            340                 345                 350

Thr Val Asn Pro Ile Val Thr Glu Lys Asp Ser Pro Val Asn Ile Glu
        355                 360                 365

Ala Glu Pro Pro Phe Gly Asp Ser Tyr Ile Ile Ile Gly Val Glu Pro
    370                 375                 380

Gly Gln Leu Lys Leu Asn Trp Phe Lys Lys Gly Ser Ser Ile Gly Gln
385                 390                 395                 400

Met Phe Glu Thr Thr Met Arg Gly Ala Lys Arg Met Ala Ile Leu Gly
                405                 410                 415

Asp Thr Ala Trp Asp Phe Gly Ser Leu Gly Gly Val Phe Thr Ser Ile
            420                 425                 430

Gly Lys Ala Leu His Gln Val Phe Gly Ala Ile Tyr Gly Ala Ala Phe
        435                 440                 445

Ser Gly Val Ser Trp Thr Met Lys Ile Leu Ile Gly Val Ile Ile Thr
    450                 455                 460

Trp Ile Gly Met Asn Ser Arg Ser Thr Ser Leu Ser Val Ser Leu Val
465                 470                 475                 480

Leu Val Gly Ile Ile Thr Leu Tyr Leu Gly Ala Met Val Gln Ala
                485                 490                 495

<210>  17
<211>  75
<212>  PRT
<213>  Artificial Sequence

<220>
<223>  M consensus

<400>  17

Ser Val Ala Leu Val Pro His Val Gly Met Gly Leu Glu Thr Arg Thr
1               5                   10                  15

Glu Thr Trp Met Ser Ser Glu Gly Ala Trp Lys His Val Gln Arg Ile
            20                  25                  30

Glu Thr Trp Ile Leu Arg His Pro Gly Phe Thr Ile Met Ala Ala Ile
        35                  40                  45

Leu Ala Tyr Thr Ile Gly Thr Thr His Phe Gln Arg Ala Leu Ile Phe
    50                  55                  60

Ile Leu Leu Thr Ala Val Ala Pro Ser Met Thr
65                  70                  75

<210>  18
<211>  495
<212>  PRT
<213>  Artificial Sequence

<220>

<223>  E circulating strain

<400>  18

Met Arg Cys Ile Gly Ile Ser Asn Arg Asp Phe Val Glu Gly Val Ser
1               5                   10                  15

Gly Gly Ser Trp Val Asp Ile Val Leu Glu His Gly Ser Cys Val Thr
            20                  25                  30

Thr Met Ala Lys Asn Lys Pro Thr Leu Asp Phe Glu Leu Ile Lys Thr
        35                  40                  45

Glu Ala Lys Gln Pro Ala Thr Leu Arg Lys Tyr Cys Ile Glu Ala Lys
    50                  55                  60

Leu Thr Asn Thr Thr Thr Glu Ser Arg Cys Pro Thr Gln Gly Glu Pro
65                  70                  75                  80

Ser Leu Lys Glu Glu Gln Asp Lys Arg Phe Val Cys Lys His Ser Met
                85                  90                  95

Val Asp Arg Gly Trp Gly Asn Gly Cys Gly Leu Phe Gly Lys Gly Gly
            100                 105                 110

Ile Val Thr Cys Ala Met Phe Thr Cys Lys Lys Asn Met Glu Gly Lys
        115                 120                 125

Ile Val Gln Pro Glu Asn Leu Glu Tyr Thr Ile Val Val Thr Pro His
    130                 135                 140

Ser Gly Glu Glu His Ala Val Gly Asn Asp Thr Gly Lys His Gly Lys
145                 150                 155                 160

Glu Ile Lys Val Thr Pro Gln Ser Ser Ile Thr Glu Ala Glu Leu Thr
                165                 170                 175

Gly Tyr Gly Thr Val Thr Met Glu Cys Ser Pro Arg Thr Gly Leu Asp
            180                 185                 190

Phe Asn Glu Met Val Leu Leu Gln Met Glu Asn Lys Ala Trp Leu Val
        195                 200                 205

His Arg Gln Trp Phe Leu Asp Leu Pro Leu Pro Trp Leu Pro Gly Ala
    210                 215                 220

Asp Lys Gln Glu Ser Asn Trp Ile Gln Lys Glu Thr Leu Val Thr Phe
225                 230                 235                 240

Lys Asn Pro His Ala Lys Lys Gln Asp Val Val Val Leu Gly Ser Gln
                245                 250                 255

Glu Gly Ala Met His Thr Ala Leu Thr Gly Ala Thr Glu Ile Gln Met
            260                 265                 270

Ser Ser Gly Asn Leu Leu Phe Thr Gly His Leu Lys Cys Arg Leu Arg
        275                 280                 285

Met Asp Lys Leu Gln Leu Lys Gly Met Ser Tyr Ser Met Cys Thr Gly
    290                 295                 300

Lys Phe Lys Val Val Lys Glu Ile Ala Glu Thr Gln His Gly Thr Ile
305                 310                 315                 320

Val Ile Arg Val Gln Tyr Glu Gly Asp Gly Ser Pro Cys Lys Ile Pro
                325                 330                 335

Phe Glu Ile Met Asp Leu Glu Lys Arg Tyr Val Leu Gly Arg Leu Ile
            340                 345                 350

Thr Val Asn Pro Ile Val Thr Glu Lys Asp Ser Pro Val Asn Ile Glu
        355                 360                 365

Ala Glu Pro Pro Phe Gly Asp Ser Tyr Ile Ile Ile Gly Val Glu Pro
    370                 375                 380

Gly Gln Leu Lys Leu Asn Trp Phe Lys Lys Gly Ser Ser Ile Gly Gln
385                 390                 395                 400

Met Phe Glu Thr Thr Met Arg Gly Ala Lys Arg Met Ala Ile Leu Gly
                405                 410                 415

Asp Thr Ala Trp Asp Phe Gly Ser Leu Gly Gly Val Phe Thr Ser Ile
            420                 425                 430

Gly Lys Ala Leu His Gln Val Phe Gly Ala Ile Tyr Gly Ala Ala Phe
        435                 440                 445

Ser Gly Val Ser Trp Thr Met Lys Ile Leu Ile Gly Val Ile Ile Thr
    450                 455                 460

Trp Ile Gly Met Asn Ser Arg Ser Thr Ser Leu Ser Val Ser Leu Val
465                 470                 475                 480

Leu Val Gly Ile Val Thr Leu Tyr Leu Gly Val Met Val Gln Ala
                485                 490                 495

<210>  19
<211>  75
<212>  PRT
<213>  Artificial Sequence

<220>
<223>  M LAV2

<400>  19

Ser Val Ala Leu Val Pro His Val Gly Met Gly Leu Glu Thr Arg Thr
1               5                   10                  15

Glu Thr Trp Met Ser Ser Glu Gly Ala Trp Lys His Val Gln Arg Ile
            20                  25                  30

Glu Thr Trp Ile Leu Arg His Pro Gly Phe Thr Met Met Ala Ala Ile
        35                  40                  45

Leu Ala Tyr Thr Ile Gly Thr Thr His Phe Gln Arg Ala Leu Ile Phe
    50                  55                  60

Ile Leu Leu Thr Ala Val Thr Pro Ser Met Thr
65                  70                  75

<210>  20
<211>  75
<212>  PRT
<213>  Artificial Sequence

<220>
<223>  M BID/V585

<400>  20

Ser Val Ala Leu Val Pro His Val Gly Met Gly Leu Glu Thr Arg Thr
1               5                   10                  15

Glu Thr Trp Met Ser Ser Glu Gly Ala Trp Lys His Val Gln Arg Ile
            20                  25                  30

Glu Thr Trp Ile Leu Arg His Pro Gly Phe Thr Ile Met Ala Ala Ile
        35                  40                  45

Leu Ala Tyr Thr Ile Gly Thr Thr His Phe Gln Arg Ala Leu Ile Phe
    50                  55                  60

Ile Leu Leu Thr Ala Val Ala Pro Ser Met Thr
65                  70                  75

<210>  21

<211>  75
<212>  PRT
<213>  Artificial Sequence

<220>
<223>  M PR/DB023

<400>  21

Ser Val Ala Leu Val Pro His Val Gly Met Gly Leu Glu Thr Arg Thr
1               5                   10                  15

Glu Thr Trp Met Ser Ser Glu Gly Ala Trp Lys His Val Gln Arg Ile
            20                  25                  30

Glu Thr Trp Ile Leu Arg His Pro Gly Phe Thr Ile Met Ala Ala Ile
        35                  40                  45

Leu Ala Tyr Thr Ile Gly Thr Thr His Phe Gln Arg Ala Leu Ile Phe
    50                  55                  60

Ile Leu Leu Thr Ala Val Ala Pro Ser Met Thr
65                  70                  75

<210>  22
<211>  75
<212>  PRT
<213>  Artificial Sequence

<220>
<223>  M MD1280

<400>  22

Ser Val Ala Leu Val Pro His Val Gly Met Gly Leu Glu Thr Arg Thr
1               5                   10                  15

Glu Thr Trp Met Ser Ser Glu Gly Ala Trp Lys His Ala Gln Arg Ile
            20                  25                  30

Glu Thr Trp Ile Leu Arg His Pro Gly Phe Thr Ile Met Ala Ala Ile
        35                  40                  45

Leu Ala Tyr Thr Val Gly Thr Thr His Phe Gln Arg Ala Leu Ile Phe
    50                  55                  60

Ile Leu Leu Ala Ala Val Ala Pro Ser Met Thr
65                  70                  75

<210>  23
<211>  75
<212>  PRT
<213>  Artificial Sequence

<220>
<223>  M clinical trial circulating strain

<400>  23

Ser Val Ala Leu Val Pro His Val Gly Met Gly Leu Glu Thr Arg Thr
1               5                   10                  15

Glu Thr Trp Met Ser Ser Glu Gly Ala Trp Lys His Ala Gln Arg Ile
            20                  25                  30

Glu Thr Trp Ile Leu Arg His Pro Gly Phe Thr Ile Met Ala Ala Ile
        35                  40                  45

Leu Ala Tyr Thr Ile Gly Thr Thr His Phe Gln Arg Val Leu Ile Phe
    50                  55                  60

Ile Leu Leu Thr Ala Val Ala Pro Ser Met Thr
65                  70                  75

<210>  24
<211>  10723
<212>  RNA
<213>  Artificial Sequence

<220>
<223>  VDV2

<400>  24
aguuguuagu cuacguggac cgacaaagac agauucuuug agggagcuaa gcucaaugua      60

guucuaacag uuuuuuaauu agagagcaga ucucugauga auaaccaacg gaaaaaggcg     120

aaaaacacgc cuuucaauau gcugaaacgc gagagaaacc gcgugucgac ugugcaacag     180

cugacaaaga gauucucacu uggaaugcug cagggacgag gaccauuaaa acuguucaug     240

gcccuggugg cguuccuucg uuuccuaaca aucccaccaa cagcagggau auugaagaga     300

uggggaacaa uuaaaaaauc aaaagcuauu aauguuuuga gaggguucag gaaagagauu     360

ggaaggaugc ugaacaucuu gaauaggaga cgcagaucug caggcaugau cauuaugcug     420

auuccaacag ugauggcguu ccauuuaacc acacguaacg gagaaccaca caugaucguc     480

agcagacaag agaaagggaa aagucuucug uuuaaaacag agguuggcgu gaacaugugu     540

acccucaugg ccauggaccu uggugaauug ugugaagaca caaucacgua caaguguccc     600

cuucucaggc agaaugagcc agaagacaua gacuguuggu gcaacucuac guccacgugg     660

guaacuuaug ggacguguac caccauggga gaacauagaa gagaaaaaag aucaguggca     720

cucguuccac augugcgaau gggacuggag acacgaacug aaacauggau gucaucagaa     780

ggggccugga aacaugucca gagaauugaa acuuggaucu ugagacaucc aggcuucacc     840

augauggcag caauccuggc auacaccaua ggaacgacac auuuccaaag agcccugauu 900

uucaucuuac ugacagcugu cacuccuuca augacaaugc guugcauagg aaugucaaau 960

agagacuuug uggaaggggu uucaggagga agcuggguug acauagucuu agaacaugga 1020

agcuguguga cgacgauggc aaaaaacaaa ccaacauugg auuuugaacu gauaaaaaca 1080

gaagccaaac agccugccac ccuaaggaag uacuguauag aggcaaagcu aaccaacaca 1140

acaacagaau cucgcugccc aacacaaggg gaacccagcc uaaaugaaga gcaggacaaa 1200

agguucgucu gcaaacacuc caugguagac agaggauggg gaaauggaug uggacuauuu 1260

ggaaagggag gcauugugac cugugcuaug uucagaugca aaaagaacau ggaaggaaaa 1320

guugugcaac cagaaaacuu ggaauacacc auugugauaa caccucacuc aggggaagag 1380

caugcagucg gaaaugacac aggaaaacau ggcaaggaaa ucaaaauaac accacagagu 1440

uccaucacag aagcagaauu gacagguuau ggcacuguca caauggagug cucuccaaga 1500

acgggccucg acuucaauga gauggguuug cugcagaugg aaaauaaagc uuggcuggug 1560

cacaggcaau gguuccuaga ccugccguua ccaugguugc ccggagcgga cacacaagag 1620

ucaaauugga uacagaagga gacauugguc acuuucaaaa auccccaugc gaagaaacag 1680

gauguuguug uuuuaggauc ccaagaaggg gccaugcaca cagcacuuac aggggccaca 1740

gaaauccaaa ugucaucagg aaacuuacuc uucacaggac aucucaagug caggcugaga 1800

auggacaagc uacagcucaa aggaauguca uacucuaugu gcacaggaaa guuuaaaguu 1860

gugaaggaaa uagcagaaac acaacaugga acaauaguua ucagagugca auaugaaggg 1920

gacggcucuc caugcaagau cccuuuugag auaauggauu uggaaaaaag acaugucuua 1980

ggucgccuga uuacagucaa cccaauugug acagaaaaag auagcccagu caacauagaa 2040

gcagaaccuc cauuuggaga cagcuacauc aucauaggag uagagccggg acaacugaag 2100

cucaacuggu uuaagaaagg aaguucuauc ggccaaaugu uugagacaac aaugaggggg 2160

gcgaagagaa uggccauuuu aggugacaca gccugggauu uuggauccuu gggaggagug 2220

uuuacaucua uaggaaaggc ucuccaccaa gucuuuggag caaucuaugg agcugccuuc 2280

agugggguuu cauggacuau gaaaauccuc auaggaguca uuaucacaug gauaggaaug 2340

aauucacgca gcaccucacu gucugugaca cuaguauugg ugggaauugu gacacuguau 2400

uugggaguca uggugcaggc cgauaguggu ugcguuguga gcuggaaaaa caaagaacug 2460

aaauguggca gugggauuuu caucacagac aacgugcaca cauggacaga acaauacaaa 2520

uuccaaccag aauccccuuc aaaacuagcu ucagcuaucc agaaagccca ugaagaggac 2580

auuuguggaa uccgcucagu aacaagacug gagaaucuga uguggaaaca aauaacacca 2640

gaauugaauc acauucuauc agaaaaugag gugaaguuaa cuauuaugac aggagacauc 2700

aaaggaauca ugcaggcagg aaaacgaucu cugcggccuc agcccacuga gcugaaguau 2760

ucauggaaaa cauggggcaa agcaaaaaug cucucuacag agucucauaa ccagaccuuu 2820

cucauugaug gccccgaaac agcagaaugc cccaacacaa auagagcuug gaauucguug 2880

gaaguugaag acuauggcuu uggaguauuc accaccaaua uauggcuaaa auugaaagaa 2940

aaacaggaug uauucugcga cucaaaacuc augucagcgg ccauaaaaga caacagagcc 3000

guccaugccg auauggguua uuggauagaa agugcacuca augacacaug gaagauagag 3060

aaagccucuu ucauugaagu uaaaaacugc cacuggccaa aaucacacac ccucuggagc 3120

aauggagugc uagaaaguga gaugauaauu ccaaagaauc ucgcuggacc agugucucaa 3180

cacaacuaua gaccaggcua ccauacacaa auaacaggac cauggcaucu agguaagcuu 3240

gagauggacu uugauuucug ugauggaaca acagugguag ugacugagga cugcggaaau 3300

agaggacccu cuuugagaac aaccacugcc ucuggaaaac ucauaacaga auggugcugc 3360

cgaucuugca cauuaccacc gcuaagauac agaggugagg augggugcug guacgggaug 3420

gaaaucagac cauugaagga gaaagaagag aauuugguca acuccuuggu cacagcugga 3480

caugggcagg ucgacaacuu uucacuagga gucuugggaa uggcauuguu ccuggaggaa 3540

augcuuagga cccgaguagg aacgaaacau gcaauacuac uaguugcagu uucuuuugug 3600

acauugauca cagggaacau guccuuuaga gaccugggaa gagugauggu uaugguaggc 3660

gccacuauga cggaugacau agguaugggc gugacuuauc uugcccuacu agcagccuuc 3720

aaagucagac caacuuuugc agcuggacua cucuugagaa agcugaccuc caaggaauug 3780

augaugacua cuauaggaau uguacuccuc ucccagagca ccauaccaga gaccauucuu 3840

gaguugacug augcguuagc cuuaggcaug augguccuca aaauggugag aaauauggaa 3900

aaguaucaau uggcagugac uaucauggcu aucuugugcg ucccaaacgc agugauauua 3960

caaaacgcau ggaaagugag uugcacaaua uuggcagugg uguccguuuc cccacuguuc 4020

uuaacauccu cacagcaaaa aacagauugg auaccauuag cauugacgau caaaggucuc 4080

aauccaacag cuauuuuucu aacaacccuc ucaagaacca gcaagaaaag gagcuggcca 4140

uuaaaugagg cuaucauggc agucgggaug gugagcauuu uagccaguuc ucuccuaaaa 4200

aaugauauuc ccaugacagg accauuagug gcuggagggc uccucacugu gugcuacgug 4260

cucacuggac gaucggccga uuuggaacug gagagagcag ccgaugucaa augggaagac 4320

caggcagaga uaucaggaag caguccaauc cugucaauaa caauaucaga agaugguagc 4380

augucgauaa aaaaugaaga ggaagaacaa acacugacca uacucauuag aacaggauug 4440

cuggugaucu caggacuuuu uccuguauca auaccaauca cggcagcagc augguaccug 4500

ugggaaguga agaaacaacg ggccggagua uugugggaug uuccuucacc cccacccaug 4560

ggaaaggcug aacuggaaga uggagccuau agaauuaagc aaaaagggau ucuuggauau 4620

ucccagaucg gagccggagu uuacaaagaa ggaacauucc auacaaugug gcaugucaca 4680

cguggcgcug uucuaaugca uaaaggaaag aggauugaac caacaugggc ggacgucaag 4740

aaagaccuaa uaucauaugg aggaggcugg aaguuagaag gagaauggaa ggaaggagaa 4800

gaaguccagg uauuggcacu ggagccugga aaaaauccaa gagccgucca aacgaaaccu 4860

ggucuuuuca aaaccaacgc cggaacaaua ggugcuguau cucuggacuu uucuccugga 4920

acgucaggau cuccaauuau cgacaaaaaa ggaaaaguug ugggucuuua ugguaauggu 4980

guuguuacaa ggaguggagc auaugugagu gcuauagccc agacugaaaa aagcauugaa 5040

gacaacccag agaucgaaga ucacauuuuc cgaaagagaa gacugaccau cauggaccuc 5100

cacccaggag cgggaaagac gaagagauac cuuccggcca uagucagaga agcuauaaaa 5160

cgggguuuga gaacauuaau cuuggccccc acuagaguug uggcagcuga aauggaggaa 5220

gcccuuagag gacuuccaau aagauaccag accccagcca ucagagcuga gcacaccggg 5280

cgggagauug uggaccuaau gugucaugcc acauuuacca ugaggcugcu aucaccaguu 5340

agagugccaa acuacaaccu gauuaucaug gacgaagccc auuucacaga cccagcaagu 5400

auagcagcua gaggauacau cucaacucga guggagaugg gugaggcagc ugggauuuuu 5460

augacagcca cucccccggg aagcagagac ccauuuccuc agagcaaugc accaaucaua 5520

gaugaagaaa gagaaauccc ugaacgcucg uggaauuccg gacaugaaug ggucacggau 5580

uuuaaaggga agacuguuug guucguucca aguauaaaag caggaaauga uauagcagcu 5640

ugccugagga aaaauggaaa gaaagugaua caacucagua ggaagaccuu ugauucugag 5700

uaugucaaga cuagaaccaa ugauugggac uucgugguua caacugacau uucagaaaug 5760

ggugccaauu ucaaggcuga gaggguuaua gaccccagac gcugcaugaa accagucaua 5820

cuaacagaug gugaagagcg ggugauucug gcaggaccua ugccagugac ccacucuagu 5880

gcagcacaaa gaagagggag aauaggaaga aauccaaaaa augagaauga ccaguacaua 5940

uacauggggg aaccucugga aaaugaugaa gacugugcac acuggaaaga agcuaaaaug 6000

cuccuagaua acaucaacac gccagaagga aucauuccua gcauguucga accagagcgu 6060

gaaaaggugg augccauuga uggcgaauac cgcuugagag gagaagcaag gaaaaccuuu 6120

guagacuuaa ugagaagagg agaccuacca gucugguugg ccuacagagu ggcagcugaa 6180

ggcaucaacu acgcagacag aagguggugu uuugauggag ucaagaacaa ccaaauccua 6240

gaagaaaacg uggaaguuga aaucuggaca aaagaagggg aaaggaagaa auugaaaccc 6300

agaugguugg augcuaggau cuauucugac ccacuggcgc uaaaagaauu uaaggaauuu 6360

gcagccggaa gaaagucucu gacccugaac cuaaucacag aaauggguag gcucccaacc 6420

uucaugacuc agaaggcaag agacgcacug gacaacuuag cagugcugca cacggcugag 6480

gcagguggaa gggcguacaa ccaugcucuc agugaacugc cggagacccu ggagacauug 6540

cuuuuacuga cacuucuggc uacagucacg ggagggaucu uuuuauucuu gaugagcgca 6600

aggggcauag ggaagaugac ccugggaaug ugcugcauaa ucacggcuag cauccuccua 6660

ugguacgcac aaauacagcc acacuggaua gcagcuucaa uaauacugga guuuuuucuc 6720

auaguuuugc uuauuccaga accugaaaaa cagagaacac cccaagacaa ccaacugacc 6780

uacguuguca uagccauccu cacaguggug gccgcaacca uggcaaacga gauggguuuc 6840

cuagaaaaaa cgaagaaaga ucucggauug ggaagcauug caacccagca acccgagagc 6900

aacauccugg acauagaucu acguccugca ucagcaugga cgcuguaugc cguggccaca 6960

acauuuguua caccaauguu gagacauagc auugaaaauu ccucagugaa ugugucccua 7020

acagcuauag ccaaccaagc cacaguguua augggucucg ggaaaggaug gccauuguca 7080

aagauggaca ucggaguucc ccuucucgcc auuggaugcu acucacaagu caaccccaua 7140

acucucacag cagcucuuuu cuuauuggua gcacauuaug ccaucauagg gccaggacuc 7200

caagcaaaag caaccagaga agcucagaaa agagcagcgg cgggcaucau gaaaaaccca 7260

acugucgaug gaauaacagu gauugaccua gauccaauac cuuaugaucc aaaguuugaa 7320

aagcaguugg gacaaguaau gcuccuaguc cucugcguga cucaaguauu gaugaugagg 7380

acuacauggg cucuguguga ggcuuuaacc uuagcuaccg ggcccaucuc cacauugugg 7440

gaaggaaauc cagggagguu uuggaacacu accauugcgg ugucaauggc uaacauuuuu 7500

agagggaguu acuuggccgg agcuggacuu cucuuuucua uuaugaagaa cacaaccaac 7560

acaagaaggg gaacuggcaa cauaggagag acgcuuggag agaaauggaa aagccgauug 7620

aacgcauugg gaaaaaguga auuccagauc uacaagaaaa guggaaucca ggaaguggau 7680

agaaccuuag caaaagaagg cauuaaaaga ggagaaacgg accaucacgc ugugucgcga 7740

ggcucagcaa aacugagaug guucguugag agaaacaugg ucacaccaga agggaaagua 7800

guggaccucg guuguggcag aggaggcugg ucauacuauu guggaggacu aaagaaugua 7860

agagaaguca aaggccuaac aaaaggagga ccaggacacg aagaacccau ccccauguca 7920

acauaugggu ggaaucuagu gcgucuucaa aguggaguug acguuuucuu caucccgcca 7980

gaaaagugug acacauuauu gugugacaua ggggagucau caccaaaucc cacaguggaa 8040

gcaggacgaa cacucagagu ccuuaacuua guagaaaauu gguugaacaa caacacucaa 8100

uuuugcauaa agguucucaa cccauauaug cccucaguca uagaaaaaau ggaagcacua 8160

caaaggaaau auggaggagc cuuagugagg aauccacucu cacgaaacuc cacacaugag 8220

auguacuggg uauccaaugc uuccgggaac auagugucau cagugaacau gauuucaagg 8280

auguugauca acagauuuac aaugagauac aagaaagcca cuuacgagcc ggauguugac 8340

cucggaagcg gaacccguaa caucgggauu gaaagugaga uaccaaaccu agauauaauu 8400

gggaaaagaa uagaaaaaau aaagcaagag caugaaacau cauggcacua ugaccaagac 8460

cacccauaca aaacgugggc auaccauggu agcuaugaaa caaaacagac uggaucagca 8520

ucauccaugg ucaacggagu ggucaggcug cugacaaaac cuugggacgu uguccccaug 8580

gugacacaga uggcaaugac agacacgacu ccauuuggac aacagcgcgu uuuuaaagag 8640

aaaguggaca cgagaaccca agaaccgaaa gaaggcacga agaaacuaau gaaaauaaca 8700

gcagaguggc uuuggaaaga auuagggaag aaaaagacac ccaggaugug caccagagaa 8760

gaauucacaa gaaaggugag aagcaaugca gccuuggggg ccauauucac ugaugagaac 8820

aaguggaagu cggcacguga ggcuguugaa gauaguaggu uuugggagcu gguugacaag 8880

gaaaggaauc uccaucuuga aggaaagugu gaaacaugug uguacaacau gaugggaaaa 8940

agagagaaga agcuagggga auucggcaag gcaaaaggca gcagagccau augguacaug 9000

uggcuuggag cacgcuucuu agaguuugaa gcccuaggau ucuuaaauga agaucacugg 9060

uucuccagag agaacucccu gaguggagug gaaggagaag ggcugcacaa gcuagguuac 9120

auucuaagag acgugagcaa gaaagaggga ggagcaaugu augccgauga caccgcagga 9180

ugggauacaa aaaucacacu agaagaccua aaaaaugaag agaugguaac aaaccacaug 9240

gaaggagaac acaagaaacu agccgaggcc auuuucaaac uaacguacca aaacaaggug 9300

gugcgugugc aaagaccaac accaagaggc acaguaaugg acaucauauc gagaagagac 9360

caaagaggua guggacaagu uggcaccuau ggacucaaua cuuucaccaa uauggaagcc 9420

caacuaauca gacagaugga gggagaagga gucuuuaaaa gcauucagca ccuaacaauc 9480

acagaagaaa ucgcugugca aaacugguua gcaagagugg ggcgcgaaag guuaucaaga 9540

auggccauca guggagauga uuguguugug aaaccuuuag augacagguu cgcaagcgcu 9600

uuaacagcuc uaaaugacau gggaaagauu aggaaagaca uacaacaaug ggaaccuuca 9660

agaggaugga augauuggac acaagugccc uucuguucac accauuucca ugaguuaauc 9720

augaaagacg gucgcguacu cguuguucca uguagaaacc aagaugaacu gauuggcaga 9780

gcccgaaucu cccaaggagc aggguggucu uugcgggaga cggccuguuu ggggaagucu 9840

uacgcccaaa uguggagcuu gauguacuuc cacagacgcg accucaggcu ggcggcaaau 9900

gcuauuugcu cggcaguacc aucacauugg guuccaacaa gucgaacaac cugguccaua 9960

caugcuaaac augaauggau gacaacggaa gacaugcuga cagucuggaa cagggugugg 10020

auucaagaaa acccauggau ggaagacaaa acuccagugg aaacauggga ggaaauccca 10080

uacuugggga aaagagaaga ccaauggugc ggcucauuga uuggguuaac aagcagggcc 10140

accugggcaa agaacaucca agcagcaaua aaucaaguua gaucccuuau aggcaaugaa 10200

gaauacacag auuacaugcc auccaugaaa agauucagaa gagaagagga agaagcagga 10260

guucuguggu agaaagcaaa acuaacauga aacaaggcua gaagucaggu cggauuaagc 10320

cauaguacgg aaaaaacuau gcuaccugug agccccgucc aaggacguua aaagaaguca 10380

ggccaucaua aaugccauag cuugaguaaa cuaugcagcc uguagcucca ccugagaagg 10440

uguaaaaaau ccgggaggcc acaaaccaug gaagcuguac gcauggcgua guggacuagc 10500

gguuagggga gaccccuccc uuacaaaucg cagcaacaau gggggcccaa ggcgagauga 10560

agcuguaguc ucgcuggaag gacuagaggu uagaggagac ccccccgaaa caaaaaacag 10620

cauauugacg cugggaaaga ccagagaucc ugcugucucc ucagcaucau uccaggcaca 10680

gaacgccaga aaauggaaug gugcuguuga aucaacaggu ucu 10723

<210> 25
<211> 1983
<212> RNA
<213> Artificial Sequence

<220>
<223> prM+E VDV2

<400> 25
   uuccauuuaa ccacacguaa cggagaacca cacaugaucg ucagcagaca agagaaaggg 60
   aaaagucuuc uguuuaaaac agagguuggc gugaacaugu guacccucau ggccauggac 120
   cuuggugaau ugugugaaga cacaaucacg uacaaguguc cccuucucag gcagaaugag 180
   ccagaagaca uagacuguug gugcaacucu acguccacgu ggguaacuua ugggacgugu 240
   accaccaugg gagaacauag aagagaaaaa agaucagugg cacucguucc acaugugcga 300
   augggacugg agacacgaac ugaaacaugg augucaucag aaggggccug gaaacauguc 360
   cagagaauug aaacuuggau cuugagacau ccaggcuuca ccaugauggc agcaauccug 420
   gcauacacca uaggaacgac acauuuccaa agagcccuga uuuucaucuu acugacagcu 480
   gucacuccuu caaugacaau gcguugcaua ggaaugucaa auagagacuu uguggaaggg 540
   guuucaggag gaagcugggu ugacauaguc uuagaacaug gaagcugugu gacgacgaug 600
   gcaaaaaaca aaccaacauu ggauuuugaa cugauaaaaa cagaagccaa acagccugcc 660
   acccuaagga aguacuguau agaggcaaag cuaaccaaca caacaacaga aucucgcugc 720
   ccaacacaag gggaacccag ccuaaaugaa gagcaggaca aaagguucgu cugcaaacac 780
   uccaugguag acagaggaug gggaaaugga uguggacuau uuggaaaggg aggcauugug 840
   accugugcua uguucagaug caaaaagaac auggaaggaa aaguugugca accagaaaac 900
   uuggaauaca ccauugugau aacaccucac ucaggggaag agcaugcagu cggaaaugac 960
   acaggaaaac auggcaagga aaucaaaaua acaccacaga guuccaucac agaagcagaa 1020
   uugacagguu auggcacugu cacaauggag ugcucuccaa gaacgggccu cgacuucaau 1080
   gagauggugu ugcugcagau ggaaaauaaa gcuuggcugg ugcacaggca augguuccua 1140
   gaccugccgu uaccaugguu gcccggagcg gacacacaag agucaaauug gauacagaag 1200
   gagacauugg ucacuuucaa aaauccccau gcgaagaaac aggauguugu uguuuuagga 1260
   ucccaagaag gggccaugca cacagcacuu acaggggcca cagaaaucca aaugucauca 1320
   ggaaacuuac ucuucacagg acaucucaag ugcaggcuga gaauggacaa gcuacagcuc 1380
   aaaggaaugu cauacucuau gugcacagga aaguuuaaag uugugaagga aauagcagaa 1440
   acacaacaug gaacaauagu uaucagagug caauaugaag gggacggcuc uccaugcaag 1500
   aucccuuuug agauaaugga uuuggaaaaa agacaugucu uaggucgccu gauuacaguc 1560
   aacccaauug ugacagaaaa agauagccca gucaacauag aagcagaacc uccauuugga 1620
   gacagcuaca ucaucauagg aguagagccg ggacaacuga agcucaacug guuuaagaaa 1680
   ggaaguucua ucggccaaau guuugagaca acaaugaggg gggcgaagag aauggccauu 1740
   uuaggugaca cagccuggga uuuuggaucc uugggaggag uguuuacauc uauaggaaag 1800
   gcucuccacc aagucuuugg agcaaucuau ggagcugccu ucaguggggu uucauggacu 1860
   augaaaaucc ucauaggagu cauuaucaca uggauaggaa ugaauucacg cagcaccuca 1920
   cugucuguga cacuaguauu ggugggaauu gugacacugu auuugggagu cauggugcag 1980
   gcc 1983

<210> 26
<211> 495
<212> PRT
<213> Artificial Sequence

<220>
<223> E VDV2

<400> 26

Met Arg Cys Ile Gly Met Ser Asn Arg Asp Phe Val Glu Gly Val Ser
1               5                   10                  15

Gly Gly Ser Trp Val Asp Ile Val Leu Glu His Gly Ser Cys Val Thr
            20                  25                  30

Thr Met Ala Lys Asn Lys Pro Thr Leu Asp Phe Glu Leu Ile Lys Thr
        35                  40                  45

Glu Ala Lys Gln Pro Ala Thr Leu Arg Lys Tyr Cys Ile Glu Ala Lys
    50                  55                  60

Leu Thr Asn Thr Thr Thr Glu Ser Arg Cys Pro Thr Gln Gly Glu Pro
65                  70                  75                  80

Ser Leu Asn Glu Glu Gln Asp Lys Arg Phe Val Cys Lys His Ser Met
                85                  90                  95

Val Asp Arg Gly Trp Gly Asn Gly Cys Gly Leu Phe Gly Lys Gly Gly
        100                 105                 110

Ile Val Thr Cys Ala Met Phe Arg Cys Lys Lys Asn Met Glu Gly Lys
    115                 120                 125

Val Val Gln Pro Glu Asn Leu Glu Tyr Thr Ile Val Ile Thr Pro His
    130                 135                 140

Ser Gly Glu Glu His Ala Val Gly Asn Asp Thr Gly Lys His Gly Lys
145                 150                 155                 160

Glu Ile Lys Ile Thr Pro Gln Ser Ser Ile Thr Glu Ala Glu Leu Thr
                165                 170                 175

Gly Tyr Gly Thr Val Thr Met Glu Cys Ser Pro Arg Thr Gly Leu Asp
            180                 185                 190

Phe Asn Glu Met Val Leu Leu Gln Met Glu Asn Lys Ala Trp Leu Val
        195                 200                 205

His Arg Gln Trp Phe Leu Asp Leu Pro Leu Pro Trp Leu Pro Gly Ala
    210                 215                 220

Asp Thr Gln Glu Ser Asn Trp Ile Gln Lys Glu Thr Leu Val Thr Phe
225                 230                 235                 240

Lys Asn Pro His Ala Lys Lys Gln Asp Val Val Val Leu Gly Ser Gln
                245                 250                 255

Glu Gly Ala Met His Thr Ala Leu Thr Gly Ala Thr Glu Ile Gln Met
            260                 265                 270

Ser Ser Gly Asn Leu Leu Phe Thr Gly His Leu Lys Cys Arg Leu Arg
        275                 280                 285

Met Asp Lys Leu Gln Leu Lys Gly Met Ser Tyr Ser Met Cys Thr Gly
    290                 295                 300

Lys Phe Lys Val Val Lys Glu Ile Ala Glu Thr Gln His Gly Thr Ile
305                 310                 315                 320

Val Ile Arg Val Gln Tyr Glu Gly Asp Gly Ser Pro Cys Lys Ile Pro
                325                 330                 335

Phe Glu Ile Met Asp Leu Glu Lys Arg His Val Leu Gly Arg Leu Ile

            340                 345                 350

Thr Val Asn Pro Ile Val Thr Glu Lys Asp Ser Pro Val Asn Ile Glu
        355                 360                 365

Ala Glu Pro Pro Phe Gly Asp Ser Tyr Ile Ile Ile Gly Val Glu Pro
    370                 375                 380

Gly Gln Leu Lys Leu Asn Trp Phe Lys Lys Gly Ser Ser Ile Gly Gln
385                 390                 395                 400

Met Phe Glu Thr Thr Met Arg Gly Ala Lys Arg Met Ala Ile Leu Gly
                405                 410                 415

Asp Thr Ala Trp Asp Phe Gly Ser Leu Gly Gly Val Phe Thr Ser Ile
            420                 425                 430

Gly Lys Ala Leu His Gln Val Phe Gly Ala Ile Tyr Gly Ala Ala Phe
        435                 440                 445

Ser Gly Val Ser Trp Thr Met Lys Ile Leu Ile Gly Val Ile Ile Thr
    450                 455                 460

Trp Ile Gly Met Asn Ser Arg Ser Thr Ser Leu Ser Val Thr Leu Val
465                 470                 475                 480

Leu Val Gly Ile Val Thr Leu Tyr Leu Gly Val Met Val Gln Ala
                485                 490                 495

<210> 27
<211> 75
<212> PRT
<213> Artificial Sequence

<220>
<223> M VDV2

<400> 27

Ser Val Ala Leu Val Pro His Val Arg Met Gly Leu Glu Thr Arg Thr
1               5                   10                  15

Glu Thr Trp Met Ser Ser Glu Gly Ala Trp Lys His Val Gln Arg Ile
            20                  25                  30

Glu Thr Trp Ile Leu Arg His Pro Gly Phe Thr Met Met Ala Ala Ile
        35                  40                  45

Leu Ala Tyr Thr Ile Gly Thr Thr His Phe Gln Arg Ala Leu Ile Phe
    50                  55                  60

Ile Leu Leu Thr Ala Val Thr Pro Ser Met Thr
65                  70                  75